# EUROPEAN PATENT APPLICATION

(11) **EP 3 184 514 A1**
(43) Date of publication of application: **28.06.2017**
(21) Application number: 15202510.2
(22) Date of filing: 23.12.2015
(51) Int. Cl.: C07D 251/08, C07D 311/56, A61K 31/53, A61K 31/37, A61P 35/00

(54) **AUTOPHAGY INHIBITORS**

(71) Applicant: Deutsches Krebsforschungszentrum, 69120 Heidelberg (DE); Johann Wolfgang Goethe-Universität Frankfurt am Main, 60323 Frankfurt am Main (DE)
(72) Inventor: The designation of the inventor has not yet been filed
(74) Representative: Rippel, Hans Christoph

(57) **Abstract**

A compound, which is
a) a tetrahydrotriazine derivative of the formula (I), 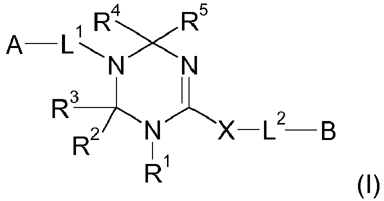 a tautomer, a pharmaceutically acceptable salt, a solvate or hydrate thereof, were the symbols have the meanings given in the description,
or
b) a coumarin derivative of the formula (II), 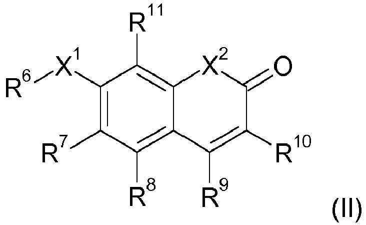 a tautomer, a pharmaceutically acceptable salt, solvate or hydrate thereof, were the symbols have the meanings given in the description,
is useful in a therapeutical method for inhibiting autophagy in a cell and for the treatment of cancer.

## Description

The invention relates to small molecule autophagy inhibitors, in particular inhibitors of LC3B, and their use for the treatment of cancer. The invention further relates to pharmaceutical compositions comprising compounds of the invention and to methods for treating cancer by application of the compounds of the invention.

Cancer is after cardiovascular diseases one of the most frequent causes of death. The last years have shown that the options for the treatment of cancer are still insufficient and, therefore, it is necessary to develop new therapies with selective targeted active ingredients. Moreover, it is important to identify the effective combinations of drugs that simultaneously target the primary drivers of the tumor and the secondary resistance mechanism to avoid the relapse of the cancer. This aspect for cancer drug development paired with characterization of new innovative targets and showing their druggability to get a deeper input in resistance mechanism is necessary for successful future personalized cancer medicine. In this line, the important role of autophagy for future cancer treatment has come into the focus in the last years. Macroautophagy (hereafter referred to as autophagy) is a lysosomal degradation pathway that enables cells to engulf and digest portions of their cytoplasm in a regulated manner, thereby accomplishing quality and quantity control of the cell's interior (Mizushima N. et al., Annual review of cell and developmental biology 2013, 27: 107-132). Autophagy starts with the isolation membrane, a crescent shaped structure originating from the endoplasmatic reticulum (ER), Golgi, plasma membrane or mitochondria (Mari M. et al., F1000 biology reports 2011, 3: 25). This so-called phagophore extends to the autophagosome, which encapsulates and seals its cargo before delivering it to the lysosome. Upon fusion with the lysosome, the cargo is broken down into its constituent components and recycled to fuel the anabolic needs and energy demands of the cell. Over 30 autophagy-related (ATG) genes involved in the process of autophagosome biogenesis have been identified (Mizushima N. et al., Annual review of cell and developmental biology 2013, 27: 107-132). The core group of essential, highly conserved ATG proteins can be divided into subgroups that regulate (i) the initial formation of pre-autophagosomal membrane structures, (ii) the fusion of suchstructures to form autophagosomes, (iii) the recruitment of cargo, and (iv) the autophagosome-lysosome fusion. Amongst them is an ubiquitin-like (Ubl) protein conjugation system with two Ubls, ATG8 and ATG12. While ATG12 is conjugated to ATG5 and functions as an ATG8 ligase scaffold, ATG8 is conjugated to phosphatidylethanolamine (PE). ATG8-PE conjugates are incorporated into both layers of incipient autophagosomes and are important for isolation membrane elongation and complete closure. The ATG8 family in humans consists of eight members of which the MAP1 LC3B (LC3B) is the best-characterized member and serves as the bona fide autophagy marker (Slobodkin MR et al., Essays in biochemistry, 2013, 55: 51-64). Autophagy was long considered as a non-selective bulk process, which only fulfills catabolic needs. Today, accumulated evidence indicates that autophagy has a broader role in enabling cells, tissues and organs to respond to different cellular demands in a highly regulated, selective fashion (Levine B. et al, Cell, 2008, 132: 27-42). This form of autophagy is termed selective autophagy and was shown to also play an important role under nutrient-rich conditions. Among the established selective autophagy pathways is the removal of protein aggregates, of damaged or surplus cellular organelles like mitochondria or peroxisomes, as well as removal of pathogens. By removing dangerous cytosolic components, selective autophagy protects cells from oxidative and genotoxic stresses intracellular pathogens or accumulated protein aggregates. The exact mechanism of cargo recognition during selective autophagy remains largely elusive. First mechanistic insight has been gained through the discovery of specific cargo receptors. For example, SQSTM1 (also known as p62) exerts its action by binding to LC3 via an LC3-interacting region (LIR) motif and to ubiquitylated proteins via a C-terminal ubiquitin-associated domain (Johansen T. et al., Autophagy, 2011, 7: 279-296). A deleterious effect of autophagy ablation was shown in a cell and mouse model of non-small cell lung cancer (Karsli-Uzunbas G. et al., Cancer discovery, 2014, 4: 914-927; Bokobza SM et al., Oncotarget, 2014, 5: 4765-4778). While induction of autophagy may inhibit early stages of cancer development, inhibition of autophagy is expected to combat cancer cells at the stages where cancers are diagnosed in patients. Thus, the inhibition of autophagy is a new source for innovative drugs for the treatment of cancer. The inhibition of autophagy is also actively considered for combination treatments in future cancer therapies.

In order to identify pharmacological inhibitors of selective autophagy, previous drug discovery approaches have phenotypically screened libraries of compounds, for example, by following the cellular localization of GFP-LC3 molecules (Peppard JV et al., Curr Chem Genomics Transl Med. 2014; 8 (Suppl-1): 3-15). Also, standard targeted approaches are the inhibition of groups of proteins that participate in autophagy and where the groups are validated druggable targets, like protein kinases and PI3-kinases (the Ser/Thr protein kinase ULK1, and the PI3-kinase family member Vsp34). Alternatively, it may be possible to target selected autophagy by targeting other proteins involved in the process. However, there is a significant risk for the development of drugs to groups of proteins which are not known to be targeted by small compounds. LC3 homologs have been crystallized and the complex with the docking sequence LIR has also been solved. Molecules that displace the binding of LIR from LC3 can be expected to inhibit selective autophagy. Previous chemical biology research using NMR technology identified low affinity small compounds binding to LC3, with affinities in the millimolar range. More recent work investigated the possibility to displace the interaction between ATG8 and ATG3 from homologs from Plasmodium falciparum, with the assumption that the interaction site would be significantly different from that of humans (Hain AUP et al., Journal of Med.Chem., 2014, 57: 4521-4531). The authors described the development of a surface Plasmon Resonance interaction displacement assay between the ATG8 and ATG3 homologs Plasmodium falciparum, tested 200 compounds and identified hits that displace the interaction in vitro and have anti-parasitic effects. The authors developed a compound that displaces the interaction in their SPR assay with an IC50: 2,8 µM. However, there is no evidence that the compounds displacing the ATG3-ATG8 interaction in cells are the cause of cytotoxicity to P. falciparum. The potential of those compounds to bind to human LC3 has not been investigated.

WO 2012/049 521 discloses aminocoumarins and their use in anticancer treatment. Further aminocoumarins comprising a sugar moiety are disclosed in DE 10 2010 055 566 as potential antibiotics. Neither of the documents mentions an activity of the compounds as autophagy inhibitors.

There is still an unmet need for compounds that inhibit autophagy for cancer treatment, e.g., by means of inhibiting the formation of autophagosomes.

Surprisingly, it has now been found that small molecule compounds inhibiting LC3 proteins and, thus, the LC3/GABARAP interaction with p62 and other autophagy receptors are suitible for inhibiting autophagia. Thereby, these compounds open new routes for the treatment of disorders and/or diseases, in particular cancer, associated with autophagy.

More specifically, it has now been found that certain derivatives of 1,2,3,4-tetra-hydro-1,3,5-triazine and certain coumarin derivatives show activity as inhibitors of LC3B, and, thus, act as autophagy inhibitors.

Accordingly, in a first aspect of the invention there is provided a compound which is
a) a tetrahydrotriazine derivative of the formula (I), 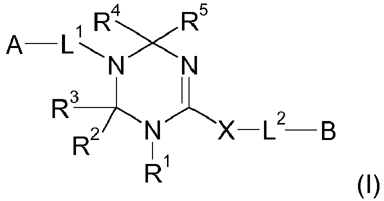 a tautomer, a pharmaceutically acceptable salt, a solvate or hydrate thereof, or
b) a coumarin derivative of the formula (II), 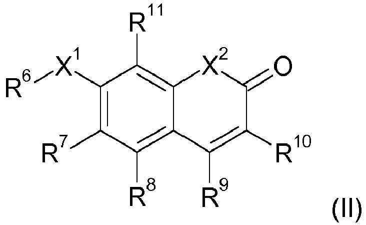 or a tautomer, a pharmaceutically acceptable salt, solvate or hydrate thereof, for use in a method for treating or preventing a pathologic condition associated with a pathologic cell or tissue having upregulated autophagic activity, wherein in formula (I) the symbols have the following meanings:
   - A: is 3-pyridyl, 3-fluorophenyl, 3,4-difluorophenyl or 4-fluorophenyl;
   - L¹: is CH₂, CH(CH₃), CHSiR^{a}₃, CH(halogen), C(halogen)₂, CO, CS, C₂-C₄-alkylene, which is unsubstituted or substituted with one or more halogen, with =O or with =S, or is a bond;
   - X: is S, SO, SO₂ or O;
   - L²: is CH₂, CH(CH₃), CH(halogen), C(halogen)₂, C₂-C₄-alkylene, which is unsubstituted or substituted with one or more halogen, or is a bond;
   - R¹: is H, C₁-C₈-alkyl, benzyl or phenyl, wherein alkyl is unsubstituted or substituted with one or more halogen and wherein benzyl and phenyl is unsubstituted or substituted with one or more substituents selected from halogen and C₁-C₄-alkyl;
   - R², R³, R⁴, R⁵: are each independently H or C₁-C₄-alkyl or R² and R³ and/or R³ and R⁴ together are O or S;
   - each R^{a}: is independently halogen, C₁-C₆-alkyl, C₁-C₆-haloalkyl, C₁-C₆-alkoxyalkyl, C₂-C₆-alkenyl, C₂-C₆-haloalkenyl, C₂-C₆-alkynyl, C₂-C₆-halo-alkynyl, C₃-C₈-cycloalkyl, C₃-C₈-halocycloalkyl, C₁-C₆-haloalkoxyalkyl, phenyl or a 3-, 4-, 5-, 6- or 7-membered saturated, partially unsaturated or aromatic heterocyclic ring containing 1, 2 or 3 heteroatoms or heteroatom groups selected from N, O, S, NO, SO, SO₂;
   - B: is C₆-C₁₀-aryl, 5- or 6-membered heteroaryl, C₅-C₈-cycloalkyl, 5 to 7 membered heterocyclyl, C₁-C₈-alkyl, which groups are unsubstituted or substituted with one or more substituents R^{b}, or B is H;
   - each R^{b}: is independently halogen, CN, N₃, OCN, NCO, CNO, SCN, NCS, C₁-C₆ alkyl, C₃-C₇ cycloalkyl, C₃-C₇ heterocyclyl, C₂-C₆ alkenyl, C₂-C₆ alkynyl, C₃-C₇ aryl, C₃-C₇ heteroaryl, C₄-C₁₅ aralkyl, C₄-C₁₅ heteroarylalkyl, C₄-C₁₅ cycloalkylalkyl, C4-C15 heterocyclylalkyl, C(O)R^{bb}, C(O)OR^{bb}, C(O)N(R^{bb}₂), C(O)N(R^{bb})OR^{bb}, C(O)N(R^{bb})N(R^{bb}₂), C(NR^{bb})N(R^{bb}₂), C(NR^{bb})N(R^{bb})OR^{bb}, C(NR^{bb})N(R^{bb})N(R^{bb}₂), C(R^{bb})N(R^{bb}), C(NR^{bb}₂)NOR^{bb}, C(NRb^{b}2)NOC(O)R^{bb}, C(R^{bb})NN(R^{bb}₂), CR^{bb}NOR^{bb}, OR^{bb} , OC(O)R^{bb}, OC(O)N(R^{bb}₂), SR^{bb}, S(O)R^{bb}, S(O)₂R^{bb}, S(O)₂OR^{bb}, S(O)₂N(R^{bb}₂), S(O)N(R^{bb}₂), S(O)₂N(R^{bb})C(O)N(R^{bb}₂), N(R^{bb})S(O)₂N(R^{bb}₂), N(R^{bb})S(O)₂R^{bb}, N(^{bb})S(O)R^{bb}, N(R^{bb})S(O)₂OR^{bb}, N(R^{bb}₂), N(R^{bb})C(O)R^{bb}, N(R^{bb})C(O)N(R^{bb}₂), or N(R^{bb})C(O)OR^{bb}, wherein alkyl, cycloalkyl, heterocyclyl, alkenyl, alkynyl, aryl, heteroaryl, aralkyl, and heteroarylalkyl are unsubstituted or substituted with one or more substituents R^{B};
   - each: R^{bb} is independently H, C₁-C₆ alkyl, C₃-C₇ cycloalkyl, C₃-C₇ heterocyclyl, C₂-C₆ alkenyl, C₂-C₆ alkynyl, C₃-C₇ aryl, C₃-C₇ heteroaryl, C₄-C₁₅ aralkyl or C₄-C₁₅ heteroarylalkyl which are unsubstituted or substituted with one or more R^{B};
   - each: R^{B} is independently halogen, CN, OH, C₁-C₆ alkyl, OR^{BB}, C(O)R^{BB}, C(O)OR^{BB}, C(O)N(R^{BB}₂), N(R^{BB}₂), OC(O)R^{BB}, N(R^{BB})C(O)R^{BB}, SR^{BB}, S(O)R^{BB}, S(O)₂R^{BB}, S(O)₂OR^{BB}, S(O)₂N(R^{BB}₂), S(O)N(R^{BB}₂), S(O)₂N(R^{BB})C(O)N(R^{BB}₂), N(R^{BB})S(O)₂N(R^{BB}₂), N(R^{BB})S(O)₂R^{BB}, N(R^{BB})S(O)R^{BB}, N(R^{B})C(O)N(R^{BB}₂), N(R^{BB})C(O)OR^{BB}, and OC(O)N(R^{BB}₂), wherein C₁-C₆ alkyl is unsubstituted or substituted with one or more R';
   - each R^{BB}: is independently H or C₁-C₆ alkyl, wherein C₁-C₆ alkyl is unsubstituted or substituted with one or more halogen;
   - each R': is independently halogen, CN, OH, OR", C(O)R", C(O)OR", C(O)N(R"₂), N(R"₂), OC(O)R", N(R")C(O) R", SR", S(O)R", S(O)₂R", S(O)₂OR", S(O)₂N(R"₂), S(O)N(R"₂), S(O)₂N(R")C(O)N(R"₂), N(R")S(O)₂N(R"₂), N(R")S(O)₂R", N(R")S(O)R", N(R")C(O)N(R"₂), N(R")C(O)O R" or OC(O)N(R"₂) and
   - each R": is independently H or C₁-C₆ alkyl, wherein C₁-C₆ alkyl is unsubstituted or substituted with one or more halogen;
and wherein in formula (II) the symbols have the following meanings:
- X¹: is O, S, SO, SO₂ or NR^{x}, R^{x}NSO₂∼, where ∼ denotes the bond to the coumarin scaffold;
- X²: is O or NR^{x};
- R^{x}: is H, CH₃ or C₂H₅;
- R⁶: is L³ - D;
- L³: is a bond, C₁-C₆-alkylene, wherein one CH₂-group not linked to X¹ can be replaced by NR^{x}; O, S, SO, SO₂, CO, CO-O or O-CO-;
- D: is H, an optionally derivatized sugar moiety, C₆-C₁₂-aryl, mono or bicyclic 3 to 9 membered hetaryl, C₃-C₈-cycloalkyl or mono or bicyclic 3 to 9 membered heterocyclyl, wherein the aryl, hetaryl, cycloalkyl or heterocyclyl group is unsubstituted or substituted with one or more substituents R^{c};
- each R^{c}: is independently halogen, CN, N₃, OCN, NCO, CNO, SCN, NCS, C₁-C₆ alkyl, C₃-C₇ cycloalkyl, C₃-C₇ heterocyclyl, C₂-C₆ alkenyl, C₂-C₆ alkynyl, C₃-C₇ aryl, C₃-C₇ heteroaryl, C₄-C₁₅ aralkyl, C₄-C₁₅ heteroarylalkyl, C(O)R^{cc}, C(O)OR^{cc}, C(O)N(R^{cc}₂), C(O)N(R^{cc})OR^{cc}, C(O)N(R^{cc})N(R^{cc}₂), C(NR^{cc})N(R^{cc}₂), C(NR^{cc})N(R^{cc})OR^{cc}, C(NR^{cc})N(R^{cc})N(R^{cc}₂), C(R^{cc})N(R^{cc}), C(NR^{cc}₂)NOR^{cc}, C(NR^{cc}₂)NOC(O)R^{cc}, C(R^{cc})NN(R^{cc}₂), CR^{cc}NOR^{cc}, OR^{cc}, OC(O)R^{cc}, OC(O)N(R^{cc}₂), SR^{cc}, S(O)R^{cc}, S(O)₂R^{cc}, S(O)₂OR^{cc}, S(O)₂N(R^{cc}₂), S(O)N(R^{cc}₂), S(O)₂N(R^{cc})C(O)N(R^{cc}₂), N(R^{cc})S(O)₂N(R^{cc}₂), N(R^{cc})S(O)₂R^{cc}, N(^{cc})S(O)R^{cc}, N(R^{cc})S(O)₂OR^{cc}, N(R^{cc}₂), N(R^{cc})C(O)R^{cc}, N(R^{cc})C(O)N(R^{cc}₂), or N(R^{cc})C(O)OR^{cc}, wherein alkyl, cycloalkyl, heterocyclyl, alkenyl, alkynyl, aryl, heteroaryl, aralkyl, and heteroarylalkyl are unsubstituted or substituted with one or more substituents R^{c};
- each R^{cc}: is independently H, C₁-C₆ alkyl, C₃-C₇ cycloalkyl, C₃-C₇ heterocyclyl, C₂-C₆ alkenyl, C₂-C₆ alkynyl, C₃-C₇ aryl, C₃-C₇ heteroaryl, C₄-C₁₅ aralkyl or C₄-C₁₅ heteroarylalkyl which are unsubstituted or substituted with one or more R^{C};
- each R^{C}: is independently halogen, CN, OH, C₁-C₆ alkyl, OR^{CC}C(O)R^{CC}, C(O)OR^{CC}, C(O)N(R^{CC}₂), N(R^{CC}₂), OC(O)R^{CC}, N(R^{CC})C(O)R^{CC}, SR^{CC}, S(O)R^{CC}, S(O)₂R^{CC}, S(O)₂OR^{CC}, S(O)₂N(R^{CC}₂), S(O)N(R^{CC}₂), S(O)₂N(R^{CC})C(O)N(R^{CC}₂), N(R^{CC})S(O)₂N(R^{CC}₂), N(R^{CC})S(O)₂R^{CC}, N(R^{CC})S(O)R^{CC}, N(R^{CC})C(O)N(R^{CC}₂), N(R^{CC})C(O)OR^{CC} or OC(O)N(R^{CC}₂), wherein C₁-C₆ alkyl is unsubstituted or substituted with one or more R';
- each R^{CC}: is independently H or C₁-C₆ alkyl, wherein C₁-C₆ alkyl is unsubstituted or substituted with one or more halogen;
- each: R' is independently halogen, CN, OH, OR", C(O)R", C(O)OR", C(O)N(R"₂), N(R"₂), OC(O)R", N(R")C(O) R", SR", S(O)R", S(O)₂ R", S(O)₂OR", S(O)₂N(R"₂)₃ S(O)N(R"₂), S(O)₂N(R")C(O)N(R"₂), N(R")S(O)₂N(R"₂), N(R")S(O)₂R", N(R")S(O)R", N(R")C(O)N(R"₂), N(R")C(O)OR" or OC(O)N(R"₂) and
- each R": is independently H or C₁-C₆ alkyl, wherein C₁-C₆ alkyl is unsubstituted or substituted with one or more halogen;
- R⁹: is OH, halogen, O-C₁-C₆-alkyl or O-benzyl, wherein alkyl or benzyl are unsubstituted or substituted with one or more halogen;
- R¹⁰: is L⁴ - E;
- L⁴: is -NR^{DD}-C(O)-, CO-NR^{DD}, SO₂-NR^{DD} or NR^{DD}-SO₂;
- E: is a residue of an α-amino acid, wherein the amino group optionally carries a protective group; C₆-C₁₀-aryl, 5- or 6-membered heteroaryl, C₅-C₈-cycloalkyl, 5 to 7 membered heterocyclyl, C₁-C₈-alkyl, which groups are unsubstituted or substituted with one or more substituents R^{d};
- or L⁴-E: together with R⁹ forms an oxazole group condensed to the coumarin scaffold;
- each R^{d}: is independently halogen, CN, N₃, OCN, NCO, CNO, SCN, NCS, C₁-C₆ alkyl, C₃-C₇ cycloalkyl, C₃-C₇ heterocyclyl, C₂-C₆ alkenyl, C₂-C₆ alkynyl, C₃-C₇ aryl, C₃-C₇ heteroaryl, C₄-C₁₅ aralkyl, C₄-C₁₅ heteroarylalkyl, C(O)R^{dd}, C(O)OR^{dd}, C(O)N(R^{dd}₂), C(O)N(R^{dd})OR^{dd}, C(O)N(R^{dd})N(R^{dd}₂), C(NR^{dd})N(R^{dd}₂), C(NR^{dd})N(R^{dd})OR^{dd}, C(NR^{dd})N(R^{dd})N(R^{dd}₂), C(R^{dd})N(R^{dd}), C(NR^{dd}₂)NOR^{dd}, C(NR^{dd}₂)NOC(O)R^{dd}, C(R^{dd})NN(R^{dd}₂), CR^{dd}NOR^{dd}, OR^{dd}, OC(O)R^{dd}, OC(O)N(R^{dd}₂), SR^{dd}, S(O)R^{dd}, S(O)₂R^{dd}, S(O)₂OR^{bb}, S(O)₂N(R^{dd}₂), S(O)N(R^{dd}₂), S(O)₂N(R^{dd})C(O)N(R^{dd}₂), N(R^{dd})S(O)₂N(R^{dd}₂), N(R^{dd})S(O)₂R^{dd} , N(^{dd})S(O)R^{dd}, N(R^{dd})S(O)₂OR^{dd} , N(R^{dd}₂), N(R^{dd})C(O)R^{dd}, N(R^{dd})C(O)N(R^{dd}₂), or N(R^{dd})C(O)OR^{dd}, wherein alkyl, cycloalkyl, heterocyclyl, alkenyl, alkynyl, aryl, heteroaryl, aralkyl, and heteroarylalkyl are unsubstituted or substituted with one or more substituents R^{D};
- each R^{dd}: is independently H, C₁-C₆ alkyl, C₃-C₇ cycloalkyl, C₃-C₇ heterocyclyl, C₂-C₆ alkenyl, C₂-C₆ alkynyl, C₃-C₇ aryl, C₃-C₇ heteroaryl, C₄-C₁₅ aralkyl or C₄-C₁₅ heteroarylalkyl which are unsubstituted or substituted with one or more R^{D};
- each R^{D}: is independently halogen, CN, OH, C₁-C₆ alkyl, OR^{DD}, C(O)R^{DD}, C(O)OR^{DD}, C(O)N(R^{DD}₂), N(R^{DD}₂), OC(O)R^{DD}, N(R^{DD})C(O)R^{DD}, SR^{DD}, S(O)R^{DD}, S(O)₂R^{DD}, S(O)₂OR^{DD}, S(O)₂N(R^{DD}₂), S(O)N(R^{DD}₂), S(O)₂N(R^{DD})C(O)N(R^{DD}₂), N(R^{DD})S(O)₂N(R^{DD}₂), N(R^{DD})S(O)₂R^{DD}, N(R^{DD})S(O)R^{DD}, N(R^{DD})C(O)N(R^{DD}₂), N(R^{DD})C(O)OR^{DD}, and OC(O)N(RD^{D2}), wherein C₁-C₆ alkyl is unsubstituted or substituted with one or more R';
- each R^{DD}: is independently H or C₁-C₆ alkyl, wherein C₁-C₆ alkyl is unsubstituted or substituted with one or more halogen;
- each R': is independently halogen, CN, OH, OR", C(O)R", C(O)OR", C(O)N(R"₂), N(R"₂), OC(O)R", N(R")C(O) R", SR", S(O)R", S(O)₂R", S(O)₂OR", S(O)₂N(R"₂), S(O)N(R"₂), S(O)₂N(R")C(O)N(R"₂), N(R")S(O)₂N(R"₂), N(R")S(O)₂R", N(R")S(O)R", N(R")C(O)N(R"₂), N(R")C(O)O R" or OC(O)N(R"₂) and
- each R": is independently H or C₁-C₆ alkyl, wherein C₁-C₆ alkyl is unsubstituted or substituted with one or more halogen;
- R⁷: is H, CH₃, halogen or NO₂;
- R⁸: is H, CH₃, halogen or NO₂ and
- R¹¹: is CH₃, halogen or H.

In one preferred embodiment, R⁶ in formula (II) is not a sugar moiety.

In other words, the present invention relates to a method for treating or preventing a pathologic condition in a patient associated with a pathologic cell or tissue having upregulated autophagic activity, comprising the step of administering to the patient a therapeutically effective amount of a compound of formula (I) and/or a compound of formula (II), a tautomer, pharmaceutically acceptable salt, solvate or hydrate of either, preferably wherein R⁶ in formula (II) is not a sugar moiety.

The method of treating or preventing of the present invention is preferably a therapeutic method.

A preferred embodiment of the present invention refers to a compound of formula (I) and/or a compound of formula (II) or a tautomer, a pharmaceutically acceptable salt, a solvate or hydrate of any of the aforementioned for use in a method for treating a pathologic condition associated with a pathologic cell or tissue having upregulated autophagic activity.

Alternatively or additionally, the present invention refers to a compound of formula (I) or a compound of formula (II) or a tautomer, a pharmaceutically acceptable salt, a solvate or hydrate of any of the aforementioned for use in a method for preventing a pathologic condition associated with a pathologic cell or tissue having upregulated autophagic activity.

As used herein, the term "treating" may be understood in the broadest sense as any means for improving the health status of the patient suffering from a pathologic condition associated with a pathologic cell or tissue having upregulated autophagic activity. Preferably, such treating includes
(i) a partly or entire removal of the pathologic cell(s) or tissue(s) having upregulated autophagic activity from the patient's body;
(ii) a partly or entire curing of said pathologic cell(s) or tissue(s) in the patient's body in that these cells bear reduced autophagic activity in comparison to the untreated cells, and/or
(iii) a partly or entire arrest in proliferation of said pathologic cell(s) or tissue(s) in the patient's body.

As used herein, the term "preventing" may be understood in the broadest sense as any means for decreasing the risk of a patient of developing a pathologic condition associated with a pathologic cell or tissue having upregulated autophagic activity. Exemplarily, such risk may be decreased by at least 10%, at least 20%, at least 30%, at least 40%, at least 50%, at least 60%, at least 70%, at least 80%, at least 90%, at least 95% or even entirely.

As used herein, the term "partly" may be understood in that the designated effect, (such as, e.g., a decrease) accounts for below 100%, but preferably at least 10%, at least 20%, at least 30%, at least 40%, at least 50%, at least 60%, at least 70%, at least 80%, at least 90%, or at least 95%.As used herein, the term "entirely" may be understood in that the designated effect (such as, e.g., a decrease) accounts for (nearly) 100%.

A pathologic condition may be understood in the broadest sense as any non-healthy status of a patient, i.e., any health status deviating from a status considered as healthy. Preferably, a pathologic condition refers to a disease, in particular a disease enlisted in the International Classification of Diseases No. 10 (ICD-10) of the World Health Organization (WHO). Preferably, the pathologic condition of the present invention is a neoplastic disease. In general, a neoplastic disease may be malignant neoplastic disease or a benign neoplastic disease. More preferably, the pathologic condition of the present invention is a malignant neoplastic disease. In particular, the pathologic condition of the present invention is cancer. Several preferred types of cancer are provided below.

According to the present invention, the pathologic condition is associated with a pathologic cell or tissue having upregulated autophagic activity. Therefore, a pathologic cell of the present invention is preferably a cell forming part of neoplastic tissue and/or being suitible for developing neoplastic tissue. In particular, the pathologic cell of the present invention is a cancer cell. Accordingly, a pathologic tissue of the present invention is preferably a neoplastic tissue. Optionally, neoplastic tissue may also be innervated by blood vessels. Preferably, neoplastic tissue is malignant neoplastic tissue. In particular, the pathologic tissue of the present invention is a cancer tissue.

Preferably, the pathologic cell or tissue is subjected to stress such as, e.g., undersupply of nutrients and/or oxygen (optionally caused by a neoplastic/cancer bulk), chemical stress (e.g., antineoplastic/anticancer agents), genotoxic stress, oxidative stress and/or accumulated protein aggregates.

According to the present invention, the pathologic cell or tissue has upregulated autophagic activity. Having upregulated autophagic activity may be understood in the broadest sense as having a higher autophagic activity than cells/tissues of the same type (preferably also obtained from the same patient) which are healthy, i.e., not bearing the pathologic condition of the present invention. In other words, the ratio (a):(b) between (a) the autophagic activity of the pathologic cells/tissues and (b) the autophagic activity of comparable healthy cells/tissues (typically of the same cell/tissue type, preferably also from the same patient), the ratio (a):(b) is >1. The person skilled in the art is well aware of several means for determining autophagic activity. Some examples of well-suitible means are provided in the example section below. Exemplarily, microscopic means may be used, such as, e.g., those based on determining the amount of lipidation of MAP1 LC3B (LC3B) (lipidated LC3B-II) and LC3B-positive punctae (also: autophagic vesicles). LC3B is also usable as autophagy marker. Exemplarily, there are commercial LC3 antibodies commercially available. One quantitative example of determining the autophagic activity is using the LC3B IF/WB assay as explained in detail below. Such upregulation of autophagic activity may optionally be due to the overexpression/upregulated expression of pro-autophagic proteins such as, e.g., LC3B and/or an increased activity of pro-autophagic proteins such as, e.g., LC3B. In particular, in the context of the present invention, upregulated autophagic activity is associated with upregulation of LC3B, more in particular (i) increased amounts/cellular concentrations of lipidated LC3B-II, (ii) increased occurance of LC3B-positive punctae (also: autophagic vesicles) and/or (iii) increased ratios between non-lipidated (cytosiolic) LC3B-II and lipidated LC3B-I (ratio LC3B-II:LC3B-I).

Preferably, the autophagic activity (also: autophagy or autophagia) is selective autophagic activity (also: selective autophagy or selective autophagia) is autophagic activity that is not mainly for catabolic needs but rather associated with role in enabling cells, tissues and organs to respond to different cellular demands in a highly regulated, selective fashion also occurring under nutrient-rich conditions.

According to a particularly preferred embodiment, the present invention refers to a compound of formula (I) or a compound of formula (II) or a tautomer, a pharmaceutically acceptable salt, a solvate or hydrate of any of the aforementioned for use in a method for treating or preventing cancer associated with a pathologic cancer cell or tissue having upregulated autophagic activity, wherein said pathologic cancer cell or tissue is subjected to stress.

Preferably, the compound of formula (I) or a compound of formula (II) or a tautomer, a pharmaceutically acceptable salt, a solvate or hydrate of any of the aforementioned inhibits the autophagic activity in a cell or tissue and/or in a cell-free system. As mentioned herein, the person skilled in the art is well aware of several means for determining autophagic activity, exemplarily those provided in the example section below.

More preferably, the compound of formula (I) or a compound of formula (II) or a tautomer, a pharmaceutically acceptable salt, a solvate or hydrate of any of the aforementioned inhibits the activity of at least one MAP1 LC3 protein (LC3), such as LC3A, LC3B and/or LC3C. In particular, the compound of formula (I) or a compound of formula (II) or a tautomer, a pharmaceutically acceptable salt, a solvate or hydrate of any of the aforementioned inhibits the activity of MAP1 LC3B (LC3B). In this context, the inhibiting effect may occur in a cell-free system, in cell or tissue culture and/or in a cell and/or tissue in a patient.

Particularly preferably, the compound of formula (I) or a compound of formula (II) or a tautomer, a pharmaceutically acceptable salt, a solvate or hydrate of any of the aforementioned inhibits the activity of LC3B with an IC50 in the nanomolar or micromolar range. Preferably, the IC50 is not higher than 200 µM, more preferably not higher than 100 µM, 90 µM, 80 µM, 70 µM, 60 µM, 50 µM, 40 µM, 30 µM, 20 µM or even not higher than 10 µM or lower. Optionally, these values may be determined by the assay provided below. Examples are also depicted in the Examples below.

Preferably, in a cell-based assay, the compound of formula (I) or a compound of formula (II) or a tautomer, a pharmaceutically acceptable salt, a solvate or hydrate of any of the aforementioned leads to a at least 1.1 fold, more preferably at least 1.2fold, 1.3fold, 1.4fold or even higher increase of LC3B spots in the cells treated with 100 µM of said compounds for 1h. The fold increase in LC3B spots was determined relative to a comparative DMSO control. The exact procedure is depicted below. Therefore, the compound of formula (I) or a compound of formula (II) or a tautomer, a pharmaceutically acceptable salt, a solvate or hydrate of any of the aforementioned leads to a cellular increase of LC3B spots of at least 10%, more preferably at least 20%, at least 30%, at least 40% or higher. As described herein, an increase of LC3B spots indicates a decrease (i.e., inhibiting effect) of autophagic activity.

The compound of formula (I) or a compound of formula (II) or a tautomer, a pharmaceutically acceptable salt, a solvate or hydrate of any of the aforementioned may optionally bind to LC3B covalently and/or competitively with a dissociation constant (Kd) of not more than 100 µM, not more than 50 µM, not more than 10 µM or even not more than 1 µM or less.

Exemplarily, the compound of formula (I) or a compound of formula (II) or a tautomer, a pharmaceutically acceptable salt, a solvate or hydrate of any of the aforementioned may inhibit the interaction of LC3B with SQSTM1 (p62). In particular, the compound of formula (I) or a compound of formula (II) or a tautomer, a pharmaceutically acceptable salt, a solvate or hydrate of any of the aforementioned may inhibit the interaction of the LIR domain of LC3B with p62, i.e., displace the binding of LIR from LC3. Thus, said compounds are also suitible to inhibit selective autophagy.

In a further aspect of the invention there is provided a tetrahydrotriazine derivative of formula (I) and/or a coumarin derivative of formula (II) or a tautomer, pharmaceutically acceptable salt, solvate or hydrate of either, for use in a method for treating cancer in a patient, with the proviso that R⁶ in formula (II) is not a sugar moiety.

In yet a further aspect of the invention there is provided a tetrahydrotriazine derivative of formula (I) a tautomer, pharmaceutically acceptable salt, solvate or hydrate of either, excluding compounds (I-1) and (1-2) below.

In yet a further aspect of the invention there is provided a coumarin derivative of formula (IIa), a tautomer, pharmaceutically acceptable salt, solvate or hydrate of either, with the proviso that R⁶ in formula (II) is not a sugar moiety.

In yet a further aspect of the invention there is provided a pharmaceutical composition, comprising
a) a tetrahydrotriazine derivative of formula (I) and/or a derivative of formula (II) or a tautomer, pharmaceutically acceptable coumarin salt, solvate or hydrate of either
and
b) a pharmaceutically acceptable carrier,
with the proviso that R⁸ in formula (II) is not a sugar moiety.

In yet a further aspect of the invention there is provided a method for treating cancer in a patient comprising the step of administering to the patient a therapeutically effective amount of a tetrahydrotriazine derivative of formula (I) and/or a coumarin derivative of formula (II), a tautomer, pharmaceutically acceptable salt, solvate or hydrate of either, with the proviso that R⁸ in formula (II) is not a sugar moiety

In yet a further aspect of the invention there is provided the use of a tetrahydrotriazine derivative of formula (I) and/or a coumarin derivative of formula (II) or a tautomer, pharmaceutically acceptable salt, solvate or hydrate of either for the preparation of a medicament for treating cancer, with the proviso that R⁶ in formula (II) is not a sugar moiety

In yet a further aspect of the invention there is provided a method of inhibiting autophagia in a cell culture or tissue culture *in vitro,* comprising the step of administering to said cell culture or tissue culture an effective amount of a tetrahydrotriazine derivative of formula (I) and/or a coumarin derivative of formula (II), a tautomer, pharmaceutically acceptable salt, solvate or hydrate of either.

In a preferred embodiment, the cell culture or tissue culture as used in this context is a cancer the cell culture or cancer tissue culture. This may optionally include primary cells and/or cell lines and, optionally, one or more additional extracellular matrices.

In yet a further aspect of the invention there is provided a compound inhibiting an LC3/GABARAP interaction with p62 for use in a method for treating cancer in a patient, in particular wherein said compound is defined as defined herein.

The LC3B inhibitors of the invention are particularly useful in reducing resistance to treatment with other anti-cancer drugs, and thus in combination with such anti-cancer drugs.

According to the invention the solvates, hydrates, and/or pharmaceutically acceptable salts of all compounds of formulae (I) and (II) as well as of the preferred embodiments described below are included herein.

Further included are tautomers of the compounds of formulae (I) and (II), in particular the following tautomers of the tetrahydrotriazine compounds of formula (I) where B is H and L² is a bond:

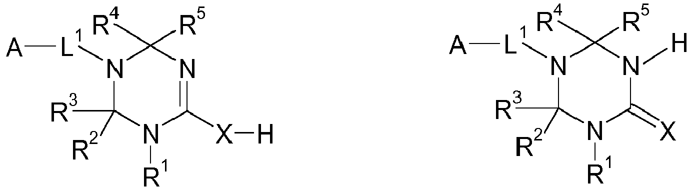

Some of the compounds of the invention and/or salts thereof will exist in different stereoisomeric forms. All of these forms are subjects of the invention.

Described below are exemplary salts of the compounds according to the invention which are included herein. The list of the different salts stated below is not meant to be complete and limiting.

Compounds according to the invention which contain one or more acidic groups can be used according to the invention, e.g. as their alkali metal salts, alkaline earth metal salts or ammonium salts. More precise examples of such salts include sodium salts, potassium salts, calcium salts, magnesium salts or salts with ammonia or organic amines such as, e.g. ethylamine, ethanolamine, triethanolamine or amino acids.

Compounds according to the invention which contain one or more basic groups, i.e. groups which can be protonated, can be used according to the invention in the form of their addition salts with inorganic or organic acids.

Examples for suitable acids include hydrogen chloride, hydrogen bromide, phosphoric acid, sulfuric acid, nitric acid, methanesulfonic acid, p-toluenesulfonic acid, napthalenedisulfonic acid, oxalic acid, acetic acid, tartaric acid, lactic acid, salicylic acid, benzoic acid, formic acid, propionic acid, pivalic acid, diethylacetic acid, malonic acid, succinic acid, pimelic acid, fumaric acid, maleic acid, malic acid, sulfamic acid, phenylpropionic acid, gluconic acid, ascorbic acid, isonicotinic acid, citric acid, adipic acid and other acids known to a person skilled in the art.

Compounds according to the invention which contain several basic groups can simultaneously form different salts.

If a compound according to the invention simultaneously contains acidic and basic groups in the molecule, the invention also includes, in addition to the salt forms mentioned, inner salts or betaines.

The respective salts of the compounds according to the invention can be obtained by customary methods which are known to the person skilled in the art, for example by contacting these with an organic or inorganic acid or base in a solvent or dispersant, or by anion exchange or cation exchange with other salts.

The term "pharmaceutically acceptable" means approved by a regulatory agency such as the EMA (Europe) and/or the FDA (US) and/or any other national regulatory agency for use in animals, preferably in humans.

Furthermore, the invention includes all salts of the compounds according to the invention which, owing to low physiological compatibility, are not directly suitable for use in pharmaceuticals but which can be used, for example, as intermediates for chemical reactions or for the preparation of pharmaceutically acceptable salts or which might be suitable for studying autophagy inhibition, in particular LC3B inhibition, of a compound according of the invention in any suitable manner, such as any suitable in vitro assay.

The present invention furthermore includes all solvates of the compounds according to the invention.

The present invention furthermore includes prodrugs (including the salts thereof) of the compounds according to the invention which contain physiologically tolerable and cleavable groups and which are metabolized in animals, preferably mammals, most preferably humans into a compound according to the invention. Such prodrugs and methods to obtain them are described, e.g., in Nature Drug Discovery Review 2008, 7, 255-270 and in B. Testa and J.M. Mayer, Hydrolyses in Drug and Prodrug Metabolism, Wiley - VCH 2003.

The present invention furthermore includes the metabolites of the compounds according to the invention.

The term "metabolites" refers to all molecules derived from any of the compounds according to the invention in a cell or organism, preferably mammal.

Preferably the term "metabolites" relates to molecules which differ from any molecule which is present in any such cell or organism under physiological conditions.

In any embodiment of the invention one or more tetrahydrotiazine derivates (I), one or more coumarin derivates (II) or a mixture of one or more tetrahydrotiazine derivates (I) and one or more coumarin derivates (II) can be used.

The structure of the metabolites of the compounds according to the invention will be obvious to any person skilled in the art, using the various appropriate methods.

In the tetrahydrotriazine derivatives of formula (I) and the coumarin derivatives of formula (II) as well as the preferred embodiments the following terms have the meanings indicated below.

"Alkyl" means a linear or branched saturated aliphatic hydrocarbon group with the indicated number of carbon atoms, e.g. methyl, ethyl, n-propyl, i-propyl, n-butyl, i-butyl, n-pentyl, i-pentyl, n-hexyl, i-hexyl and 3-methyl-pentyl and the like.

"Alkylene" means a linear or branched saturated alkanediyl group, e.g., ethylene, propylene and the like.

"Alkenyl" means a linear or branched unsaturated aliphatic hydrocarbon group with at least one double bond, and with the indicated number of carbon atoms, e.g. ethenyl, propenyl (allyl), 1-butenyl, 2-butenyl, 1-pentenyl, 2-pentenyl, 1-hexenyl, 2-hexenyl and 3-hexenyl and the like.

"Alkynyl" means a linear or branched unsaturated aliphatic hydrocarbon group with at least one triple bond, and with the indicated number of carbon atoms, e.g. ethinyl, propinyl, 1-butinyl, 2-butinyl and the like.

"Cycloalkyl" means a saturated 3 to 8-membered hydrocarbon ring with the indicated number of carbon atoms, e.g. cyclopropyl, cyclobutyl, cyclopentyl, cyclohexyl, cycloheptyl or cyclooctyl.

"Heterocyclyl" and "heterocyclic" mean a saturated or unsaturated but not aromatic 3- to 8-membered mono or bicyclic hydrocarbon ring containing 1 to 4 heteroatoms and/or heteratom containing groups selected from the group constisting of N, O, S and C(O) in the ring. The one or more heteroatoms and hetero atoms containing groups present in the ring replace a -CH₂- group or a -CH= in the ring. Preferred C₃-C₈ heterocyclyl groups containing 1 to 4 heteroatoms and heteroatom containing groups selected from the group consisting of N, O, S and C(O) are derived from the following heterocyclic compounds: tetrahydrofurane, pyrrolidine, tetrahydrothiophene, oxazolidine, piperidine, tetrahydropyrane, piperazine, dioxane, morpholine, cyclopentanone and trioxane.

"Aryl" and "aromatic" mean an aromatic 3- to 10-membered hydrocarbon ring e.g. phenyl, naphthyl, and the like, as well as 6 to 12 membered bicyclic systems containing an aromatic ring such as tetraline.

"Heteroaryl" and "heteroaromatic" mean an aromatic 3- to 9-membered, mono or bicyclic hydrocarbon ring containing 1 to 4 heteroatoms selected from the group N, O and S in the ring. The one or more heteroatoms present in the ring replace a -CH= group in the ring. Preferred C₃-C₇ heteroaryl groups containing 1 to 4 heteroatoms selected from the group N, O and S are derived from the following heteroaromatic compounds: pyrrole, pyrazole, imidazole, triazole, tetrazole, furane, thiophene, oxazole, isoxazole, oxadiazole, thiazole, isothiazole, thiadiazole, pyridine, pyridazine, pyrimidine, pyrazine, triazine and indole.

"Aralkyl" means a C₃-C₇ aryl group substituted with at least one C₁-C₈ alkyl group, wherein aryl and alkyl are defined as above. Examples for aralkyl are tolyl, benzyl and xylyl. The aralkyl group may be bound via the C₁-C₈ alkyl group or via the C₃-C₇ aryl group.

"Heteroarylalkyl" means a C₃-C₇ heteroaryl group substituted with at least one C₁-C₈ alkyl group, wherein heteroaryl and alkyl are defined as above. The heteroarylalkyl group may be bound via the C₁-C₈ alkyl group or via the C₃-C₇ heteroaryl group.

Cycloalkylalkyl or heterocyclylalkyl means a C₃-C₇ alkyl group or heterocyclyl group substituted with at least one C₁-C₈ alkyl group, both defined as above. The groups may be bound through the alkyl group or through the cycloalkyl or heterocyclyl group.

### "Halogen" means F, Cl, Br and I.

"Residue of an α-amino acid" means the group attached to the carboxylic group of the α-amino acid, e.g. -CH(NH₂)-CH₂-CH(CH₃)₂ for leucine, -CH(NH₂)-CH₂-C₆H₅-for phenylalanine or -CH(NH₂)-(CH₂)₂-C₆H₅ for homophenylalanine. Preferred are natural α-amino acids (leucine, phenylalanine, alanine, valine, methionine, proline, tryptophane, tyrosine, threonine, glutamine, glycine, serine, cysteine, asparagine, lysine, arginine, histidine, glutamic acid and aspartic acid) and homophenylalanine. Leucine, phenylalanine and homophenylalanine are especially preferred.

"Protective group" in connection with an amino group means any suitable protective group for amines, preferably t-butylcarbonyl (BOC), carbobenzyloxy (Cbz), p-methoxybenzylcarbonyl (Moz), 9-fluorenylmethoxycarbonyl (FMOC), acetyl (Ac), benzoyl (Bz), benzyl (Bn), carbamate, p-methoxybenzyl (PMB), 3,4-dimethoxybenzyl (DMPM), p-methoxyphenyl (PMP), tosyl (Ts) or a sulphonamide like Nosyl or Nps. BOC is especially preferred.

"Sugar moiety" preferably means a sugar as defined in WO 2012/049521, i.e. sugars including monosaccharides, disaccharides, and polysaccharides, substances derived from the monosaccharides by oxidation of one or more terminal groups to carboxylic acid and substrates derived from monosaccharides by replacement of one or more hydroxyl groups by a hydrogen atom, an amino group, a thiol group or similar heteroatomic groups.

In one embodiment, the sugar is a monosaccharide. Monosaccharides are polyhydroxy aldehydes (H-(CHOH)n)-CHO) or polyhydroxyl ketones (H- (CHOH)n-CO-(CHOH)m-H) with three (those), four (terose), five (pentose), six (hexose), seven (heptose) or more carbon atoms. The monosaccharide may have a terminal, i.e. aldehydic, (potential) carbonyl group (aldose) or a nonterminal, i.e. ketonic, (potential) carbonyl group (ketose). The monosaccharides have more than one (potential) carbonyl group, i.e. may be a daildose, diketose or aldoketose. A potential carbonyl group is a hemiacetal group that arises from the formation of a ring structure.

The monosaccharide may form a ring structure and be a cyclic hemiacetal or hemiketal. Cyclic forms include oxiroses (C₃), Oxetoses (C₄), furanoses (C₅) pyranoses (C₆), septanoses (C₇) and octanoses (C₈). The position at which the ring closes may vary.

Monosaccharides may also be modified at various positions, such modifications include, but are not limited to, substitution of an alcoholic hydroxyl group with hydrogen (deoxy sugar), substitution of an alcoholic hydroxyl group or a ring oxygen with an amino group (amino sugar), substitution of a hydroxyl by thiol or a ring oxygen with sulphur (thiosugar), substitution with selenium (selenosugar), substitution of a ring carbon with nitrogen (azasugar), substation of a aldehydic group with a carboxyl group (aldonic acid), substitution of a carbonyl group with an alcoholic group (aldonic acid or ketoaldonic acid) and oxidizing an aldoxe or aldose derivative to a carboxyl group (uronic acid).

Suitable monosaccharides include, but are not limited to, arabinose, ribose, ribulose, xylose, xyulose, lyxose, allose, altrose, glucose (Glc), N-acetylglucosamine (GlcNAc), N-acetylgalacotosamine (GalNAc), fructose (Frc), galactose (Gal), fucose (Fuc), gulose, idose, mannose (Man), sorbose, talose, tagatose, sialic acid, glucuronoic acid and iduronic acid. Preferred monosaccharides are glucose, galactose and mannose. More preferred monosaccharides are glucose and galactose.

In another embodiment, the sugars comprise a disaccharide. Disaccharides may be derived from the combination of any two of the monosaccharides described above. Suitable disaccharides include, but a not limited to, sucrose (Sue), lactose (Lac), maltose (Mai), isomaltose (Isomal), trehalose (Tre) and cellobiose.

Further examples of suitable sugars are given in WO 2006/003456.

Glucose and glactose are particularly preferred.

"Derivatized" in connection with a sugar moiety preferably means that in OH groups hydrogen is substituted by a group common in sugar chemistry, preferably a protecting-group, more preferred carbamoyl (-CO-NH₂), acetyl (Ac), methyl, benzoyl (Bz), benzyl (Bn), β-methoxyethoxymethyl (MEM), dimethoxytrityl (DMT), methoxymethyl (MOM), methoxytrityl (MMT), p-methoxybenzyl (PMB), methylthiomethyl, pivaloyl (Piv), tetrahydropyranyl /THP), tetrahydrofuran (THF), trityl (Tr), silyl ether, such as trimethylsilyl and ethoxy ethyl. Carbamoyl, methyl and acetyl are particularly preferred.

In one preferred embodiment the compound of the invention is a tetrahydrotriazine derivative (I)

Preferred are tetrahydrotriazine derivatives (I) wherein one or more of the symbols have the following meanings.
A is preferably 3-pyridyl, 3-fluorophenyl, 3,4-difluorophenyl or 4-fluorophenyl.
X is S, SO or SO₂.
L¹ is preferably -CH₂-, -CH(CH₃)-, trimethylsilyl, triethylsilyl or tert.-butyldimethylsilyl.
L² is - preferably CH₂-, CH(CH₃)-, -CH₂-CH₂-, -CH(CH₂)-CH₂-, -CH₂-CH(CH₃)- or a bond.
R¹ is preferably H or methyl.
R², R³, R⁴ and R⁵ are preferably H or
R² and R³ together and/or R⁴ and R⁵ together are =O.
B is preferably C₁-C₈-aryl, 5- or 6-membered heteroaryl, C₅-C₆-cycloalkyl, 5 to 6 membered heterocyclyl, C₁-C₆-alkyl, which groups are unsubstituted or substituted with one or more substituents R^{b}, or B is H.
Each R^{b} independently is preferably halogen, preferably F, Cl, Br, CN, N₃, OCN, NCO, CNO, SCN, NCS, C₁-C₆ alkyl, C₃-C₆ cycloalkyl, C₃-C₇ heterocyclyl, C₂-C₆ alkenyl, C₂-C₆ alkynyl, phenyl, C₅-C₆ heteroaryl, benzyl, C₆-C₁₄ heteroarylalkyl, C₄-C₁₀ cycloalkylalkyl,
   C(O)R^{bb}, C(O)OR^{bb}, C(O)N(R^{bb}₂), C(O)N(R^{bb})OR^{bb}, C(O)N(R^{bb})N(R^{bb}₂), C(NR^{bb})N(R^{bb}₂), C(NR^{bb})N(R^{bb})OR^{bb}, C(NR^{bb})N(R^{bb})N(R^{bb}₂), C(R^{bb})N(R^{bb}), C(NR^{bb}₂)NOR^{bb}, C(NR^{bb}₂)NOC(O)R^{bb}, C(R^{bb})NN(R^{bb}₂), CR^{bb}NOR^{bb}, OR^{bb}, OC(O)R^{bb}, OC(O)N(R^{bb}₂), SR^{bb}, S(O)R^{bb}, S(O)₂R^{bb}, S(O)₂OR^{bb}, S(O)₂N(R^{bb}₂), S(O)N(R^{bb}2), S(O)₂N(R^{bb})C(O)N(R^{bb}₂), N(R^{bb})S(O)₂N(R^{bb}₂), N(R^{bb})S(O)₂R^{bb}, N(bb)S(O)R^{bb}, N(R^{bb})S(O)₂OR^{bb}, N(R^{bb}₂), N(R^{bb})C(O)R^{bb}, N(R^{bb})C(O)N(Rbb²), or N(R^{bb})C(O)OR^{bb}, wherein alkyl, cycloalkyl, heterocyclyl, alkenyl, alkynyl, phenyl, heteroaryl, benzyl, and heteroarylalkyl are unsubstituted or substituted with one or more substituents R^{B}.
Each R^{bb} independently is preferably H, C₁-C₆ alkyl, C₃-C₇ cycloalkyl, C₃-C₇ heterocyclyl, C₂-C₆ alkenyl, C₂-C₆ alkynyl, C₃-C₇ aryl, C₃-C₇ heteroaryl, C₄-C₁₅ aralkyl or C₄-C₁₅ heteroarylalkyl which are unsubstituted or substituted with one or more R^{B}.
Each R^{B} independently is preferably halogen, CN, OH, C₁-C₆ alkyl, OR^{BB}, C(O)R^{BB}, C(O)OR^{BB}, C(O)N(R^{BB}₂), N(R^{BB}₂), OC(O)R^{BB}, N(R^{BB})C(O)R^{BB}, SR^{BB}, S(O)R^{BB}, S(O)₂R^{BB}, S(O)₂OR^{BB}, S(O)₂N(R^{BB}₂), S(O)N(R^{BB}₂), S(O)₂N(R^{BB})C(O)N(R^{BB}₂), N(R^{BB})S(O)₂N(R^{BB}₂), N(R^{BB})S(O)₂R^{BB}, N(R^{BB})S(O)R^{BB}, N(R^{BB})C(O)N(R^{BB}₂), N(R^{BB})C(O)OR^{BB}, and OC(O)N(R^{BB}₂), wherein C₁-C₆ alkyl is unsubstituted or substituted with one or more substituents R'.
Each R^{BB} independently is preferably H or C₁-C₆ alkyl, wherein C₁-C₆ alkyl is unsubstituted or substituted with one or more halogen.
Each R' independently is preferably halogen, CN, OH, OR", C(O)R", C(O)OR", C(O)N(R"₂), N(R"₂), OC(O)R", N(R")C(O)R", SR", S(O)R", S(O)₂R", S(O)₂OR", S(O)₂N(R"₂), S(O)N(R"₂), S(O)₂N(R")C(O)N(R"₂), N(R")S(O)₂N(R"₂), N(R")S(O)₂R", N(R")S(O)R", N(R")C(O)N(R"₂), N(R")C(O)OR" or OC(O)N(R"2.
Each R" independently is preferably H or C₁-C₆ alkyl, wherein C₁-C₆ alkyl is unsubstituted or substituted with one or more halogen.

Preferred are also tetrahydrotriazine derivatives (I) wherein all of the symbols have the preferred meanings.

More preferred are tetrahydrotriazine derivatives (I) wherein one or more of the symbols have the following meanings.
- A: is more preferably 3-pyridyl, 3-fluorophenyl, 3,4-difluorophenyl or 4-fluorophenyl;
- X: is more preferably S, SO or SO₂.
- L¹: is more preferably CH₂ or CH(CH₃).
- L²: is more preferably CH₂.
- B: is more preferably phenyl or 5 or 6 membered hetero aryl, which groups are unsubstituted or substituted with one or more, preferably one or two, more preferably one substituent R^{b}.
- Each of R¹, R², R³, R⁴ and R⁵: is H or
- R² and R³: together and/or R⁴ and R⁵ together are =O.
- R^{b}: is more preferably halogen, preferably F, Cl or Br, CN, OH, C₁-C₄-alkyl, C₃-C₆ cycloalkyl, C₄-C₈ cyclolakylalkyl, preferably methyl, C₁-C₄-haloalkyl, preferably CF₃, C₁-C₄-alkoxy, preferably methoxy, C₁-C₄-haloalkoxy, preferably OCF₃, COOH, COO-R^{bb}, CONH₂, CONHR^{bb}, CONR^{bb}₂, phenyl or phenoxy, where the last two groups are unsubstituted or substituted with one or more halogen, preferably F, Cl or Br, CN, OH, C₁-C₄-alkyl, preferably methyl, C₁-C₄-haloalkyl, preferably CF₃, C₁-C₄-alkoxy, preferably methoxy, C₁-C₄-haloalkoxy, preferably OCF₃, COOH, COO-R^{bb}, CONH₂, CONHR^{bb} or CONR^{bb}₂.
- R^{bb}: is more preferably C₁-C₆-alkyl, benzyl or phenyl, which groups are unsubstituted or substituted with one or more halogen or a substituent R^{B}.
- Each R^{B}: independently is more preferably COOH or COO-C₁-C₆-alkyl.

More preferred are also tetrahydrotriazine derivatives (I) wherein all of the symbols in formula (I) have the more preferred meanings.

Particularly preferred are tetrahydrotriazine derivatives (I) wherein one or more of the symbols have the following meanings.
- A: is particularly preferably 3-pyridyl, 3-fluorophenyl, 3,4-difluorophenyl or 4-fluorophenyl.
- X: is particularly preferably S, SO or SO₂.
- L¹: is particularly preferably CH₂.
- L²: is particularly preferably CH₂.
- Each of R¹, R², R³, R⁴ and R⁵: is particularly preferably H.
- B: is particularly preferably phenyl which is unsubstituted or substituted with one or more, preferably one or two, more preferably one substituent R^{b}, where one substituent R^{b} is preferably in the para position with respect to L².
- Each R^{b}: independently is particularly preferably F, Cl, Br, CN, OH, C₁-C₄-alkyl, C₁-C₄-haloalkyl, C₁-C₄-alkoxy, C₁-C₄-haloalkoxy, COOH, COOR^{bb}, CONH₂, CONHR^{bb}, CONR^{bb}₂, phenyl, phenoxy, where the last two groups are unsubstituted or substituted with one or more halogen, preferably F, Cl or Br, CN, OH, C₁-C₄-alkyl, preferably methyl, C₁-C₄-haloalkyl, preferably CF₃, C₁-C₄-alkoxy, preferably methoxy, C₁-C₄-haloalkoxy, preferably OCF₃, COOH, COO-R^{bb}, CONH₂, CONHR^{bb} or CONR^{bb}₂.
- Each R^{bb}: independently is particularly preferably C₁-C₆-alkyl, benzyl or phenyl.

Particularly preferred are also tetrahydrotriazine derivatives (I) wherein all of the symbols have the particularly preferred meanings.

Further preferred are tetrahydrotriazine derivatives of the following formulae (Ia) to (Io), where the symbols have the above indicated preferred, more preferred and particularly preferred meanings:

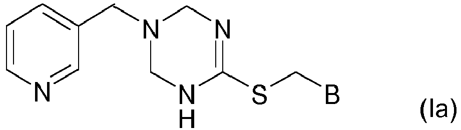

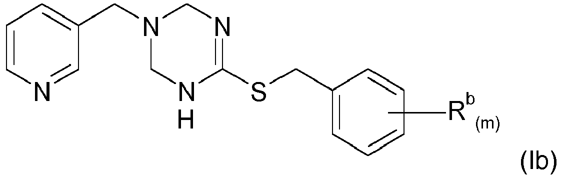

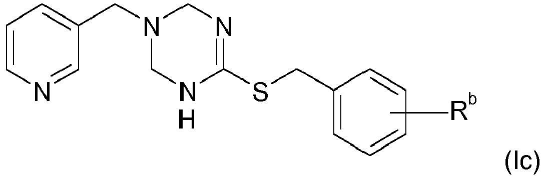

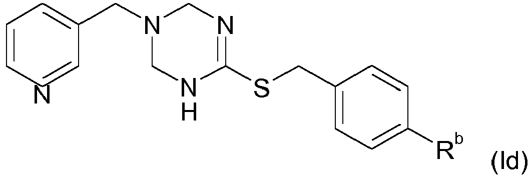

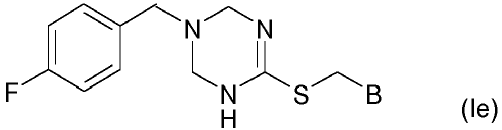

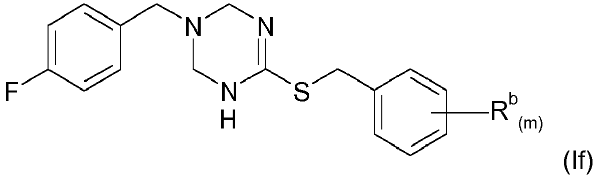

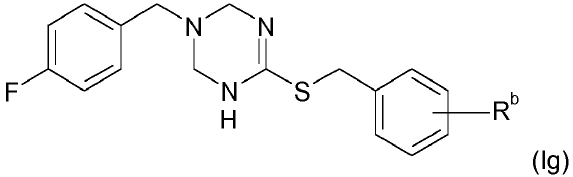

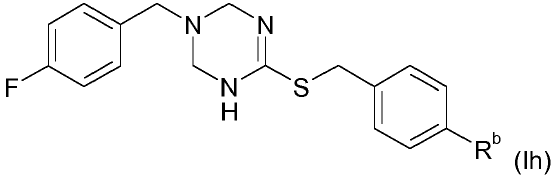

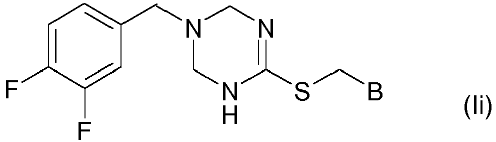

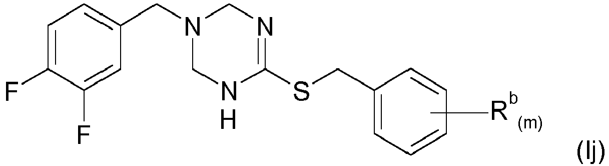

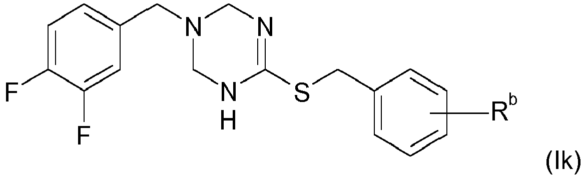

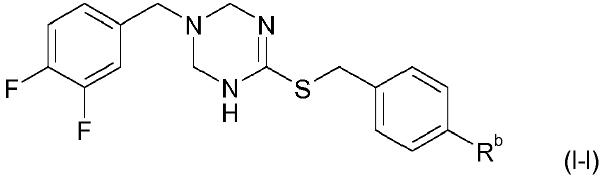

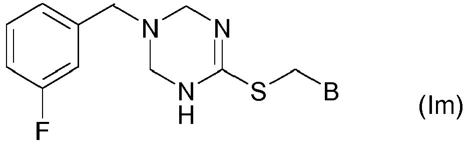

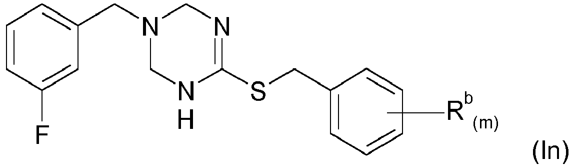

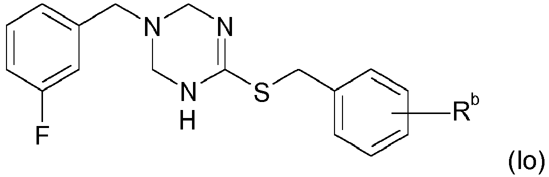

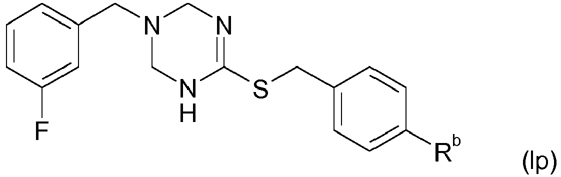

Further preferred tetrahydrotriazine derivatives of formula (Ih) are listed in Table I.

**Table I Tetrahydrotriazine derivatives of formula (Ih)**

| | | | |
|---|---|---|---|
| 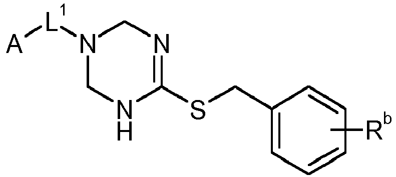 | | | |

| Compound | L¹ | A | R^{b} |
|---|---|---|---|
| I-1 | CH₂ | 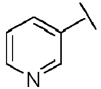 | p-Br |
| I-2 | CH₂ | 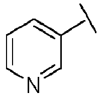 | p-Cl |
| I-3 | CH₂ | 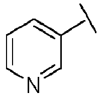 | p-F |
| I-4 | CH₂ | 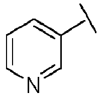 | H |
| I-5 | CH₂ | 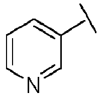 | p-CF₃ |
| I-6 | CH₂ | 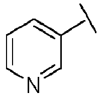 | p-OCH₃ |
| I-7 | CH₂ | 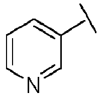 | 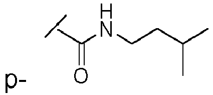 |
| I-8 | CH₂ | 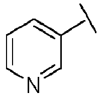 | m- Br |
| I-9 | CH₂ | 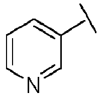 | m- OCH₃ |
| I-10 | CH₂ | 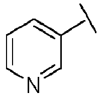 | 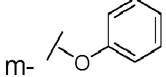 |
| I-11 | CH₂ | 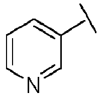 | 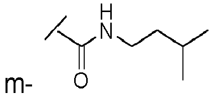 |
| I-12 | CH₂ | 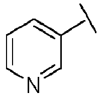 | 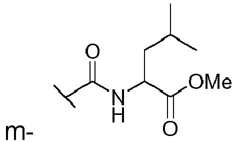 |
| I-13 | CHCH₃ | 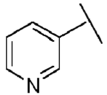 | p- Br |
| I-14 | CH₂ | 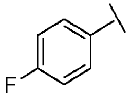 | p- Br |
| I-15 | CH₂ | 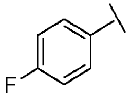 | p-Cl |
| I-16 | CH₂ | 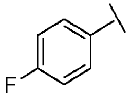 | 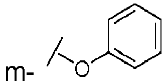 |
| I-17 | CH₂ | 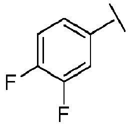 | p-Br |
| I-18 | CH₂ | 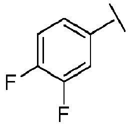 | 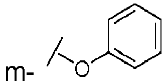 |
| I-19 | CH₂ | 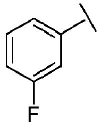 | p-Br |
| I-20 | CH₂ | 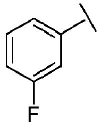 | 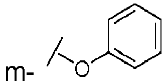 |

| | | | |
|---|---|---|---|
| p: para substitution; m: meta substitution | | | |

Further preferred are the tetrahydrotriazine derivatives (I) shown in the following Scheme 1:

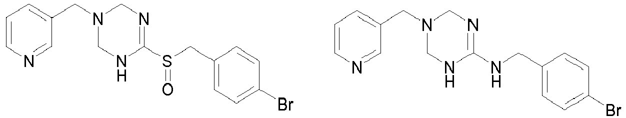

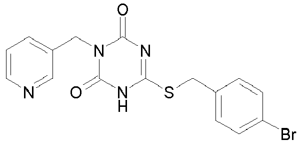

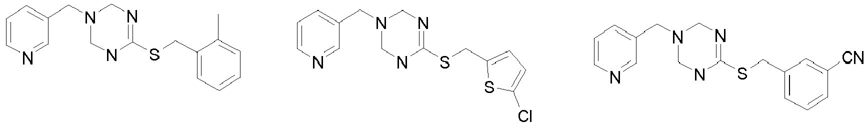

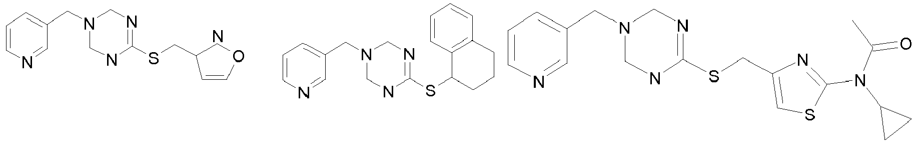

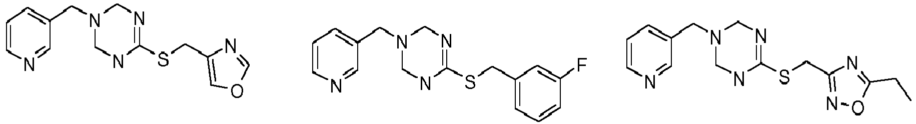

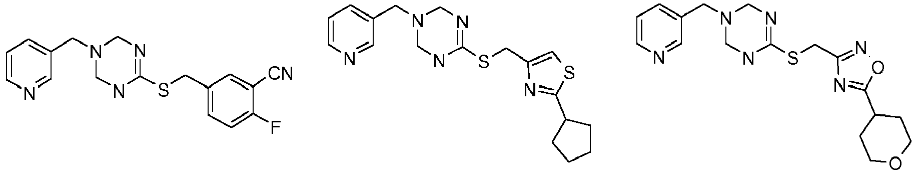

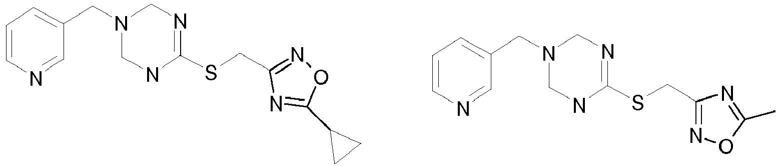

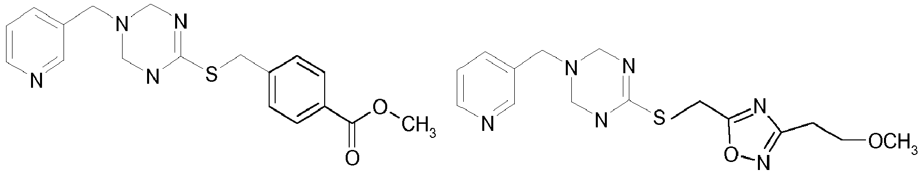

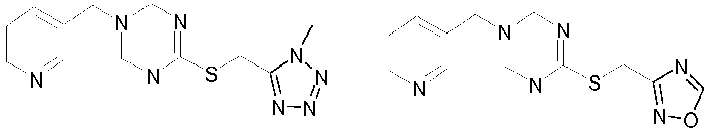

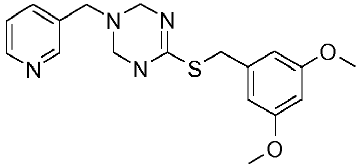

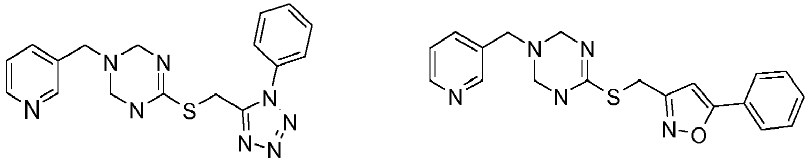

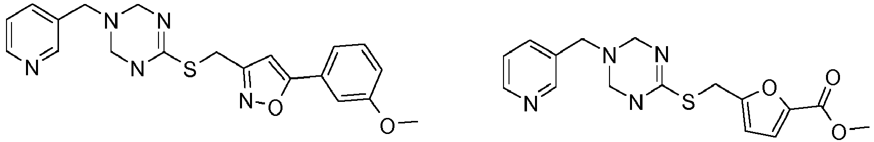

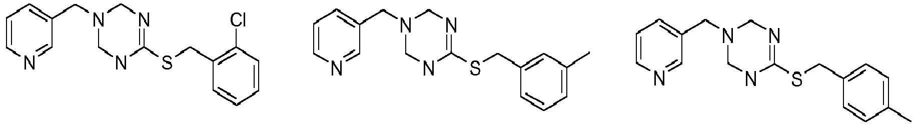

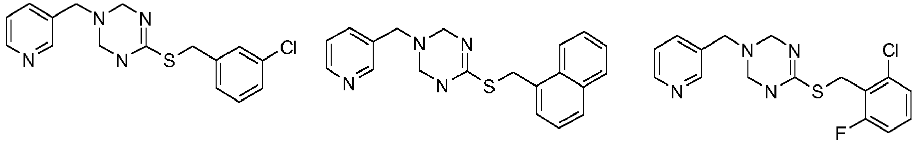

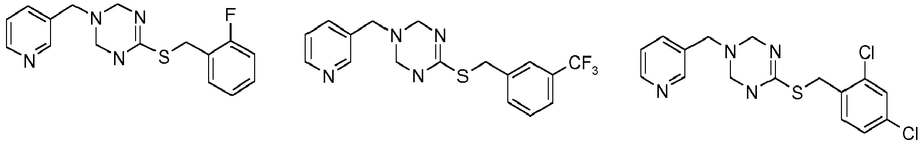

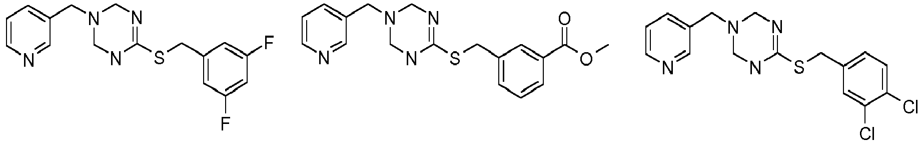

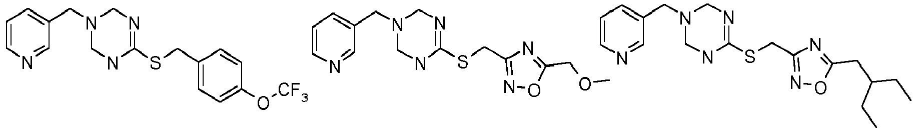

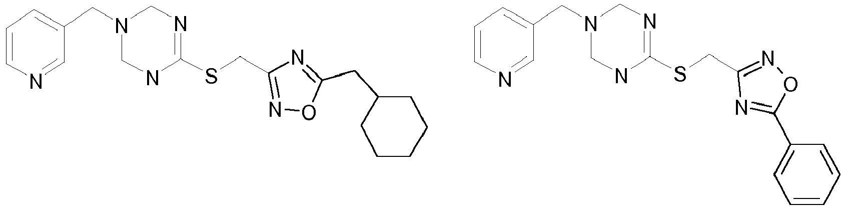

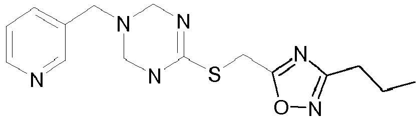

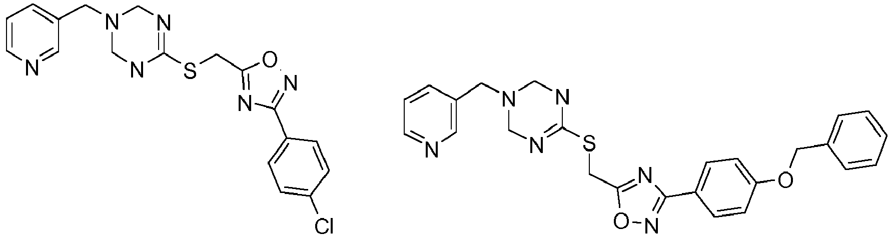

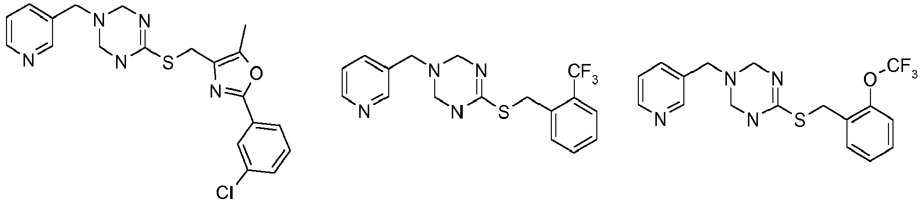

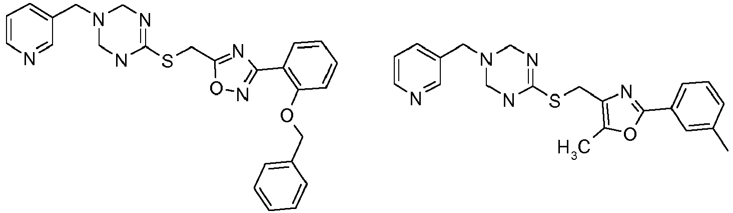

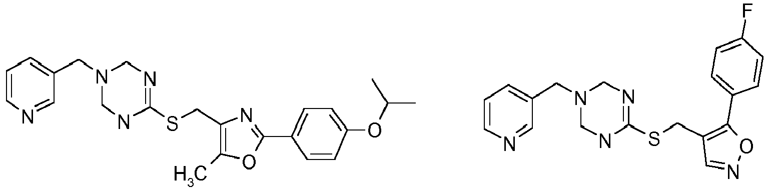

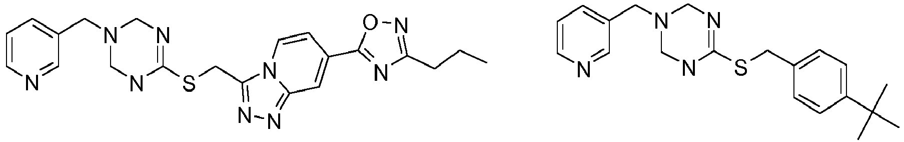

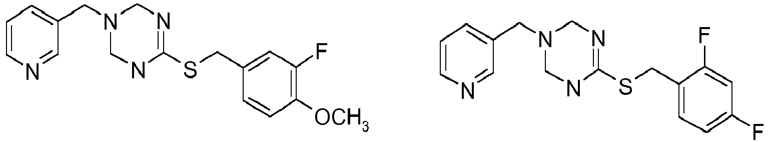

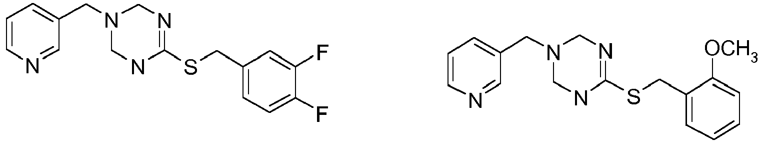

In another preferred embodiment the compound of the invention is a coumarin derivative (II).

Preferred are coumarin derivatives of formula (II) where D is not a sugar moiety.

In one preferred embodiment of the coumarin derivative (II) L⁴ is NR^{DD}-SO₂ or SO₂NR^{DD}.

In another preferred embodiment of the coumarin derivative (II) L⁴ is NR^{DD}-C(O) or C(O)-NR^{DD}.

In another preferred embodiment of the coumarin derivative (II) L³ is a bond, C₁-C₆-alkylene, wherein one CH₂-group not linked to X¹ can be replaced by NR'; O, S, SO, SO₂, CO, CO-O or O-CO- and
D is H, C₆-C₁₂-aryl, mono or bicyclic 3 to 9 membered hetaryl, C₃-C₈-cycloalkyl or mono or bicyclic 3 to 9 membered heterocyclyl, wherein the aryl, hetaryl, cycloalkyl or heterocyclyl group is unsubstituted or substituted with one or more substituents R^{c}.

In further preferred, more preferred and particularly preferred embodiments the group R^{c}, R^{cc}, R^{C}, R^{CC}, R' and R" and R^{d}, R^{dd}, R^{D}, R^{DD}, R' and R" in formula (II) have the preferred, more preferred, and particularly preferred meanings of the corresponding groups R^{b}, R^{bb}, R^{B}, R^{BB}, R' and R" in formula (I).

### Further preferred are the coumarin derivatives listed in Table 2

**Table 2 Coumarin Derivatives (II)**

| | | | |
|---|---|---|---|
| 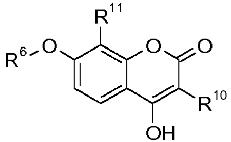 | | | |

| Compound | R⁶ | R¹¹ | R¹⁰ |
|---|---|---|---|
| 2-0 | 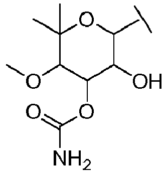 | CH₃ | 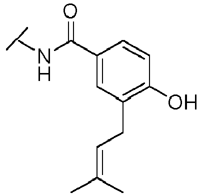 |
| 2-1 | H | CH₃ | 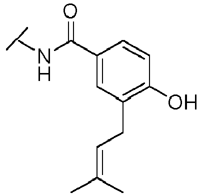 |
| 2-2 | 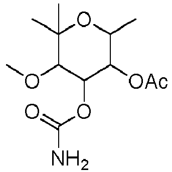 | CH₃ | 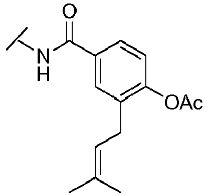 |
| 2-3 | 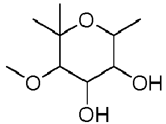 | CH₃ | 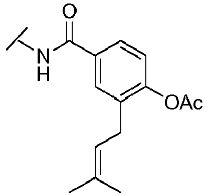 |
| 2-4 | 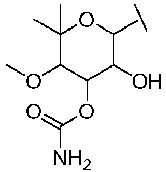 | CH₃ | 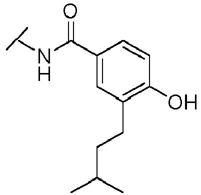 |
| 2-5 | 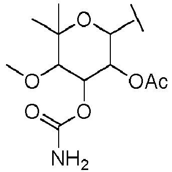 | CH₃ | 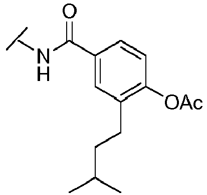 |
| 2-6 | H | CH₃ | 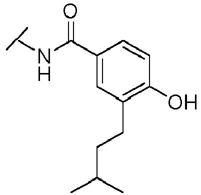 |
| 2-7 | 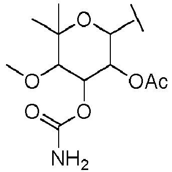 | CH₃ | 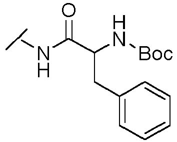 |
| 2-8 | H | CH₃ | 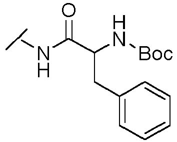 |
| 2-9 | H | CH₃ | 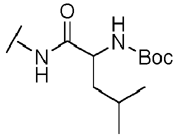 |
| 2-10 | H | CH₃ | 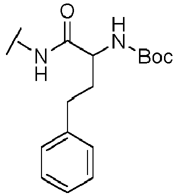 |
| 2-11 | H | CH₃ | 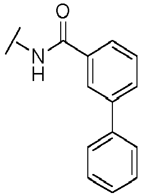 |
| 2-12 | H | CH₃ | 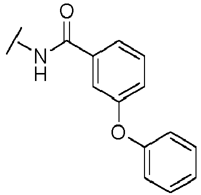 |
| 2-13 | H | H | 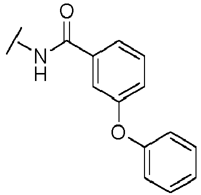 |

### Chemistry

### Tetrahydrotriazine derivatives of formula (I)

For the synthesis of the tetrahydrotriazine derivatives (I) a two-step procedure can be used which is slightly modified from the procedure disclosed in R. Pathania et al., Nature Chemical Biology, 2009, 5 (11), 849-956. It is exemplified in Scheme 2 for the synthesis of tetrahydrotriazine derivatives (I). The first step is the synthesis of the alkylated thiourea derivatives using thiourea and a substituted benzyl-chloride leading to high yielded products 95 % which can usually be used without further purification. The second step is carried out using the corresponding amine, formaldehyde and a substituted thiourea to yield compounds (11-27). 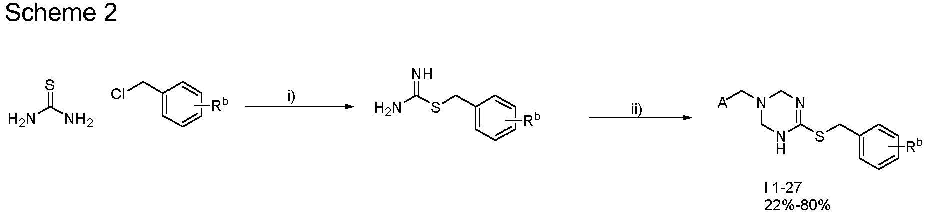 i) EtOH, 80°C 2h, rt 24h ii) formaldehyde, dioxane, 2,5h 70°C, 24h rt

Further methods for the preparation of the tetrahydrotriazine derivatives (I) are known to the person skilled in the art.

Depending on the circumstances of the individual case, in order to avoid side reactions during the synthesis of a compound of the general formula (I) and its preferred embodiments, it can be necessary or advantageous to temporarily block functional groups by introducing protective groups and to deprotect them in a later stage of the synthesis, or to introduce functional groups in the form of precursor groups and at a later stage to convert them into the desired functional groups. Suitable synthetic strategies, protective groups and precursor groups are known to the person skilled in the art.

If desired, the compounds of the formula (I) can be purified by customary purification procedures, for example by recrystallization or chromatography. The starting materials for the preparation of the compounds of the formulae (I) and its preferred embodiments are commercially available or can be prepared according to or analogously to literature procedures.

### Coumarin Derivatives (II)

Coumarin derivatives (II) wherein L³-D is a sugar moiety can be prepared by the methods disclosed in WO 2012/049521 and DE 10 2010 055 566.

For the synthesis of the coumarin derivatives (II) we have developed a number of synthetic strategies is applicable. One example is given in the Scheme 2. The synthesis can be carried out by starting with the alkylation of 2',4'-dihydroxy-3'-methyl-acetophenon using the Finkelstein method followed by a transesterification and a claisen ester condensation yielding intermediate 7-(benzyloxy)-4-hydroxy-8-methyl-2H-chromen-2-one in good yields. The introduction of the group R⁸ could be achieved by using a reaction cascade starting with a common nitration, reduction with H₂ generating the amino group which is coupled in the last step with a carboxylic acid under coupling conditions shown in Scheme 3 yielding the coumarin derivatives with an overall yield of 25%.

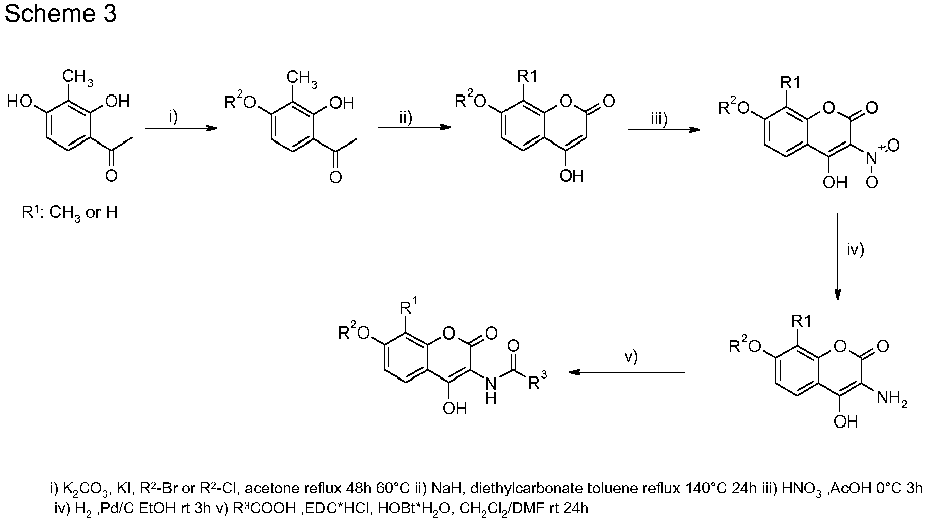

Further methods for the preparation of the coumarin derivatives (II) are known to the person skilled in the art.

Depending on the circumstances of the individual case, in order to avoid side reactions during the synthesis of a coumarin derivative (II) and its preferred embodiments, it can be necessary or advantageous to temporarily block functional groups by introducing protective groups and to deprotect them in a later stage of the synthesis, or to introduce functional groups in the form of precursor groups and at a later stage to convert them into the desired functional groups. Suitable synthetic strategies, protective groups and precursor groups are known to the person skilled in the art.

If desired, the coumarin derivatives (I) can be purified by customary purification procedures, for example by recrystallization or chromatography. The starting materials for the preparation of the compounds of the formulae (I) and its preferred embodiments are commercially available or can be prepared according to or analogously to literature procedures.

In one aspect of the invention there is provided a tetrahydrotriazine derivative of formula (I) or a coumarin derivative of formula (II), a tautomer, pharmaceutically acceptable salt, solvate or hydrate of either for use in a method for treating cancer in a patient.

The cancer varieties which can be treated with the compounds as defined beforehand are mamalion, preferably human malignancies, preferably solid neo-plasias or haematological malignancies.

Various human malignancies are potential targets for autophagy inhibition since many malignancies examined to date are modified by autophagy. Autophagy involvement has been reported in pancreatic epitheloid carbinoma, colon colorectal carcinoma, lung carcinoma, breast cancer, brain cancer, prostate cancer, lung cancer, ovarian cancer, Further the treatment of any cancer susceptible to chemo therapy or signal transduction modulation is feasible.

In a further embodiment, the present invention relates to a tetrahydrotriazine derivative of the formula (I) and/or a coumarin derivative of the formula (II), wherein R⁶ (L³-D) is not a sugar moiety, as defined above in general form or in preferred embodiments for use as medicament.

In a further aspect of the invention there is provided a pharmaceutical composition, comprising
a) a tetrahydrotriazine derivative of formula (I), a coumarin derivative of formula (II), a tautomer, pharmaceutically acceptable salt, solvate or hydrate of either
and
b) a pharmaceutically acceptable carrier,
wherein in the coumarin derivative (II) R⁶ (L³-D) is not a sugar moiety.

The term "pharmaceutical composition" is sometimes referred to as "pharmaceutical" or "medicament" hereinafter or in the prior art. Said terms shall have the same meaning and may be used interchangeably.

A pharmaceutically acceptable carrier as used herein may be understood in the broadest sense as any additive that is pharmaceutically acceptable, therefore, any additive that is non-toxic to the patient. Exemplarily, a pharmaceutically acceptable carrier may comprise or consist of a solvent such as, e.g., water, dimethyl sulfoxide (DMSO), ethanol, vegetable oil, paraffin oil or combinations thereof. Furthermore, a carrier may contain one or more detergent(s), one or more foaming agent(s) (e.g., sodium lauryl sulfate (SLS)/ sodium doceyl sulfate (SDS)), one or more coloring agent(s) (e.g., TiO₂, food coloring), one or more vitamin(s), one or more salt(s) (e.g., sodium, potassium, calcium, zinc salts), one or more humectant(s) (e.g., sorbitol, glycerol, mannitol, propylene glycol, polydextrose), one or more enzyme(s), one or more preserving agent(s) (e.g., benzoic acid, methylparabene), one or more texturing agent(s) (e.g., carboxymethyl cellulose (CMC), polyethylene glycol (PEG), sorbitol), one or more emulsifier(s), one or more bulking agent(s), one or more glacing agent(s), one or more separating agent(s), one or more antioxidant(s), one or more herbal and plant extract(s), one or more stabilizing agent(s), one or more polymer(s) (e.g., hydroxypropyl methacrylamide (HPMA), polyethylene imine (PEI), carboxymethyl cellulose (CMC), polyethylene glycol (PEG)), one or more uptake mediator(s) (e.g., polyethylene imine (PEI), dimethyl sulfoxide (DMSO), a cell-penetrating peptide (CPP), a protein transduction domain (PTD), an antimicrobial peptide, etc.), one or more sweetener(s) (e.g., sucrose, acesulfame K, saccharin Na, stevia), one or more gustatory substance(s) and/or one or more fragrance(s).

The compounds according to the formula (I) and/or (II) act as an inhibitor of autophagy in a cell, preferably a cancer cell, in particular as an inhibitor of LC3B.

"Inhibitor of autophagy" as used herein means an inhibitor of LE3B

"Inhibitor of LE3B" as used herein means a competitive or covalent inhibitor of LE3B.

Competitive inhibitors preferably have IC50s in the low microM range, more preferably in the nanoM range.

A covalent inhibitor is defined as a compound that can efficiently covalently react with LC3B molecules and that can inhibit the interaction under experimental conditions. Whereas low microM-nanoM IC50s are preferred, this is not a requisite for covalent inhibitors. The reason for this is that once the target is covalently modified, this is not reversible: it is blocked in its function. Therefore even if this happens at a very low rate, within pharmaceutically appropriate compounds the preferred drug is one that requires the lowest concentration, bit that also is less reactive over other cellular proteins under the effective conditions towards the target.

The compounds of the present invention may be used in a method of treating or preventing a neoplastic disease, in particular cancer associated with a pathologic regulation of autophagia (in particular pathologic upregulation of autophagic activity). The compounds of the invention are useful in the therapy of neoplasia, in particular cancer.

In the context of the present invention, cancer may be understood in the broadest sense as any malignant neoplastic disease, i.e., the presence of one or more malignant neoplasm(s) in the patient. Cancer may be solid or hematologic malignancy. Exemplarily, cancer may be located on or in the lip, oral cavity and pharynx (ICD-10 classes C00-C14), on or in the digestive organs (ICD-10 classes C15-C26), on or in the respiratory system and intrathoracic organs (ICD-10 classes C30-C39), on or in the bone and articular cartilage (ICD-10 classes C40-C41), on or in the skin (ICD-10 classes C43-C44), on or in the connective and soft tissue (ICD-10 classes C45-C49), on or in the breast and female genital organs (ICD-10 classes C50-C58), on or in the male genital organs (ICD-10 classes C60-C63), on or in the urinary organs (ICD-10 classes C64-C68), on or in the eye, brain and central nervous system (ICD-10 classes C69-C72), on or in the endocrine glands and related structures (ICD-10 classes C73-C75), may be secondary and ill-defined neoplasms (ICD-10 classes C76-C80), may be stated or presumed to be primary, of lymphoid, haematopoietic and related tissue neoplasms (ICD-10 classes C81-C96), and/or may be neoplasms of independent (primary) multiple sites (ICD-10 class C97). Exemplarily, cancer may be selected from the group consisting of carcinoma (i.e., cancers derived from epithelial cells; e.g., adenocarcinoma, squamous cell carcinoma, adenosquamous carcinoma, anaplastic carcinoma, large cell carcinoma, and small cell carcinoma), sarcoma (i.e, cancers derived from connective tissue; e.g., Askin's tumor, sarcoma botryoides, chondrosarcoma, Ewing's sarcoma, malignant hemangioendothelioma, malignant Schwannoma, osteosarcoma, and soft tissue sarcomas), lymphoma and leukemia (i.e., cancers derived from hematopoietic (blood-forming) cells; e.g., mature B-cell neoplasms, mature T cell and natural killer (NK) cell neoplasms, Hodgkin lymphoma, immunodeficiency-associated lymphoproliferative disorders, lymphocytic leukemia, myelogenous leukemia), germ cell tumor (i.e., cancers derived from pluripotent cells in the sexual organs; e.g., germinoma (including dysgerminoma and seminoma), dysgerminoma, seminoma), blastoma (i.e., cancers derived from immature "precursor" cells or embryonic tissue; e.g., hepatoblastoma, medulloblastoma, nephroblastoma, neuroblastoma, pancreatoblastoma, pleuropulmonary blastoma, retinoblastoma, glioblastoma), and melanoma (i.e., cancers derived from melanocytes; e.g., Lentigo maligna (melanoma), superficial spreading melanoma, acral lentiginous melanoma, mucosal melanoma, nodular melanoma, polypoid melanoma, desmoplastic melanoma, amelanotic melanoma, soft-tissue melanoma), and glioma (i.e., cancers derived from brain or spine cells., e.g., ependymoma, astrocytoma, oligodendrogliomas, brainstem glioma, optic nerve glioma, mixed glioma).

In their capacity as inhibitors of autophagy the tetrahydrotriazine derivatives (I) and/or the coumarin derivatives (II) can also be used in combination with further pharmaceuticals, such as one or more antineoplastic agents, preferably one or more anticancer agents.

Preferred for combination therapy are compounds which are able to enhance the effect of the compounds according to the invention. Exemplarily, such antineoplastic agents (in particular anticancer agents) in addition to one or more compounds of the present invention may be selected from the group consisting of antimetabolites (e.g., 5-fluorouracil (5-fluoro-2'-deoxyuridine), hydroxyurea, thioguanine, azathioprine, fludarabine, fludarabine-5'-dihydrogen phosphate, gemcitabine, 6-mercaptopurine, pyrimidines, floxuridine, cytosine arabinoside (cytarabine), pemetrexed, pralatrexate, raltitrexed, and folic acid antagonists such as methotrexate), mitoxantrone, alkylating agents and other DNA-amending agents (e.g., fotemustine, dacarbazine or nitrogen mustard, mechlorethamine, cyclophosphamide, chlorambucil, ifosfamide), platins (e.g., cisplatin, carboplatin, oxaliplatin), plant alkaloids and terpenoids (e.g., vinca alkaloids (vincristine, vinblastine, vinorelbine, vindesine), taxanes (e.g., paclitaxel and derivatives thereof such as 6-α-hydroxy paclitaxel), cytoxan), topoisomerase inhibitors (e.g., camptothecins: irinotecan, topotecan, etoposide (VP-16), etoposide phosphate, teniposide), a statin (e.g., cerivastatin, simvastatin, lovastatin, somatostatin, fluvastatin, nystatin, rosuvastatin, atorvastatin, pravastatin, pitavastatin, pento-statin), COX inhibitor (COX-2 or COX-3 inhibitor), cytokine (e.g., TNF-α, IFN-α, IFN-β, IFN-γ, IL-2, IL-4, IL-12, IL-18, platelet factor-4) or analogs thereof, cytokine and/or cytokinin antagonists, polyclonal or monoclonal antibodies (e.g., rituximab, trastuzumab, cetuximab, bevacizumab, basiliximab, daclizumab), hormones and derivatives thereof (e.g., glucocorticoids or fenretinide, α-estradiol, estriol, estrone, ethinyl estradiol), and hormone antagonists (e.g., tamoxifen, finasteride or LHRH antagonists), kinase inhibitors (e.g., imatinib, imatinib mesylate, gefitinib, erlotinib, pazopanib, apatinib, flavopiridol, staurosporin, STI571 (CPG 57148B) or UCN-01 (7-hydroxystaurosporine), proteasome inhibitors (e.g., bortezomib), PARP inhibitors (e.g., iniparib, olaparib), VEGF inhibitors, IGF-1, warfarin, colcemid, cytochalasin A-E, topoisomerase inhibitors, doxorubicin (adriamycin) (lipo), a mycin (bleomycin, azithromycin, spiramycin, sirolimus (rapamycin), everolimus, pimecrolimus, roxithromycin, ascomycin, bafilomycin, erythromycin, midecamycin, josamycin, concancamycin, clarithromycin, troleandomycin, folimycin, tobramycin, mutamycin, dactinomycin, dactinomycin, rebeccamycin, actinomycin D, mitomycins), protein synthesis inhibitors (e.g., L-asparaginase, cycloheximide, puromycin or diphteria toxin), (viii) microtubule-directed agents (e.g., colcemid, colchicine, paclitaxel (taxol), docetaxel (taxotere), vinblastine or vincristine), trichostatin A, thioplatin, PS-341, phenylbutyrate, ET-18-OCH₃, or farnesyl transferase inhibitors (L-739749, L-744832); polyphenols, quercetin, resveratrol, piceatannol, epigallocatechine gallate, theaflavins, flavanols, procyanidins, betulinic acid and derivatives thereof, demethylating agents (e.g., 5-azacytidine, 5-aza-2'deoxycytidine, 5,6-dihydro-5-azacytidine), other DNA-fragmenting and/or cross-linking agents, other mitosis inhibitors, other intercalating agents and combinations of two or more thereof.

Additionally or alternatively, an antineoplastic therapy can also be supported by means of any other process known for this purpose such as, e.g., radiation therapy (e.g, Intensity-Modulated Radiation Therapy (IMRT), 3-Dimensional Conformal Radiotherapy (3DCRT), Stereotactic body radiation therapy (SBRT), Stereotactic radiosurgery (SRS), image-guided radiation therapy (IGRT), Particle Therapy (e.g, proton therapy), Brachytherapy, and/or Radioisotope Therapy (RIT) (e.g., with iodine-131, lutetium-177, strontium-89 and samarium (153Sm) lexidronam and/or yttrium-90)), (adult and/or embryonal) stem cell transplantation optionally including administration of differentiation inducing agents such as retinoic acid derivatives and/or MIBG-therapy.

In yet a further aspect of the invention there is provided a method for treating cancer in a patient comprising the step of administering to the patient a therapeutically effective amount of a tetrahydrotriazine derivative of formula (I), a coumarin derivative of formula (II), a tautomer, pharmaceutically acceptable salt, solvate or hydrate of either.

The compounds of the formula (I) and/or (II) and their pharmaceutically acceptable salts, optionally in combination with other pharmaceutically active compounds, can be administered to mammals, and in particular to humans, as pharmaceuticals by themselves, in mixtures with one another or in the form of pharmaceutical preparations. Further subjects of the present invention therefore also are the compounds of the formula (I) and/or (II) and their pharmaceutically acceptable salts for use as pharmaceuticals, including the use of the pharmaceuticals as inhibitor of centrosome clustering, to induce multipolar mitoses of tumor cells with supernumerary centrosomes, and to induce apoptosis. They can be used in the therapy and prophylaxis of the above-mentioned diseases and syndromes as well as for preparing pharmaceuticals for these purposes. Furthermore, subjects of the present invention are pharmaceutical preparations (or pharmaceutical compositions), which comprise an effective dose of at least one compound of the formula (I) and/or (II) and/or a pharmaceutically acceptable salt thereof and a pharmaceutically acceptable carrier, i.e. one or more pharmaceutically acceptable carrier substances and/or additives.

The pharmaceuticals according to the invention can be administered orally, for example in the form of pills, tablets, lacquered tablets, sugar-coated tablets, granules, hard and soft gelatin capsules, aqueous, alcoholic or oily solutions, syrups, emulsions or suspensions, or rectally, for example in the form of suppositories. Administration can also be carried out parenterally, for example subcutaneously, intramuscularly or intravenously in the form of solutions for injection or infusion. The preferred administration form depends, for example, on the disease to be treated and on its severity.

Administration may be administration once ((acute) single administration) or may be a repeated administration such as, e.g., administration of repeated pulse doses or chronic administration. Repeated administration may exemplarily be administration two times, three times, four times, five times, six times, seven times, eight times, nine times, ten times, more than ten times or even permanently. Between two administrations, there may be a time interval of less than one hour, one hour or more, six hours or more, twelve hours or more, 24 hours or more. Administration may be daily, may be twice daily, three times daily, four times daily, every second day, every three days, weekly, biweekly, monthly, twice a year or yearly. Clinically viable administration schemes may be determined by the person skilled in the art based on balancing efficacy and toxicity.

The preparation of the pharmaceutical preparations can be carried out in a manner known per se. To this end, one or more compounds of the formula (I) and/or (II) and/or their pharmaceutically acceptable salts, together with one or more solid or liquid pharmaceutical carrier substances and/or additives (or auxiliary substances) and, if desired, in combination with other pharmaceutically active compounds having therapeutic or prophylactic action, are brought into a suitable administration form or dosage form, which can then be used as a pharmaceutical in human or veterinary medicine.

For the production of pills, tablets, sugar-coated tablets and hard gelatin capsules it is possible to use, for example, lactose, starch, for example maize starch, or starch derivatives, talc, stearic acid or its salts, etc. Carriers for soft gelatin capsules and suppositories are, for example, fats, waxes, semisolid and liquid polyols, natural or hardened oils, etc. Suitable carriers for the preparation of solutions, for example of solutions for injection, or of emulsions or syrups are, for example, water, physiologically sodium chloride solution, alcohols such as ethanol, glycerol, polyols, sucrose, invert sugar, glucose, mannitol, vegetable oils, etc. It is also possible to lyophilize the compounds of the formula (I) and/or (II) and their pharmaceutically acceptable salts and to use the resulting lyophilisates, for example, for preparing preparations for injection or infusion.

Besides the compound or compounds according to the invention and carriers, the pharmaceutical preparations can also contain additives, for example fillers, disintegrants, binders, lubricants, wetting agents, stabilizers, emulsifiers, dispersants, preservatives, sweeteners, colorants, flavorings, aromatizers, thickeners, diluents, buffer substances, solvents, solubilizers, agents for achieving a depot effect, salts for altering the osmotic pressure, coating agents or antioxidants.

The dosage of the compound of the formula (I) and/or (II) to be administered and/or of a pharmaceutically acceptable salt thereof depends on the individual case and is, as is customary, to be adapted to the individual circumstances to achieve an optimum effect. Thus, it depends on the nature and the severity of the disorder to be treated, and also on the sex, age, weight and individual responsiveness of the human or animal to be treated, on the efficacy and duration of action of the compounds used, on whether the therapy is acute or chronic or prophylactic, or on whether other active compounds are administered in addition to compounds of the formula (I) and/or (II). The daily dose can be administered in a single dose or, in particular when larger amounts are administered, be divided into several, for example two, three or four individual doses. In some cases, depending on the individual response, it may be necessary to deviate upwards or downwards from the given daily dose.

It is to be understood that the compounds identified in accordance with the method of the present invention may be used as pharmaceutical compositions to treat cancer and, preferably, even the cancer varieties referred to beforehand. Accordingly, the method of the present invention may further comprise the steps of manufacturing the identified compound as a pharmaceutical composition as described elsewhere in this specification.

### Figures

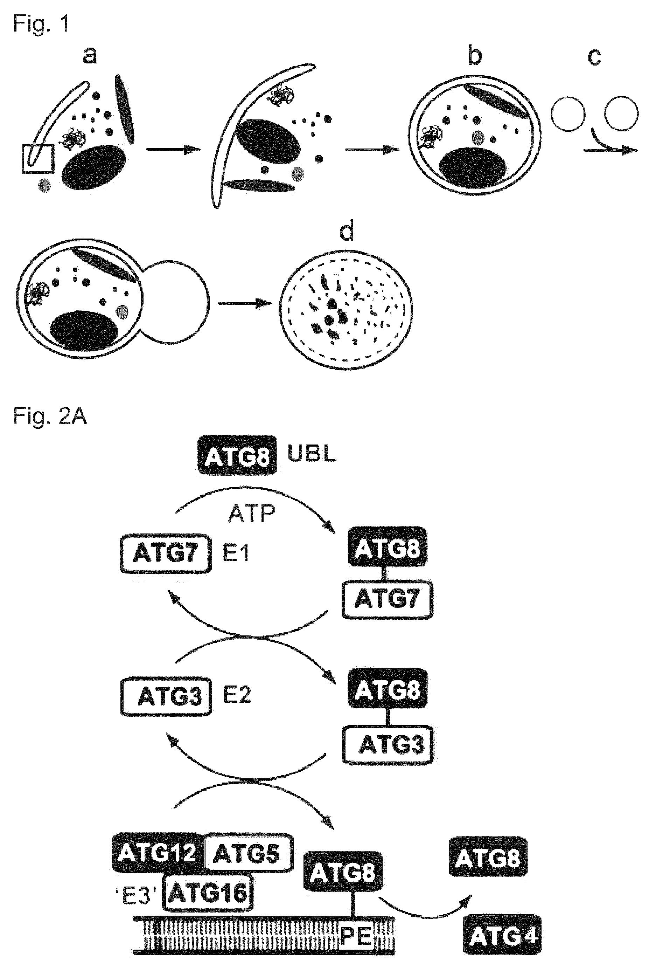

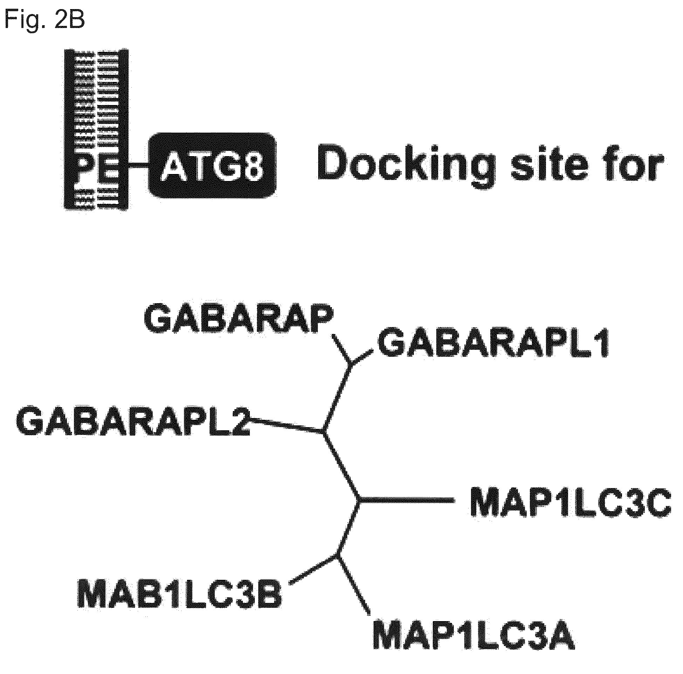

Figures 1 and 2 show the an overview of the autophagy pathway (Fig. 1), wherein a phagophore (a) transforms into an autophagosome (b) that fuses with endocytic compartments (c) thereby forming an autolysosome (d); the ATG8 conjugation cascade (Fig. 2A); and human ATG8s: binding proteins and their function (Fig. 2B).

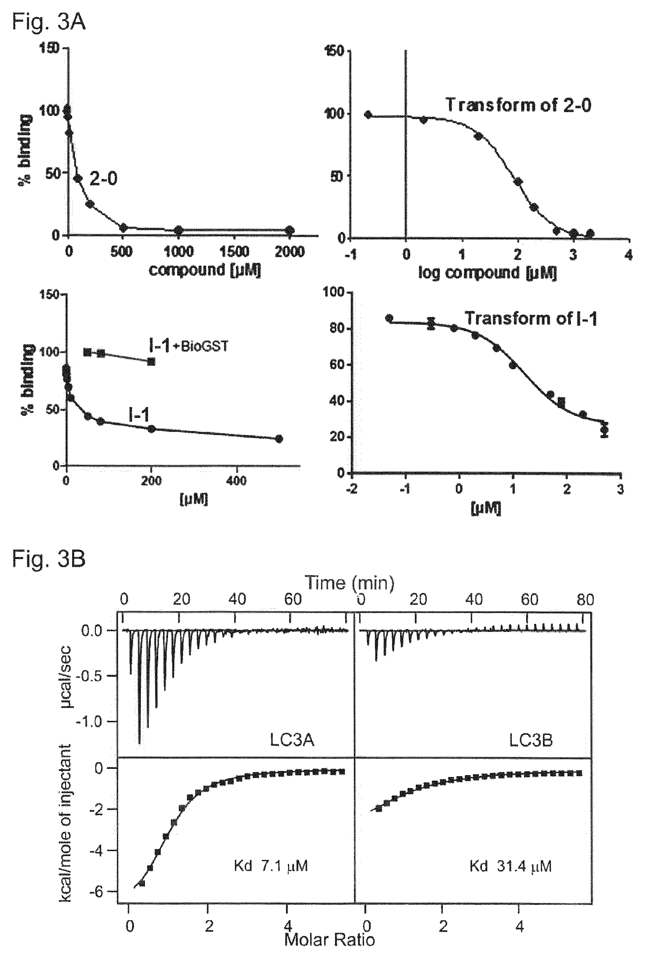

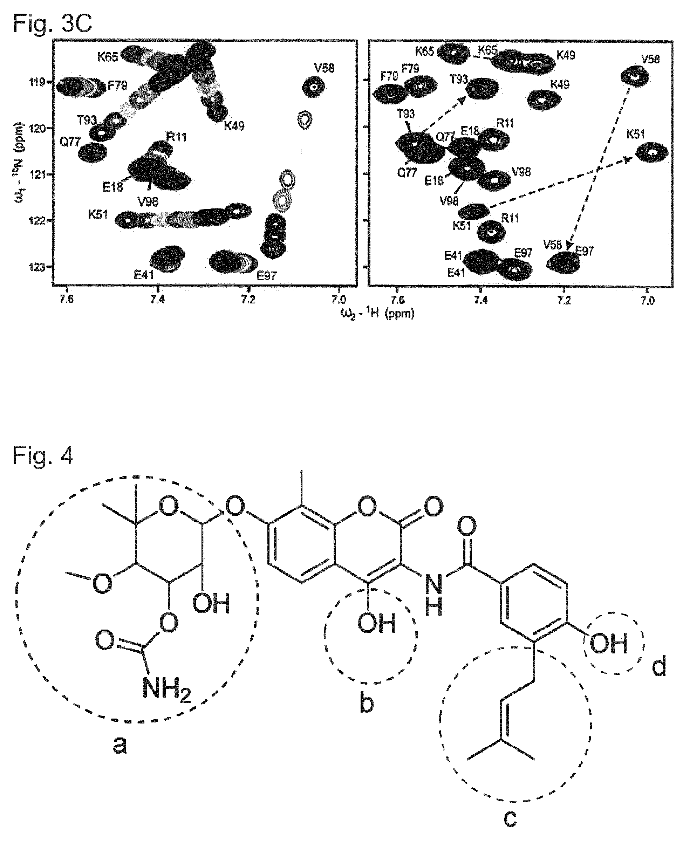

Figure 3 shows the *in vitro* interaction with protein targets. Hrerein, Fig. 3A shows the effect of compounds on the GST-LC3B / Biotin-LIRtide interaction. Fig. 3B shows the ITC characterization of the interaction of compound 2-0 with LC3A and LC3B, namely the ITC results for the titration of compound 2-0 with LC3A (left) and LC3B (right). Raw titration profiles are shown on top, molar heats of interaction (dots) and calculated on a "one-site binding" algorithm profiles (lines) are shown at the bottom. Calculated Kd are shown. Fig. 3C shows the NMR characterization of the interaction of of compound 2-0 with LC3A and LC3B, namely left plot: Fingerprint areas of the LC3B 1H-15N HSQC spectra upon titration with of compound 2-0, where the different molar ratios of protein:compound are indicated (reference, 1:8). Right plot: the same fingerprint area for free LC3B and LC3B saturated with p62 LIR (molar ratio 1:2)
Figure 4 shows a structural overview of the information provided by structure-activity-relationship (SAR) studies. Herer, (a) appears effective in influencing solubility and affinity, (b) and (d) effective for influencing affinity, and (c) for occupying the leucine binding pocket on the LIR docking site.

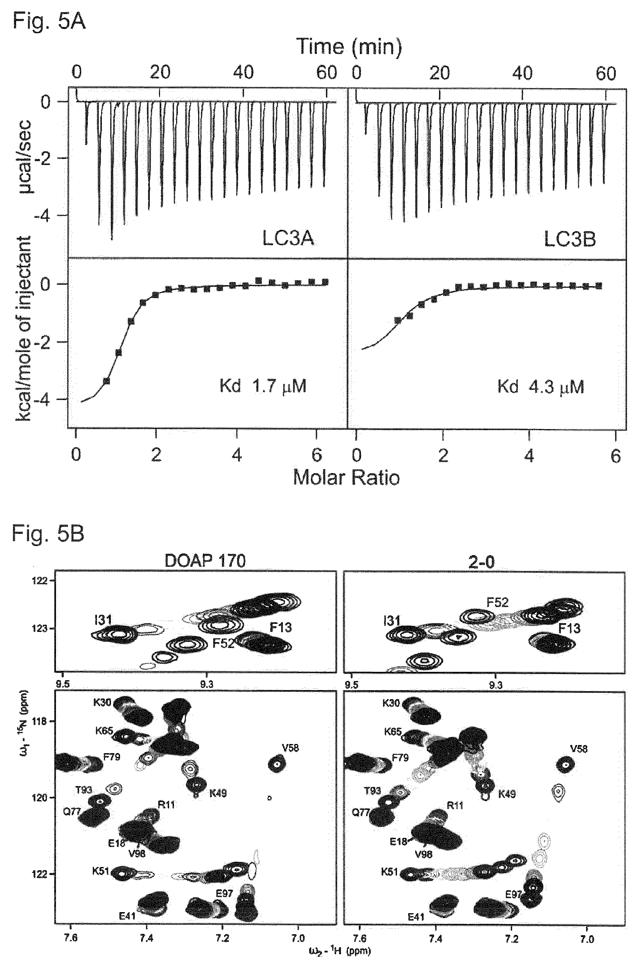

Figure 5A ITC results for the titration of compound ***13*** to LC3A (left) and LC3B (right). Raw titration profiles are shown on top, molar heats of interaction (dots) and calculated on a "one-site binding" algorithm profiles (lines) are shown at the bottom. Calculated Kd are shown. High negative dilution heat of compound ***13*** in ITC buffer contained 1% DMSO was accurately subtracted from the raw data. Fig. 5B shows representative areas of the NMR titration of DOAP 170 (left) and of compound 2-0 (right) to ¹⁵N-LC3B. ¹H-¹⁵N HSQC spectra were recorded at different molar ratios, indicated by different grey scales. Upper plots: Area near residue 131, which represents dynamic of LC3B (exchange mode upon titrations: fast to intermediate for ***13*** and pure fast mode for of compound 2-0 near HP2. Lower plots: Fingerprint areas of the LC3B ¹H-¹⁵N HSQC spectra upon titration with ***13***, where the different molar ratios of protein:compound are indicated. Right plot: the same fingerprint area for compound 2-0 titrations.

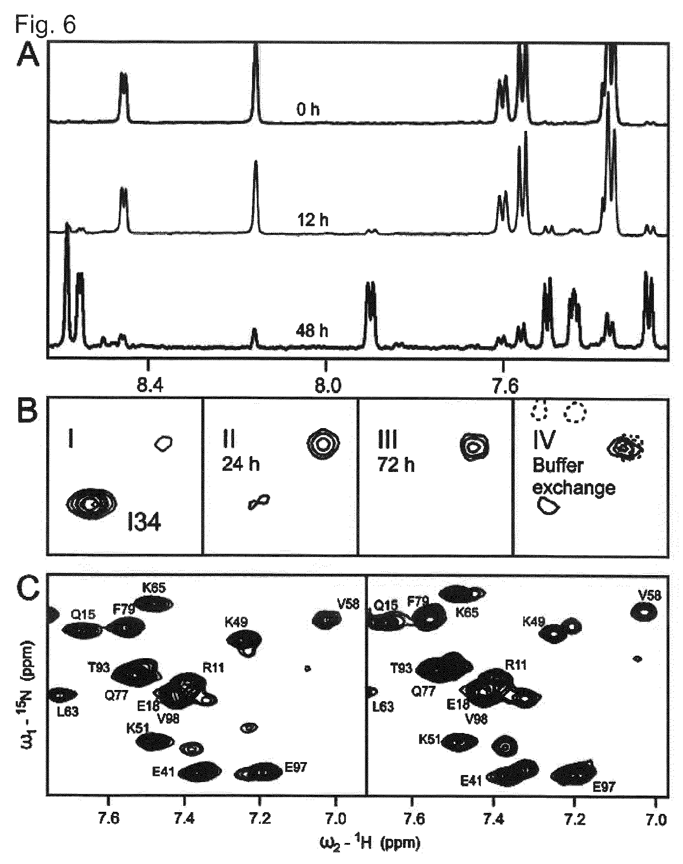

Figure 6 shows compound I-1 interaction with LC3B represents unknown mechanism involving compound I-1 hydrolysis. A) Representative region of 1D NMR spectra. 1.5 mM compound I-2 direct after addition, after overnight at 25°C, after additional incubation of the sample 24 hours at 37°C. B) Representative area of LC3B ¹H-¹⁵N HSQC spectra near I34 HN resonance upon different incubation time with compound I-1 and different operations. I - Sample directly after addition of compound I-1 (contours) compared the same sample after overnight at 25°C; II - the same sample after additional 24 hours incubation at 37°C; III - the same sample after additional 48 hours incubation at 37°C; IV - the same sample after buffer exchange by 3 times ten-fold dilution and following concentration. The I34 resonance before buffer exchange is shown. C) Left: Fingerprint area of LC3B 1H-15N HSQC spectra directly after addition of compound I-1 compared to the same sample after incubated over night at 25°C. Right: Fingerprint area of LC3B ¹H-¹⁵N HSQC spectra directly after addition of compound I-1 compared to the same sample after 48 hours incubation at 37°C.

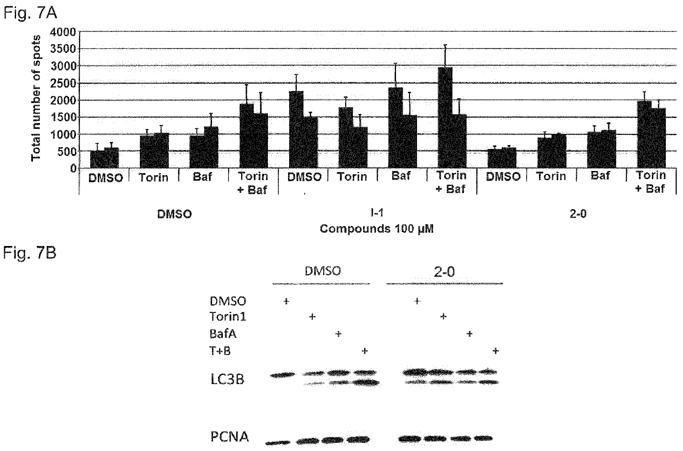

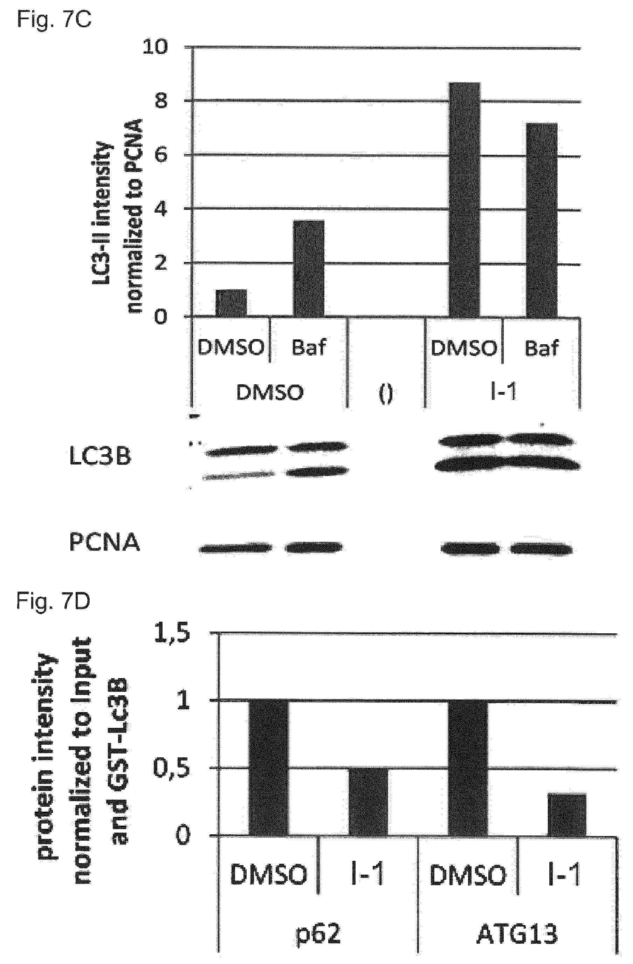

Figure 7 shows the effect of compound I-1 and of compound 2-0 on autophagic flux in U2OS cells. **A** Quantification of LC3B- and p62-positive punctae. Hererin, the left bar each represents the data for LC3, the right bar each the data for p62. **B** and **C** Lysates of U2OS cells treated as described above were analyzed by immunoblotting with indicated antibodies. PCNA served as loading control. **D** Purified GST-LC3B was incubated with lysates treated 1 hr with and without 100 µM compound I-1 and analyzed by immunoblotting, the quantification of immunoblot is shown.

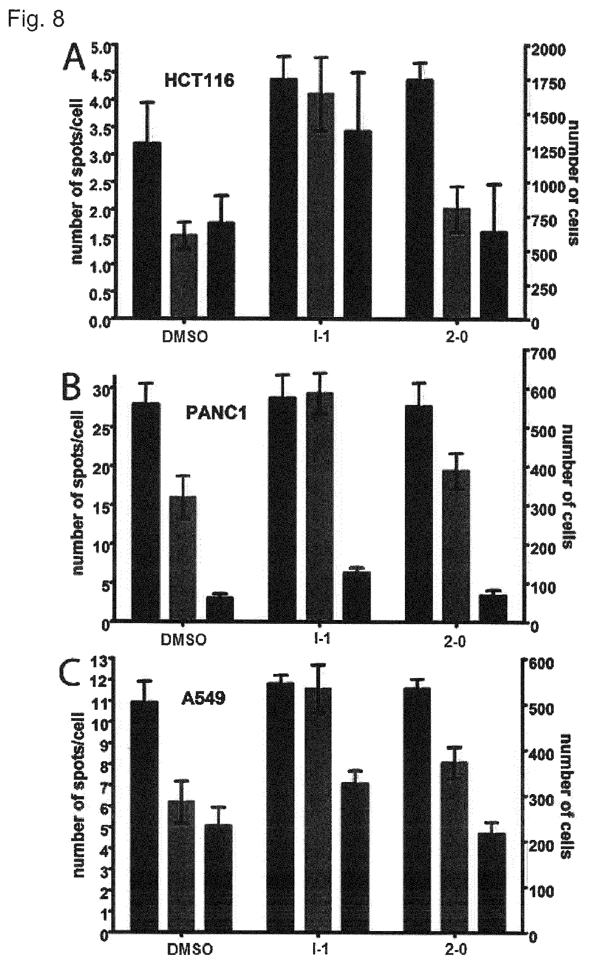

Figure 8 shows the effect of compound I-1 on autophagy in autophagy-dependent cancer cell lines. HCT116 (A), PANC1 (B), and A549 (C) were treated for 1 hr with 100 µM compound I-1 or of compound 2-0 prior to fixation and labeling with anti-LC3B and -p62 antibodies. Quantification of number of cells and spots per cells are shown. Herein, the left bar represents the number of cells, the middle bar LC3 and the right bar p62.

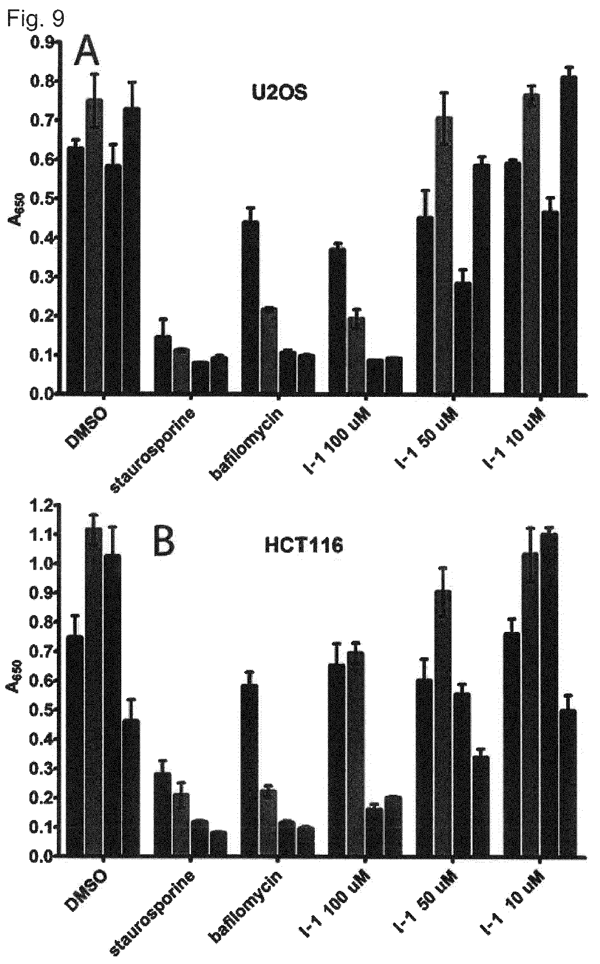

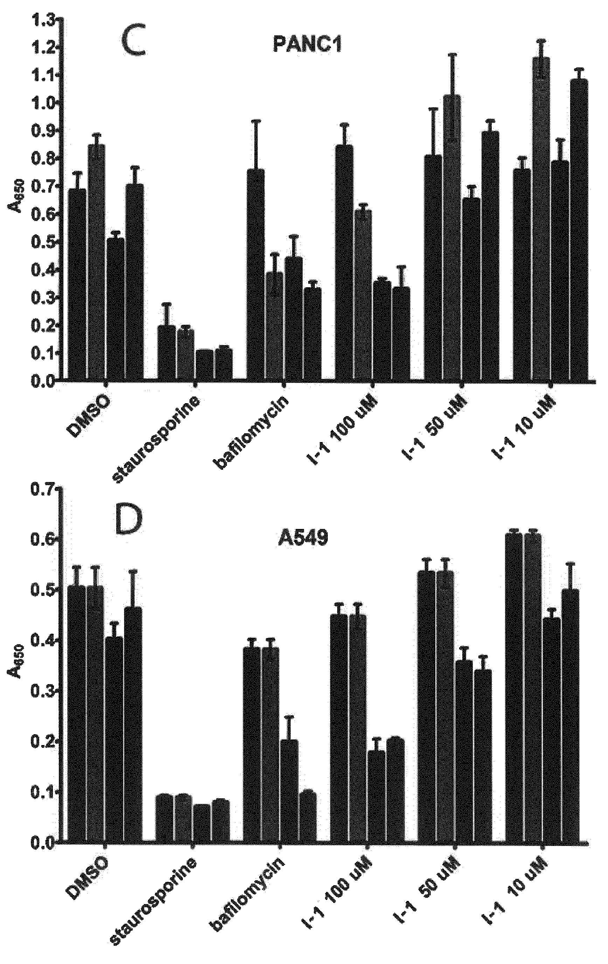

Figure 9 shows MTT cell viability assays. U2OS(A), HCT116 (B), PANC1 (C), and A549 (D) cells were treated for 96 hr with 100 µM, 50 µM or 10 µM of compound I-1. Every 24 hr, cells were subjected to the MTT assay. As positive controls, staurosporine (1 µM) and BafA1 (100 nM) were used. Herein, the bars from left to right each represent the values after 24h, 48h, 72h and 96h.

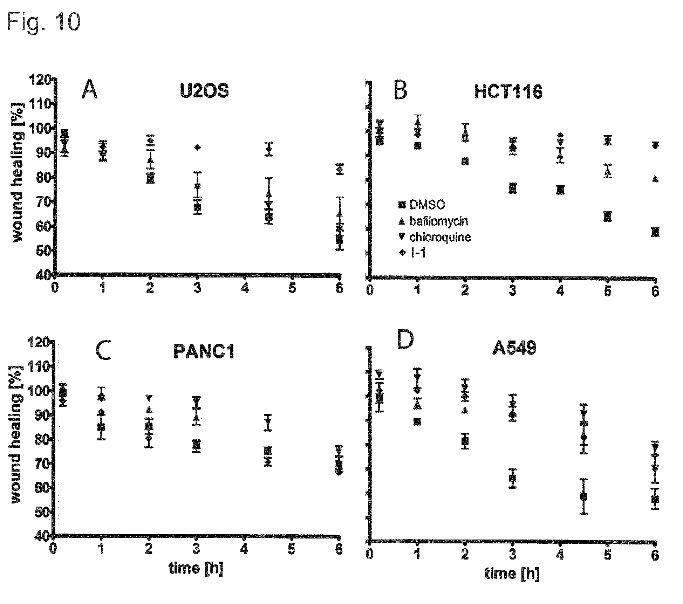

Figure 10 shows results of a wound healing assay. Monolayers of U2OS(A), HCT116 (B), PANC1 (C), and A549 (D) cells were scratched and treated for 1 hr with 100 µM compound I-1. Wound healing by migration was monitored by measuring the distance between the two scratch boarders every hour. As positive controls, chloroquine (100 µM) and BafA1 (100 nM) were used.

The invention will now be illustrated by the following Examples. However, the Examples are not meant to limit the scope of the invention in any respect.

### Examples

### A. Chemical Examples

### General procedures

All starting materials, solvents and reagents were purchased from commercial suppliers, (e.g. Sigma-Aldrich, Apollo Scientific, Acros Organics). 1H-NMR and ¹³C-NMR spectra were recorded on a Bruker AMX 250 (250 MHz), a Bruker AMX 300 (300 MHz) or a Bruker AMX 400 (400 MHz) spectrometer (Bruker, Germany). 1H-NMR data are reported in the following order: chemical shift (I) in ppm, multiplicity (br, broad; s, singlet; d, doublet; t, triplet; q, quartet; m, multiplet); approximate coupling constants *(J)* in Hertz (Hz). ESI-MS was performed on a Fisons Instruments VG Platform II (Manchester, Great Britain) in positive or negative polarity. Data (m/z) are listed as mass number (M+H⁺), (M-H⁺) and relative intensity (%). MALDI_MS was performed on Voyager DE STR(Per Septive Biosystems). High Resolution Mass Spectrometry (HRMS) was carried out on a Thermo Scientific MALDI LTQ XL Orbitrap (Waltham, USA). The values obtained by the final compounds were within ± 0.3 ppm of the theoretical values. Thin layer chromatography was performed on silica gel 60 F₂₅₄ aluminium sheets (Merck, Germany). Preparative column chromatography was performed on silica gel 63-200 µM (Merck, Germany).

### Synthesis

### A.1 Tetrahydrotriazine Derivatives (I)

### General procedure for the synthesis of the 1,2,3,4-tetrahydro-1,3,5-triazine derivatives I-2 to I-19

Step 1: benzyl carbamimidothioate ***i***ntermediates [in Schema 1): 4-chloro-benzylchloride or 4-bromobenzylbromide were dissolved in 4ml EtOH, treated with (1eq) thiocarbamide refluxed for2h and afterwards stirred for 24h at room temperature. The incurred colorless precipitate was suction filtered, washed with diethyl ether and dried to obtain the desired ***intermediates*** in good yields (95 %). Step 2: The amine (1 eq) dissolved in dioxane was mixed well with (2 eq) of 37% Formaldehyde and stirred for 0,5h at 70°C. To this solution (1 eq) of the substituted thiourea was added. The mixture was heated for 2h at 70°C and stirred for 24h at room temperature. After the reaction was complete which was controlled by using tlc in (CH₂Cl₂: MeOH (NH3)→ 9:1) or(CH₂Cl₂: MeOH → 9:1), the reaction mixture was evaporated under reduced pressure and the resulting oil purified via column chromatography using (CH₂Cl₂:MeOH (NH3)→ 9:1) as eluent for compounds with a pyridine residue and (CH₂Cl₂: MeOH → 9:1) without a pyridine residue.

Compounds (I-1) and were commercially available from Maybridge.

### 6-((4-bromobenzyl)thio)-3-(pyridin-3-ylmethyl)-1,2,3,4-tetrahydro-1,3,5-triazine (1-1)

The product was obtained as light yellow solid.
Yield: 0, 15 g(48 %)
TLC: R_{f}: 0, 66 (CH₂Cl₂: MeOH (NH₃) → 9:1)
¹H-NMR (400 MHz, DMSO-d₆) [δ ppm]: 8,46 (m, 3H, H_{Ar}); 7,51 (m, 2H, H_{Ar}); 7,36 (m, 2H, H_{Ar});4,14 (s, 2H); 4,10 (s, 4H); 3,67 (s,2H).
¹³C-NMR (100MHz, DMSO-d₆) [δ ppm]: 151,50; 149,90; 148,39; 138,85; 136,36; 134,07;131,23; 131,09; 130,40; 123,42; 119,77; 66,38; 61,37; 51,87; 31,29. ESI-MS (M+H⁺): 377.9 HRMS (M + H⁺) calculated C₁₆H₁₈BrN₄S: 377.04301 found: 377.04308.

### 6-((4-chlorobenzyl)thio)-3-(pyridin-3-ylmethyl)-12,3,4-tetrahydro-1,3,5-triazine (I-2)

The product was obtained as light yellow solid.
Yield: 0,3 g (80 %)
TLC: R_{f}: 0,44 (CH₂Cl₂: MeOH (NH₃) → 9:1)
¹H-NMR (400 MHz, DMSO-d₆) [δ ppm]: 8,49 (m, 2H, H_{Ar}); 7,71 (m, 2H, NH, H_{Ar}); 7,40 (m, 5H,H_{Ar}); 4,35 (s,2H); 4,11 (s,4H); 3,67 (s,2H).
¹³C-NMR (100MHz, DMSO-d₆) [δ ppm]: 151,57; 149,89; 148,77; 148,40; 138,40; 136,37;135,09; 134,06; 131,30; 130,60; 128,17; 123,42; 118,58; 51,86; 31,24. ESI-MS (M+H⁺): 333.4 HRMS (M + H⁺) calculated C₁₆H₁₈ClN₄S: 333.09352 found: 333.09346.

### 6-((4-fluorobenzyl) thio)-3-(pyridin-3-ylmethyl)-1,2,3,4-tetrahydro-1,3,5-triazine (I-3)

The product was obtained as light yellow solid.
Yield: 0.13g (26%)
TLC: R_{f}: 0,53 (CH₂Cl₂: MeOH (NH₃) → 9:1)
¹H-NMR (400 MHz, DMSO-d₆) [δ ppm]: 8,40 (m, 2H, H_{Ar}); 7,65 (m, 1H, H_{Ar}); 7,33 (m, 3H,H_{Ar}); 7,08 (m, 2H, H_{Ar}); 6.90 (bs, 1H, NH); 4,17 (s,2H); 4,06 (s,4H); 3,60 (s,2H).
¹³C-NMR (100MHz, DMSO-d₆) [δ ppm]: 162.28; 159.86 151,48; 149,91; 148,39; 136,38; 135,51; 135,48; 134,11; 130,68; 130,60; 123,42; 115,05; 59.91; 51,89; 31,16.
ESI-MS (M+H⁺): 317.4 HRMS (M + H⁺) calculated C₁₆H₁₈FN₄S: 317.12307 found: 317.12347.

### 6-(benzylthio)-3-(pyridin-3-ylmethyl)-1,2,3,4-tetrahydro-1,3,5-triazine (I-4)

The product was obtained as colorless solid.
Yield: 0.12g (22%)
TLC: R_{f}: 0,38 (CH₂Cl₂: MeOH (NH₃) → 9:1)
¹H-NMR (400 MHz, DMSO-d₆) [δ ppm]: 8,41 (m, 2H, H_{Ar}); 7,64 (m, 1H, H_{Ar}); 7,24 (m, 6H,H_{Ar}); 6.88 (bs, 1H, NH); 4,07 (m,6H); 3,61 (s,2H).
¹³C-NMR (100MHz, DMSO-d₆) [δ ppm]: 151,60; 149,91; 148,38; 139,09; 136,39; 134,13; 128,75; 128,25; 126,73; 123,42; 51,88; 32,05.
ESI-MS (M+H⁺): 299.2 HRMS (M + H⁺) calculated C₁₆H₁₉N₄S: 299.13249 found: 299.13298.

### 3-(pyridin-3-ylmethyl)-6-((4-(trifluoromethyl)benzyl)thio)-1,2,3,4-tetrahydro-1,3,5-triazine (I-5)

The product was obtained as colorless solid.
Yield: 0.13g (28%)
TLC: R_{f}: 0,44 (CH₂Cl₂: MeOH (NH₃) → 9:1)
¹H-NMR (400 MHz, DMSO-d₆) [δ ppm]: 8,53 (m, 2H, H_{Ar}); 7,74 (m, 3H, H_{Ar}); 7,65 (m, 2H,H_{Ar}); 7,42 (m, 1H, H_{Ar}); 7.07 (bs, 1H, NH); 4,28 (m,4H); 3,99 (s,2H); 3,70 (s, 2H).
¹³C-NMR (100MHz, DMSO-d₆) [δ ppm]: 151,12; 149,90; 148,39; 144,51; 136,33; 134,07; 129,50; 127,47; 125,66; 125,00; 123,38; 51,86; 31,38.
ESI-MS (M+H⁺): 367.15 HRMS (M + H⁺) calculated C₁₇H₁₇F₃N₄S: 367.11988 found: 367.11981.

### 6-((4-methoxybenzyl) thio)-3-(pyridin-3-ylmethyl)-1,2,3,4-tetrahydro-1,3,5-triazine (I-6)

The product was obtained as colorless solid.
Yield: 0.2g (40%)
TLC: R_{f}: 0,37 (CH₂Cl₂: MeOH (NH₃) → 9:1)
¹H-NMR (400 MHz, DMSO-d₆) [δ ppm]: 8,41 (m, 2H, H_{Ar}); 7,65 (m, 1H, H_{Ar}); 7,31 (m, 1H,H_{Ar}); 7,22 (m, 2H, H_{Ar}); 6.84 (m, 3H, NH, H_{Ar}); 4,14 (m,6H); 3,66 (s,3H); 3,61 (s, 2H).
¹³C-NMR (100MHz, DMSO-d₆) [δ ppm]: 158,84; 151,77; 149,93; 148,38; 136,33; 134,15; 130,77; 129,92; 123,42; 113,67; 55,06; 51,90; 31,62.
ESI-MS (M+H⁺): 329.15 HRMS (M + H⁺) calculated C₁₇H₂₁N₄OS: 329.14306 found: 329.14322.

### N-isopentyl-4-(((5-(pyridin-3-ylmethyl)-1,4,5,6-tetrahydro-1,3,5-triazin-2-yl)thio)methyl)benzamide (I-7)

0,15g (0,43mmol) 4-(((5-(pyridin-3-ylmethyl)-1,4,5,6-tetrahydro-1,3,5-triazin-2-yl)thio)methyl)benzoic acid (I-28) (which was synthesized using the general procedure A.1) were suspended in 4ml DMF and 4ml CH₂Cl₂. To this solution EDC*HCl 92mg (0,48mmol, 1.1 eq) was added, followed by 1H-1,2,3-benzotriazol-1-ol 74mg (0,48mmol,1.1 eq). After the added reagents were dissolved 0,05ml (0,43mmol) 3-methylbutan-1- amine was added and the reaction mixture was stirred for 48h at room temperature. The completion of the reaction was monitored by tlc using (CH₂Cl₂: MeOH (NH₃) → 9:1) as eluent. The solvent was evaporated under reduced pressure , the residue was dissolved in CH₂Cl₂, extracted with saturated aqueous NaCl, dried over MgSPO₄, filtrated and evaporated. The light yellow oil was purified via column chromatography. The product was obtained as colorless solid.
Yield: 70mg (41%)
TLC: R_{f}: 0, 34 (CH₂Cl₂: MeOH (NH₃) → 9:1)
¹H-NMR (400 MHz, DMSO-d₆) [δ ppm]: 8,42 (m, 2H, H_{Ar}); 8,29 (t, J= 5,63Hz,1H, NH) 7,69 (m, 2H, H_{Ar}); 7,62 (m, 1H,H_{Ar}); 7,37 (m, 2H, H_{Ar}); 7,28 (m, 1H, H_{Ar}); 6.95 (bs, 1H, NH); 4,11 (m,6H); 3,60 (s,2H); 3,20 (m, 2H); 1,53 (m, 1H); 1,35 (m, 2H); 0,81 (s, 6H).
¹³C-NMR (100MHz, DMSO-d₆) [δ ppm]: 165,73; 151,41; 149,91; 148,38; 142,36; 136,40; 134,15; 133,19; 128,53; 127,10; 123,41; 51,88; 31,64; 25,29; 22,42. ESI-MS (M+H⁺): 412.24 HRMS (M + H⁺) calculated C₂₂H₃₀N₅OS: 412.21656 found: 412.21636.

### 6-((3-bromobenzyl)thio)-3-(pyridin-3-ylmethyl)-1,2,3,4-tetrahydro-1,3,5-triazine (I-8)

The product was obtained as colorless oil.
Yield: 0, 25g (36 %)
TLC: R_{f}: 0, 53 (CH₂Cl₂: MeOH (NH₃) → 9:1)
¹H-NMR (400 MHz, DMSO-d₆) [δ ppm]: 8, 46 (m, 3H, H_{Ar}); 7,51 (m, 2H, H_{Ar}); 7,36 (m, 2H, H_{Ar});4,14 (s, 2H); 4,10 (s, 4H); 3,67 (s,2H).
¹³C-NMR (100MHz, DMSO-d₆) [δ ppm]: 151,26; 149,89; 148,40; 142,45; 136,37; 134,08; 131,34; 130,35; 129,52; 127,80; 123,43; 121,30; 51,88; 31,18.
ESI-MS (M+H⁺): 377.9 HRMS (M + H⁺) calculated C₁₆H₁₇BrN₄S: 377.04301 found: 377.04446.

### 6-((3-methoxybenzyl)thio)-3-(pyridin-3-ylmethyl)-1,2,3,4-tetrahydro-1,3,5-triazine (I-9)

The product was obtained as colorless solid.
Yield: 0.39g (65%)
TLC: R_{f}: 0,53 (CH₂Cl₂: MeOH (NH₃) → 9:1)
¹H-NMR (400 MHz, DMSO-d₆) [δ ppm]: 8,50 (m, 2H, H_{Ar}); 7,73 (m, 1H, H_{Ar}); 7,39 (m, 1H,H_{Ar}); 7,25 (m, 1H, H_{Ar}); 6,94 (m, 2H, H_{Ar}) 6,81 (m, 1H, H_{Ar}); 4,13 (m,6H); 3,75 (s,3H); 3,70 (s, 2H).
¹³C-NMR (100MHz, DMSO-d₆) [δ ppm]: 159,16; 151,63; 149,91; 148,38; 140,66; 136,37; 134,12; 133,90; 129,28; 123,41; 120,96; 114,40; 112,18; 54,95; 51,90; 32,01.
ESI-MS (M+H⁺): 329.21 HRMS (M + H⁺) calculated C₁₇H₂₁N₄OS: 329.14306 found: 329.14301.

### 6-((3-phenoxybenzyl)thio)-3-(pyridin-3-ylmethyl)-1,2,3,4-tetrahydro-1,3,5-triazine (I-10)

The product was obtained as colorless solid.
Yield: 0.27g (45%)
TLC: R_{f}: 0,64 (CH₂Cl₂: MeOH (NH₃) → 9:1)
¹H-NMR (400 MHz, DMSO-d₆) [δ ppm]: 8,41 (m, 2H, H_{Ar}); 7,62 (m, 1H, H_{Ar}); 7,31 (m, 4H,H_{Ar}); 7,05 (m, 2H, H_{Ar}); 6,94 (m, 4H, NH, H_{Ar}) 6,79 (m, 1H, H_{Ar}); 4,06 (m,6H); 3,56 (s, 2H).
¹³C-NMR (100MHz, DMSO-d₆) [δ ppm]: 156,69; 151,39; 150,02; 148,32; 141,86; 136,46; 134,13; 129,99; 123,85; 118,76; 116,96; 51,81; 31,78.
ESI-MS (M+H⁺): 391.19 HRMS (M + H⁺) calculated C₂₂H₂₃N₄OS: 391.15871 found: 391.15894.

### N-isopentyl-3-(((5-(pyridin-3-ylmethyl)-1,4,5,6-tetrahydro-1,3,5-triazin-2-yl)thio)methyl)benzamide (I-11)

0,15g(0,44mmol)3-(((5-(pyridin-3-ylmethyl)-1,4,5,6-tetrahydro-1,3,5-triazin-2-yl)thio)methyl)benzoic acid (I-29) (which was synthesized using the general procedure A.1) were suspended in 5ml DMF and 5ml CH₂Cl₂. To this solution EDC*HCl 92mg (0,48mmol, 1.1 eq) was added, followed by 1H-1,2,3-benzotriazol-1-ol 74mg (0,48mmol,1.1 eq). After the added reagents were dissolved 0,05ml (0,44mmol) 3-methylbutan-1- amine was added and the reaction mixture was stirred for 48h at room temperature. The completion of the reaction was monitored by tlc using (CH₂Cl₂: MeOH (NH₃) → 9:1) as eluent. The solvent was evaporated under reduced pressure , the residue was dissolved in CH₂Cl₂, extracted with saturated aqueous NaCl, dried over MgSO₄, filtrated and evaporated. The light yellow oil was purified via column chromatography. The product was obtained as colorless solid.
Yield: 40mg (22%)
TLC: R_{f}: 0,5 (CH₂Cl₂: MeOH (NH₃) → 9:1)
¹H-NMR (400 MHz, DMSO-d₆) [δ ppm]: 8,40 (m, 2H, H_{Ar}); 8,31 (t, J= 5,63Hz,1H, NH) 7,78 (m, 1H, H_{Ar}); 7,63 (m, 2H,H_{Ar}); 7,43 (m, 1H, H_{Ar}); 7,29 (m, 2H, H_{Ar}); 6.94 (bs, 1H, NH); 4,13 (m,6H); 3,60 (s,2H); 3,20 (m, 2H); 1,52 (m, 1H); 1,34 (m, 2H); 0,82 (s, 6H).
¹³C-NMR (100MHz, DMSO-d₆) [δ ppm]: 165,89; 151,42; 149,92; 148,33; 139,39; 136,35; 134,83; 134,14; 133,19; 131,27; 128,10; 127,72; 125,37; 123,38; 51,87; 31,86; 25,27; 22,40.
ESI-MS (M+H⁺): 412.28 HRMS (M + H⁺) calculated C₂₂H₃₀N₅OS: 412.21656 found: 412.21623.

### methyl(3-(((5-(pyridin-3-ylmethyl)-1,4,5,6-tetrahydro-1,3,5-triazin-2-yl)thio)methyl)benzoyl)leucinate (I-12)

0,15g(0,44mmol)3-(((5-(pyridin-3-ylmethyl)-1,4,5,6-tetrahydro-1,3,5-triazin-2-yl)thio)methyl)benzoic acid (I-29) (which was synthesized using the general procedure A.1) were suspended in 5ml DMF and 5ml CH₂Cl₂. To this solution EDC*HCl 92mg (0,48mmol, 1.1 eq) was added, followed by 1H-1,2,3-benzotriazol-1-ol 74mg (0,48mmol,1.1 eq). After the added reagents were dissolved 64mg (0,44mmol) L-leucine methylester was added and the reaction mixture was stirred for 48h at room temperature. The completion of the reaction was monitored by tlc using (CH₂Cl₂: MeOH (NH₃) → 9:1) as eluent. The solvent was evaporated under reduced pressure, the residue was dissolved in CH₂Cl₂, extracted with saturated aqueous NaCl, dried over MgSPO₄, filtrated and evaporated. The light yellow oil was purified via column chromatography. The product was obtained as colorless foam.
Yield: 95mg (48%)
TLC: R_{f}: 0,52 (CH₂Cl₂: MeOH (NH₃) → 9:1)
¹H-NMR (400 MHz, DMSO-d₆) [δ ppm]: 8,77 (d, J= 7.8Hz; 1H, NH); 8,52 (m, 2H, H_{Ar}); 7,95 (m, 1H, H_{Ar}); 7,83 (m, 1H,H_{Ar}); 7,73 (m, 1H, H_{Ar}); 7,59 (m, 1H, H_{Ar}); 7,45 (m, 2H, H_{Ar}); 6.99 (bs, 1H, NH); 4,56 (m, 1H); 4,26 (m,6H); 3,70 (s, 3H); 3,68 (s,2H); 3,20 (m, 2H); 1,84 (m, 1H); 1,71 (m, 2H); 0,97 (s, 6H).
¹³C-NMR (100MHz, DMSO-d₆) [δ ppm]: 173.08; 166,42; 151,33; 149,91; 148,32; 139,53; 136,35; 134,83; 133,76; 131,81; 128,17; 125,80; 123,38; 51,87; 50,88; 31,79; 24,41; 22,81; 21,11.
ESI-MS (M+H⁺): 470.27 HRMS (M + H⁺) calculated C₂₄H₃₂N₅O₃S: 470.22204 found: 470.22098.

### 6-((4-chlorobenzyl)thio)-3-(1-(pyridin-3-yl)ethyl)-1,2,3,4-tetrahydro-1,3,5-triazine (I-13)

The product was obtained as light yellow solid.
Yield: 0, 17 g (49 %)
TLC: R_{f}: 0, 54 (CH₂Cl₂: MeOH (NH₃) → 9:1) ¹H-NMR (400 MHz, DMSO-d₆) [δ ppm]: 8,46 (m, 3H, H_{Ar}); 7,51 (m, 2H, H_{Ar}); 7,36 (m, 2H, H_{Ar});4,14 (s, 2H); 4,10 (s, 4H); 3,91 (m, 1H) 3,67 (s,2H), 1,22 (m, 3H). ¹³C-NMR (100MHz, DMSO-d₆) [δ ppm]: 151,70; 149,88; 148,35; 139,85; 136,55; 134,75;130,23; 129,09; 128,40; 123,42; 119,77; 68,01; 66,38; 61,37; 51,87; 31,29; 18,21
ESI-MS (M+H⁺): 347.3 HRMS (M + H⁺) calculated C₁₇H₂₀ClN₄S: 347.10917 found: 347.10908.

### 6-((4-bromobenzyl)thio)-3-(4-fluorobenzyl)-1,2,3,4-tetrahydro-1,3,5-triazine (I-14)

The product was obtained as colorless oil.
Yield: 0,23 g (41%)
TLC: R_{f}: 0, 60 (CH₂Cl₂: MeOH (NH₃) → 9:1)
¹H-NMR (400 MHz, DMSO-d₆) [δ ppm]: 7,61 (m, 2H, H_{Ar}); 7,47 (m, 4H, H_{Ar}); 7,21 (m, 2H, H_{Ar}); 7,05 (bs, 1H, NH); 4,19 (m, 6H); 3,70 (s,2H).
¹³C-NMR (100MHz, DMSO-d₆) [δ ppm]: 162,49; 160,08; 151,23; 138,97; 134,79; 131,07; 130,56; 119,73; 115,00; 66,33; 54,88; 53,69; 51,87; 31,23.
ESI-MS (M+H⁺): 395.3

### 6-((4-chlorobenzyl)thio)-3-(4-fluorobenzyl)-1,2,3,4-tetrahydro-1,3,5-triazine (I-15)

The product was obtained as yellow oil.
Yield: 0,20 g (59 %)
TLC: R_{f}: 0,45 (CH₂Cl₂: MeOH → 9:1)
¹H-NMR (400 MHz, DMSO-d₆) [δ ppm]: 7,44 (m, 4H, H_{Ar}); 7,30 (m, 2H, H_{Ar}); 7,17 (m, 2H, H_{Ar});4,30 (s,2H); 4,15 (s,4H); 3,56 (s,2H).
¹³C-NMR (100MHz, DMSO-d₆) [δ ppm]: 162,61; 160,19; 137,29; 134,16; 131,73; 130,70;130,61; 128,36; 115,13; 62,45; 53,44; 31,94.
ESI-MS (M+H⁺): 351.0 HRMS (M + H⁺) calculated C₁₇H₁₈ClFN₃S: 350.08885 found: 350.08896.

### 3-(4-fluorobenzyl)-6-((3-phenoxybenzyl)thio)-1,2,3,4-tetrahydro-1,3,5-triazine (I-16)

The product was obtained as colorless solid.
Yield: 0,20 g (31 %)
¹H-NMR (400 MHz, DMSO-d₆) [δ ppm]: 7,42 (m, 5H, H_{Ar}); 7,17 (m, 4H, H_{Ar}); 7,07 (m, 1H, H_{Ar}); 7,01 (m, 2H, H_{Ar}); 6,89 (bs, 1H, NH); 4,18 (s,2H); 4,08 (s,4H); 3,60 (s,2H).
¹³C-NMR (100MHz, DMSO-d₆) [δ ppm]: 162,53; 160,12; 156,62; 152,48; 141,29; 134,58; 130,52; 130,02; 129,85; 123,48; 118,71; 63,04; 53,63; 31,77.
ESI-MS (M+H⁺): 408.16 HRMS (M + H⁺) calculated C₂₃H₂₂FN₃OS: 408.15404 found: 408.15424.

### 6-((4-bromobenzyl)thio)-3-(3,4-difluorobenzyl)-1,2,3,4-tetrahydro-1,3,5-triazine (I-17)

The product was obtained as colorless solid.
Yield: 0,35 g (60%)
TLC: R_{f}: 0,74 (CH₂Cl₂: MeOH (NH₃) → 9:1)
¹H-NMR (400 MHz, DMSO-d₆) [δ ppm]: 7,62 (m, 2H, H_{Ar}); 7,42 (m, 3H, H_{Ar}); 7,22 (m, 1H, H_{Ar}); 7,01 (bs, 1H, NH); 4,21 (m,6H); 3,72 (s,2H).
¹³C-NMR (100MHz, DMSO-d₆) [δ ppm]: 151,30; 149,87; 141,29; 138,91; 130,97; 119,73; 117,35; 53,45; 31,25.
ESI-MS (M+2H⁺): 414.05 HRMS (M + H⁺) calculated C₁₇H₁₇BrF₂N₃OS: 412.02891 found: 412.02903.

### 3-(3,4-difluorobenzyl)-6-((3-phenoxybenzyl)thio)-1,2,3,4-tetrahydro-1,3,5-triazine (I-18)

The product was obtained as colorless solid.
Yield: 0,3 g (50%)
TLC: R_{f}: 0,85 (CH₂Cl₂: MeOH (NH₃) → 9:1)
ESI-MS (M+H⁺): 426.51

### 6-((4-bromobenzyl)thio)-3-(3-fluorobenzyl)-1,2,3,4-tetrahydro-1,3,5-triazine (I-19)

The product was obtained as colorless oil.
Yield: 0,18 g (31%)
TLC: R_{f}: 0, 59 (CH₂Cl₂: MeOH (NH₃) → 9:1)
¹H-NMR (400 MHz, DMSO-d₆) [δ ppm]: 7,63 (m, 2H, H_{Ar}); 7,47 (m, 4H, H_{Ar}); 7,23 (m, 2H, H_{Ar}); 6,99 (bs, 1H, NH); 4,14 (m, 6H); 3,68 (s,2H).
¹³C-NMR (100MHz, DMSO-d₆) [δ ppm]: 162,48; 160,12; 153,43; 139,00; 134,81; 131,12; 130,36; 119,03; 115,11; 66,23; 53,99; 53,69; 51,57; 30,78.
ESI-MS (M+H⁺): 395.31.

### 3-(3-fluorobenzyl)-6-((3-phenoxybenzyl)thio)-1,2,3,4-tetrahydro-1,3,5-triazine (I-20)

The product was obtained as colorless solid.
Yield: 0,24 g (50%)
¹H-NMR (400 MHz, DMSO-d₆) [δ ppm]: 7,42 (m, 5H, H_{Ar}); 7,17 (m, 4H, H_{Ar}); 7,07 (m, 1H, H_{Ar}); 7,01 (m, 2H, H_{Ar}); 6,89 (bs, 1H, NH); 4,18 (s,2H); 4,08 (s,4H); 3,60 (s,2H).
¹³C-NMR (100MHz, DMSO-d₆) [δ ppm]: 162,53; 160,12; 156,62; 152,48; 141,29; 134,58; 130,52; 130,02; 129,85; 123,48; 118,71; 63,04; 53,63; 31,77.
ESI-MS (M+H⁺): 408.22 HRMS (M + H⁺) calculated C₂₃H₂₂FN₃OS: 408.15404 found: 408.15424.

### A.2 Coumarin Derivatives (II)

### Novobiocin Sodium Salt (compound 2-0)

Novobiocin sodium salt was commercially available from Apollo Scientific. It is a colorless powder.
TLC: R_{f}: 0.5 (CH₂Cl₂:MeOH → 9:1)
¹H-NMR (400 MHz, DMSO-d₆) [δ ppm]: 10,07 (s, 1H, OH); 9,25 (s, 1H, NH); 7,75 (m, 3H, H_{Ar}); 7,18 (d, J=9.01Hz, 1H, H_{Ar}); 6,88 (d, J= 8.04Hz, 1H, H_{Ar}); 6,64 (bs, 2H, NH₂); 5,61 (s, 1H); 5,56
(s, 1H); 5,33 (t, J=7.33Hz, 1H); 5,19 (m, 1H); 4,12 (s,1H); 3,53 (s,1H); 3,48 (s, 3H,OCH₃); 3,29
(m, 2H); 2,24 (s, 3H); 1,73 ( s, 6H); 1,31 (s,3H); 1,10 (s,3H).
¹³C-NMR (100MHz, DMSO-d₆) [δ ppm]: 170,13; 165,04; 162,34; 157,05; 156,29; 155,21;
151,97; 131,04; 129,30; 126,72; 126,59; 122,90; 122,54; 118,06; 113,54; 111,49; 107,53;
98,49; 96,63; 80,89; 77,72; 70,44; 68,88; 60,89; 28,44; 28,07; 25,56; 22,84; 17,67; 8,53.

### N-(4,7-dihydroxy-8-methyl-2-oxo-2H-chromen-3-yl)-4-hydroxy-3-(3-methylbut-2-en-1-yl)benzamide, Novobiocic Acid (2-1)

1 g Novobiocin (1.63 mmol) was dissolved in 10 ml ethanol and heated up to 80 °C. 0.22 ml Acetylchloride (3.26 mmol, 2 eq) was added, whereby the solution got
cloudy and after that turned into a yellowish solution. The reaction was refluxed for 2 hours, cooled to room temperature and transferred into a beaker filled with ice water. The incurred white precipitation was suction filtered and dried under reduced pressure. The solid was purified via column chromatography with (hexane:EtOAc → 1:1). The product was obtained as a colorless solid.
Yield: 0.33 g (51.5 %)
TLC: R_{f}: 0.47 (hexane:EtOAc → 1:1)
¹H-NMR (400 MHz, DMSO-d₆) [δ ppm]: 11,86 (bs, 1H, OH); 10,47 (s, 1H, OH); 10,08 (s, 1H, OH); 9,22 (s, 1H, NH); 7,79 (m, 2H, H_{Ar}); 7,63 (d, J=8.78Hz, 1H, H_{Ar}); 6,93 (m,2H, H_{Ar}); 5,37 (t, J= 7.35Hz; 1H); 3,26 (m,2H); 2,22 (s,3H); 1,75 (s, 6H).
¹³C-NMR (100MHz, DMSO-d₆) [δ ppm]: 166,55; 160,85; 159,59; 151,25; 130,08; 124,15;
121,49; 110,38; 108,02; 100,47; 24,87; 24,14; 22,42; 8,01.
ESI-MS (M-H⁺): 394.6; HRMS (M + H⁺) calculated C₂₂H₂₁NO₆: 396.14416 found: 396.14390.

### 5-hydroxy-6-((4-hydroxy-3-(4-hydroxy-3-isopentylbenzamido)-8-methyl-2-oxo-2H-chromen-7-yl)oxy)-3-methoxy-2,2-dimethyltetrahydro-2H-pyran-4-y/carbamate, Dihydronovobiocine, (2-4)

The reaction was carried in an autoclave. 100 mg Pd/C (w = 10 %) were added to the solution of 1 g (1,63 mmol) Novobiocin in 40 ml methanol and stirred under a H₂-atmosphere for 4h at 50°C with a pressure of 4 bar. The black reaction mixture was filtered over celite and rinsed with 200 ml methanol and evaporated under reduced pressure. The product was obtained as an off-white solid.
Yield: 1 g (99 %)
TLC: R_{f}: 0.52 (CH₂Cl₂:MeOH→ 9:1)
¹H-NMR (400 MHz, DMSO-d₆) [δ ppm]: 9,79 (s, 1H, OH); 8,33 (s, 1H, NH); 7,75 (m, 1H, H_{Ar});
7,67 (m, 2H, H_{Ar}); 6,92 (d, J= 9.14Hz, 1H, H_{Ar}); 6,82 (d, J= 8.33Hz, 1H, H_{Ar}); 6,66 (bs, 2H, NH₂); 5,54 (bs, 1H); 5,44 (s,1H); 5,20 (m, 1H); 4,08 (s,1H); 3,48 (s, 3H, OCH₃); 2,60 (m, 2H); 2,18 (s,3H); 1,58 ( m, 3H); 1,28 (s,3H); 1,10 (s,3H); 0,96 (s,6H).
¹³C-NMR (100MHz, DMSO-d₆) [δ ppm]: 170,16; 165,63; 162,86; 157,24; 156,30; 155,19; 151,96; 129,30; 127,72; 126,37; 122,90; 122,04; 118,06; 113,99; 111,47; 107,54; 98,47; 96,62; 80,87; 77,72; 70,44; 68,88; 60,89; 28,44; 27,40 22,82; 22,52; 8,53.
ESI-MS (M-H⁺): 614.1 HRMS (M + H⁺) calculated C₃₁H₃₈N₂O₁₁: 615.25484 found: 615.25398.

### N-(4,7-dihydroxy-8-methyl-2-oxo-2H-chromen-3-yl)-4-hydroxy-3-isopentylbenzamide (2-5)

0,3 g of compound **2-4** (0.49 mmol) was dissolved in 5 ml ethanol and heated up to 80 °C. 0.1 ml Acetyl chloride (0.98 mmol, 2 eq) was added, whereby the solution got cloudy and after that turned into a yellowish solution. The reaction was refluxed for 2 h, cooled to room temperature and transferred into a beaker filled with ice water. The incurred white precipitation was suction filtered and dried under reduced pressure. The product was obtained as a colorless solid.
Yield: 0.15 g (80 %)
TLC: R_{f}: 0, 38 (CH₂Cl₂:MeOH→ 95:5)
¹H-NMR (400 MHz, DMSO-d₆) [δ ppm]: 11,78 (bs, 1H, OH); 10,33 (s, 1H, OH); 9,88 (s, 1H, OH); 9,10 (s,1H, NH); 7,75 (m, 1H, H_{Ar}); 7,63 (m, 1H, H_{Ar}); 7,51 (m,1H, H_{Ar}); 6,83 (m, 2H, H_{Ar});
2,65 (m, 2H); 2,10 (s, 3H); 1,51 (m, 1H); 1,39 (m,1H); 0,85 (s,6H).
¹³C-NMR (100MHz, DMSO-d₆) [δ ppm]: 166,58; 160,86; 159,67; 158,97; 158,41; 151,26; 129,97; 128,34; 127,35; 124,90; 121,50; 114,25; 111,84; 100,37; 108,07; 100,46; 27,45; 27,36; 22,49; 8,07.
ESI-MS (M-H⁺): 396.8 HRMS (M + H⁺) calculated C₂₂H₂₄NO₆: 398.15981 found: 398.15960.

### General procedure for the synthesis of the coumarin derivatives (2-15)-(2-19)

Step 1: 2',4'-dihydroxy-3'-methyl-acetophenon was dissolved in acetone, treated with (1.1 eq) of the corresponding substituent as chloride or bromide , (3 eq) K₂CO₃, (0.1-1 eq) Kl and refluxed for 24h.

The incurred precipitate was removed through filtration, the filtrate was evaporated and the crude oily product purified via column chromatography yielding 4'-substituted-2'-hydroxy-3'-methyl-acetophenon derivatives as intermediates.

Step 2: The substituted 2'-hydroxy-3'-methyl-acetophenon was dissolved in toluene and added dropwise to an 80 °C solution containing (4 eq) NaH in toluene. After addition of (4 eq) diethyl carbonate the reaction was refluxed for 24h at 140°C, cooled down to 0°C, transferred into a beaker filled with ice water and the incurred precipitate was suction filtered. The filtrate was acidified with 2N aqueous HCl and extracted with ethyl acetate. The organic phase was dried over MgSO₄, filtered and evaporated under reduced pressure, yielding the desired coumarin derivatives **(2-15)-(2-19)**.

### 7-(benzyloxy)-4-hydroxy-8-methyl-2H-chromen-2-one (2-15)

The product was obtained as a colorless solid.
Yield: 1,1 g (57 %)
¹H-NMR (250 MHz, DMSO-d₆) [δ ppm]: 12,35 (bs, 1H, OH); 7,75 (d, J= 9.3Hz, 1H, H_{Ar}); 7,52 (m, 5H, H_{Ar}); 7,75 (d, J= 8.9Hz, 1H, H_{Ar}); 5,54 (s,1H); 5,32 (s, 2H); 2,27 (s,3H).
¹³C-NMR (100MHz, DMSO-d₆) [δ ppm]: 166,12; 162,26; 159,39; 152,54; 136,70; 128,50; 127,91; 127,37; 121,35; 112,64; 109,24; 108,38; 88,47; 69,87; 8,23. ESI-MS (M-H⁺): 281.3.

### 4-hydroxy-8-methyl-7-(3-phenylpropoxy)-2H-chromen-2-one (2-16)

The product was obtained as a colorless solid.
Yield: 20 mg (20 %)
¹H-NMR (250 MHz, DMSO-d₆) [δ ppm]: 12,35 (bs, 1H, OH); 7,65 (d, J= 9.2Hz, 1H, H_{Ar}); 7,52 (m, 5H, H_{Ar}); 6,92 (d, J= 8.9Hz, 1H, H_{Ar}); 5,27 (s,1H); 4,07 (m, 2H); 2,74 (m,2H); 2,19 (s, 3H);
2,01 (m,2H).
¹³C-NMR (100MHz, DMSO-d₆) [δ ppm]: 163,14; 159,05; 157,74; 152,74; 141,26; 128,60; 128,30; 125,80; 121,65; 111,90; 107,14; 87,23; 67,32; 8,08.
ESI-MS (M-H⁺): 309.1

### 7-((3-chlorobenzyl)oxy)-4-hydroxy 8-methyl-2H-chromen-2-one (2-17)

The product was obtained as a light yellow solid.
Yield: 54 mg (64 %)
¹H-NMR (400 MHz, DMSO-d₆) [δ ppm]: 12,35 (bs, 1H, OH); 7,69 (m, 1H, H_{Ar}); 7,57 (m, 1H, H_{Ar}); 7,49 (m, 3H, H_{Ar}); 7,11 (m, 1H, H_{Ar}); 5,47 (s,1H); 5,29 (s, 2H); 2,26 (s,3H).
¹³C-NMR (100MHz, DMSO-d₆) [δ ppm]: 167,12; 162,26; 159,39; 152,54; 136,70; 129,50;128,51; 127,37; 121,25; 112,64; 109,24; 108,38; 88,47; 69,77; 8,28. ESI-MS (M+2Na⁺): 359.0

### 4-hydroxy-8-methyl-7-(2-(phenylamino)ethoxy)-2H-chromen-2-one (2-18)

The product was obtained as a colorless solid.
Yield: 60 mg (65 %)
¹H-NMR (400 MHz, DMSO-d₆) [δ ppm]: 12,35 (bs, 1H, OH); 7,67 (m,1H, H_{Ar}); 7,20 (m, 1H, H_{Ar}); 7,14 (m, 1H, H_{Ar}); 7,09 (m, 1H, H_{Ar}); 6,82 (m, 1H, H_{Ar}); 6,70 (m, 2H, H_{Ar}); 5,50 (s,1H); 4,20 (m,2H); 3,65 (m,2H); 2,19 (s, 3H).
¹³C-NMR (100MHz, DMSO-d₆) [δ ppm]: 167,12; 162,26; 159,64; 152,54; 136,70; 129,02;128,51; 127,37; 121,40; 112,54; 109,24; 108,40; 88,65; 65,89; 42,45; 8,14.

### 4-hydroxy-8-methyl-7-(pyridin-3-ylmethoxy)-2H-chromen-2-one (2-19)

The product was obtained as a colorless solid.
Yield: 0,13 g (48 %)
¹H-NMR (250 MHz, DMSO-d₆) [δ ppm]: 12,46 (bs, 1H, OH); 8,92 (s, 1H, H_{Ar}); 8,80 (m, 1H, H_{Ar})
8,32 (m,1H, H_{Ar}); 7,78 (m, 1H, H_{Ar}) 7,71 (d, J= 9.2Hz, 1H, H_{Ar}); 7,18 (d, J= 8.6Hz, 1H, H_{Ar}); 5,54(s,1H); 5,41 (m, 2H); 2,24 (s, 3H).
¹³C-NMR (100MHz, DMSO-d₆) [δ ppm]: 166,08; 162,19; 158,83; 152,55; 145,08; 140,49;134,74; 125,58; 121,48; 112,84; 109,72; 108,30; 88,69; 66,90; 8,25.
ESI-MS (M-H⁺): 282.0

### General procedure for the synthesis of the coumarin derivatives 2-20, 2-21

N-Boc protected amino acid (0.9 eq) was dissolved in dry DMF under argon atmosphere and to this solution EDC*HCl (0.9 eq) was added, followed by 1H-1,2,3-benzotriazol-1-ol (0.9 eq). After the added reagents were dissolved 3-amino-4,7-dihydroxy-8-methyl-2H-chromen-2-one (1 eq),dissolved in 5 ml dichloromethane, 5 ml DMF and 1 ml *N,N*-Diisopropylethylamine were added. The mixture was stirred at room temperature for 24-48h.The solvent was evaporated and the residue was dissolved in CH₂Cl₂, extracted with saturated aqueous NaCl, dried over MgSO₄, filtrated and evaporated. The light yellow oil was purified via column chromatography.

### tert-butyl(1-((4,7-dihydroxy-8-methyl-2-oxo-2H-chromen-3-yl)amino)-1-oxo-3-phenylpropan-2-yl)carbamate (2-20)

The product was obtained as colourless foam.
Yield: 0,13 g (50 %)
¹H-NMR (250 MHz, DMSO-d₆) [δ ppm]: 11,98 (bs, 1H, OH); 10,38 (s, 1H, OH); 9,46 (s,1H,NH);7,53 (d, J=9.3Hz, 1H, H_{Ar}); 7,27 (m, 5H, H_{Ar}), 6,97 (m, 1H, NH); 6,83 (d, J= 8.9Hz, 1H, H_{Ar}); 4,46(s,1H); 2,65 (m, 2H); 2,16 (s,3H); 1,24 (m, 9H).
¹³C-NMR (100MHz, DMSO-d₆) [δ ppm]: 160,97; 152,89; 148,12; 139,49; 129,36; 127,99;121,60; 116,00; 115,08; 110,68; 79,66; 58,55; 28,08; 8,04.
ESI-MS (M-Na⁺): 477.08. HRMS (M + Na⁺) calculated C₂₄H₂₅N₂O₇Na: 477.16322 found: 477.16301.

### tert-butyl(1-((4,7-dihydroxy-8-methyl-2-oxo-2H-chromen-3-yl)amino)-4-methyl-1-oxopentan-2-yl)carbamate (2-21)

The product was obtained as colourless foam.
Yield: 0,08 g (20 %)
¹H-NMR (400 MHz, DMSO-d₆) [δ ppm]: 12,16 (bs, 1H, OH); 10,47 (s, 1H, OH); 9,39 (s,1H,NH); 7,60 (d, J=9.4Hz, 1H, H_{Ar}); 7,19 (m, 1H, NH); 6,92 (d, J= 8.9Hz, 1H, H_{Ar}); 4,32 (m,1H); 2,17 (s,3H); 1,70 (m,1H); 1,57 (m,2H), 1,41 (m, 9H); 0,92 (m,6H).
¹³C-NMR (100MHz, DMSO-d₆) [δ ppm]: 160,20; 159,08; 156,98; 155,56; 150,76; 125,11;123,92; 121,56; 118,58; 118,34; 116,52; 114,99; 112,94; 112,09; 110,41; 107,94; 100,64;78,49; 52,77; 28,15; 24,30; 23,08; 21,36; 8,03.
ESI-MS (M-H⁺): 420.0 HRMS (M+K⁺) calculated C₂₁H₂₈N₂O₇K: 459.15281 found: 459.15232.

### tert-butyl(1-((4,7-dihydroxy-8-methyl-2-oxo-2H-chromen-3-yl)amino)-1-oxo-4-phenylbutan-2-yl)carbamate

The product was obtained as colourless foam.
Yield: 0,09 g (7 %)
TLC: R_{f} = 0.40 (hexane:EtOAc 2:1)
¹H-NMR (250 MHz, DMSO-d₆) [δ ppm]: 12,14 (bs, 1H, OH); 10,52 (s, 1H, OH); 9,41 (s, 1H, NH); 7,64 (d, J=8.8 Hz, 1H, Hₐᵣ); 7,30 (m, 6H, NH, Hₐᵣ); 6,95 (d, J=8.8 Hz, 1H, Hₐᵣ); 4.37 (m, 1H); 2,75 (m, 2H); 2,23 (s, 3H); 2,10 (m, 2H); 1,50 (s, 9H). ¹³C-NMR (100MHz, DMSO-d₆) [δ ppm]: 173,28; 159,00; 141,38; 128,23; 128,22; 125,73; 121,47; 111,98; 110,34; 107,84; 100,35; 78,46; 78,45; 54,02; 54,00; 33,83; 31,37; 28,09; 7,95.
ESI-MS (M-H⁺): 467.1 HRMS (M+K⁺) calculated C₂₅H₂₈N₂O₇K: 507.15281 found: 507.15229.

### N-(4,7-dihydroxy-8-methyl-2-oxo-2H-chromen-3-yl)-[1,1'-biphenyl]-3-carboxamide

The product was obtained as pale yellow powder.
Yield: 0.06 g (5 %)
TLC: R_{f} = 0.39 (hexane:EtOAc 2:1)
¹H-NMR (400 MHz, DMSO-d₆) [δ ppm]: 11,71 (bs, 1H, OH); 10,44 (s, 1H, OH); 9,58 (s, 1H, NH); 8,34 (s, 1H, Hₐᵣ); 7.97 (d, J=7.6 Hz, 1H, Hₐᵣ); 7.91 (d, J=7.6 Hz, 1H, Hₐᵣ); 7,79 (d, J=7.2 Hz, 1H, Hₐᵣ); 7,61 (d, J=8.0 Hz, 2H, Hₐᵣ); 7,53 (t, J=7.6Hz, 2H, Hₐᵣ); 7,44 (t, J=7.2 Hz, 2H, Hₐᵣ);6.90 (d, J=8.8Hz,1H, Hₐᵣ), 2.19 (s, 3H).
¹³C-NMR (100MHz, DMSO-d₆) [δ ppm]: 166,38; 160,77; 160,48; 159,05; 151,41; 139,89; 139,44; 134,54; 129,56; 128,93; 128,83; 127,72; 127,05; 126,74; 126,20; 121,49; 111,77; 110,37; 107,83; 99,69; 8.00.
ESI-MS (M-H⁺): 386.2 HRMS (M+H⁺) calculated C₂₃H₁₈N₂O₅: 388.11795 found: 388.11797.

### N-(4,7-dihydroxy-8-methyl-2-oxo-2H-chromen-3-yl)-3-phenoxybenzamide

The product was obtained as pale yellow powder.
Yield: 0,09 g (22 %)
TLC: R_{f} = 0.37 (DCM:MeOH 98:2)
¹H-NMR (400 MHz, DMSO-d₆) [δ ppm]: 11,68 (bs, 1H, OH); 10,42 (s, 1H, OH); 9,45 (s, 1H, NH); 7,79 (d, J=7.6 Hz, 1H, Hₐᵣ); 7,63 (s, 1H, Hₐᵣ); 7,57 (d, J=8.8 Hz, 1H, Hₐᵣ); 7,54 (t, J=8.0 Hz, 1H, Hₐᵣ); 7,43 (t, J=7.6 Hz, 2H, Hₐᵣ), 7.25 (dd, J₁=8.4 Hz, J₂=2.0 Hz, 1H, Hₐᵣ); 7,18 (t, J=7.6 Hz, 1H, Hₐᵣ); 7,06 (d, J=7.6 Hz, 2H, Hₐᵣ); 6,88 (d, J=8.8 Hz, 1H, Hₐᵣ); 2,17 (s, 3H).
¹³C-NMR (100MHz, DMSO-d₆) [δ ppm]: 165,64; 160,70; 160,58; 159,03; 156,44; 156,43; 151,40; 135,90; 130,06; 129,83; 123,61; 123,00; 121,46; 118,61; 118,05; 111,74; 110,34; 107,80; 99,51; 7,97.
ESI-MS (M-H⁺): 402.0 HRMS (M+H⁺) calculated C₂₃H₁₇NO₆: 404.11286 found: 404.11355.

### methyl 3-(isopentyloxy)benzoate

Yield: 1,04 g (71 %)
TLC: Rf= 0,63 (hexane: EtOAc 9:1)
¹H-NMR (250 MHz, DMSO-d₆) [δ ppm]: 7,53 (m, 1H, Hₐᵣ); 7,43 (m, 2H, Hₐᵣ); 7,24 (m, 1H, Hₐᵣ); 4,04 (t, J= 6.8 Hz, 2H, OCH₂); 3,84 (s, 3H, OCH₃); 1,78 (m, 1H); 1,62 (q, J=6.5 Hz, 2H); 0,93 (d, J=6.8 Hz, 6H).
¹³C-NMR (100MHz, DMSO-d₆) [δ ppm]: 165,95; 158,61; 130,85; 129,82; 121,15; 119,64; 114,26; 66,07; 52,07; 37,22; 24,46; 22,30.
ESI-MS (M+H⁺): 223.1

### 3-(isopentyloxy)benzoic acid

Yield: 0.84 g (86 %)
¹H-NMR (250 MHz, DMSO-d₆) [δ ppm]:12,97 (bs, 1H, OH); 7,51 (m, 1H, Hₐᵣ); 7,39 (m, 2H, Hₐᵣ); 7,18 (m, 1H, Hₐᵣ); 4,03 (t, J=6.5 Hz, 2H, OCH₂); 1,78 (m, 1H); 1,62 (q, J=6.5 Hz 2H); 0,93 (d, J=6.5 Hz, 6H).
¹³C-NMR (100MHz, DMSO-d₆) [δ ppm]: 167,04; 158,54; 132,06; 129,58; 121,33; 119,23; 114,39; 66,00; 37,25; 24,48; 22,31.
ESI-MS (M-H⁺): 206.9

### General Procedure for synthesis of 3-alkoxybenzoic acids

Step1: To a solution of methyl 3-hydroxybenzoate (1,00 g, 6,6 mmol, 1 eq) and potassium carbonate (0, 55 g, 13,2 mmol, 2 eq) in 30 ml acetone was added the corresponding substituent as chloride, bromide or iodide (7,2 mmol, 1,1 eq). The solution was refluxed overnight. The solution was then filtered and concentrated. The methyl 3-alkoxybenzoates were purified through column chromatography (hexane: EtOAc 9:1).

Step2: To the methyl 3-alkoxybenzoate (6 mmol, 1eq) was added 30 ml of THF/MeOH/H₂O (1:1:2) and potassium hydroxide (30 mmol, 5 eq). This solution was refluxed for 4 hours. The mixture was then acidified using 1N HCl solution. The product was then extracted with DCM (3x), dried over magnesium sulfate, and concentrated to yield the corresponding 3-alkoxybenzoic acid (80-96 %) pure enough to carry on to the next step.

### 1-(4-(Benzyloxy)-2-hydroxyphenyl)ethanone

2',4'-dihydroxy-acetophenon (10.6 g, 68 mmol, 1 eq) was dissolved in acetone, treated with (11.4 g, 66.6 mmol, 0.98 eq) benzyl bromide , (10.3 g, 74.8 nmmol, 1.1 eq) K₂CO₃ and refluxed for 4 h.

The incurred precipitate was removed through filtration, the filtrate was evaporated and the crude oily product purified via column chromatography yielding 1-(4-(benzyloxy)-2-hydroxyphenyl)ethanone.

Yield: 15.0 g (92 %)
¹H-NMR (250 MHz, DMSO-d₆) [δ ppm]: 12,61 (s, 1H, OH); 7,84 (d, J=9 Hz, 1H, Hₐᵣ); 7,34-7,44 (m, 5H, Hₐᵣ); 6,55-6,63 (m, 2H, Hₐᵣ); 5,19 (s, 2H, OCH₂); 2,56 (s, 3H, CH₃).
¹³C-NMR (100MHz, DMSO-d₆) [δ ppm]:
ESI-MS (M-H⁺): 242.9

### 7-(benzyloxy)-4-hydroxy-2H-chromen-2-one

1-(4-(Benzyloxy)-2-hydroxyphenyl)ethanone (4.9 g, 20.3 mmol, 1 eq) was dissolved in 70 ml toluene and added dropwise to an 80 °C solution containing (2.0 g, 81.2 mmol, 4 eq) NaH (w=60 %) in 130 ml toluene. After addition of (9.9 ml, 81.2 mmol, 4 eq) diethyl carbonate the reaction was refluxed for 16 h at 140°C, cooled down to 0°C, transferred into a beaker filled with ice water and the incurred precipitate was suction filtered. The filtrate was acidified with 2N aqueous HCl and extracted with ethyl acetate. The organic phase was dried over MgSO₄, filtered and evaporated under reduced pressure, yielding 7-(benzyloxy)-4-hydroxy-2H-chromen-2-one.

Yield: 4.9 g (90 %)
¹H-NMR (250 MHz, DMSO-d₆) [δ ppm]: 12.37 (bs, 1H, OH); 7.72 (d, J=8.5 Hz, 1H, Hₐᵣ); 7,38-7,49 (m, 5H, Hₐᵣ); 6,97-7,03 (m, 2H, Hₐᵣ); 5,46 (s, 1H); 5,21 (s, 2H, OCH₂).
¹³C-NMR (100MHz, DMSO-d₆) [δ ppm]: 166,05; 162,27; 161,85; 155,28; 136,28; 128,49; 128,06; 127,87; 124,34; 112,38; 109,19; 101,42; 88,59; 69,84.
ESI-MS (M-H⁺): 267.1

### 7-(benzyloxy)-4-hydroxy-3-nitro-2H-chromen-2-one

To a suspension of 7-(benzyloxy)-4-hydroxy-2H-chromen-2-one (2.2 g, 8 mmol, 1 eq) and sodium nitrite (0.03 g, 0.4 mmol, 0.05 eq) in acetic acid (10 mL) was added 65% nitric acid (1.1 mL) in small portions with stirring at room temperature. The reaction mixture was heated with stirring at 60 °C for 15 min and the resulting solid was filtered and washed with water to give the product as a light yellow solid.

Yield: 1.7 g (69 %)
¹H-NMR (250 MHz, DMSO-d₆) [δ ppm]: 9,92 (bs, 1H, OH); 7,81 (d, J=8.7 Hz, 1H, Hₐᵣ); 7,33-7,48 (m, 5H, Hₐᵣ); 6,86-6,93 (m, 2H, Hₐᵣ); 5,18 (s, 2H, CH₂).
¹³C-NMR (100MHz, DMSO-d₆) [δ ppm]:
ESI-MS (M-H⁺): 311.8

### 3-amino-4,7-dihydroxy-2H-chromen-2-one

A suspension of 7-(benzyloxy)-4-hydroxy-3-nitro-2H-chromen-2-one (1.7 g; 5.4 mmol, 1 eq) and Pd/C (w= 10 %, 0.2 g) in 120 ml ethanol were stirred under a H₂-atmosphere at room temperature overnight. The suspension was then filtered through celite, which was washed with plenty of methanol.

Yield: 1.0 g (98 %)
¹H-NMR (400 MHz, DMSO-d₆) [δ ppm]: 7,64 (d, J=8.5 Hz, 1H, Hₐᵣ); 6,65 (dd, J₁=8.5 Hz, J₂=2.2 Hz, 1H, Hₐᵣ); 6.53 (d, J=2.2 Hz, 1H, Hₐᵣ).
¹³C-NMR (100MHz, DMSO-d₆) [δ ppm]:
ESI-MS (M-H⁺): 193,8

### N-(4,7-dihydroxy-2-oxo-2H-chromen-3-yl)-3-phenoxybenzamide

TLC: R_{f} = 0.41 (hexane:EtOAc 2:1)
¹H-NMR (400 MHz, DMSO-d₆) [δ ppm]: 11,82 (bs, 1H, OH); 10,53 (s, 1H, OH); 9,42 (s, 1H, NH); 7,78 (d, J=7.7 Hz, 1H, Hₐᵣ); 7,70 (d, J=8,7 Hz, 1H, Hₐᵣ); 7,62 (s, 1H, Hₐᵣ); 7,53 (t, J=7,9 Hz, 1H, Hₐᵣ); 7,43 (t, J=7.8 Hz, 2H, Hₐᵣ); 7,24 (dd, J₁=8.1 Hz, J₂= 1.9 Hz, 1H, Hₐᵣ); 7,18 (t, J= 7.4 Hz, 1H, Hₐᵣ); 7,05 (d, J=8.1 Hz, 2H, Hₐᵣ); 6,81 (dd, J₁=8.7 Hz, J₂= 1.8 Hz, 2H, Hₐᵣ); 6,71 (d, J=1.8 Hz, 1H, Hₐᵣ).
¹³C-NMR (100MHz, DMSO-d₆) [δ ppm]: 165,70; 161,38; 156,53; 156,50; 153,54; 136,03; 130,29; 130,15; 129,91; 125,04; 123,70; 123,04; 118,70; 118,09; 112,85; 101,86.
ESI-MS (M-H⁺): 388.1 HRMS (M+H⁺) calculated C₂₂H₁₆N₁O₆: 390.09721 found: 390.09650.

### B Biological Examples

### Material and Methods

### AlphaScreen interaction displacement assay

The AlphaScreen assay contained 50 mM Tris (pH 7.5), 100 mM NaCl, 0.01% Tween 20, 2 mM DTT, 1 % DMSO and 2-30 nM GST-LC3B and biotin-LIRtide 2-30 nM. The concentrations of GST-LC3B and biotin-LIRtide were experimentally chosen from a cross-titration assay exploring the different combinations of for each batch of protein used and when a new batch of buffer was prepared. The library comprising FDA-approved drugs was from Prestwick, while the library comprising the 144000 diverse set of small compounds was a hitfinder selection from Maybridge.

### Protein purification

LC3B in pETM60 is expressed as N-terminal Ubiquitin His tag fusion proteins in *Escherichia coli* BL21 DE3. Cells were grown at 37°C to an OD₆₀₀ of 0.5, followed by induction with 0.5 mM isopropyl-β-d-thiogalactoside and further incubation at 25 °C for 20 h before harvesting. Cells were lysed by sonication in lysis buffer (25 mM Tris, 100 mM NaCl, pH 8.5), and the lysate was cleared by centrifugation (15000 x g, 4°C, 40 min). The expressed protein was purified by *TALON* Metal Affinity Resin (Clontech), cleavaged by TEV Protease, and further purified by size exclusion chromatography (HiLoad 16/600 Superdex 75 column, GE Life Sciences) in 20 mM Tris, 100 mM NaCl, pH 8.5.

### Crystallization, X-ray data collection and structure determination

The crystals of LC3A in complex with compound 2-0 were obtained using 25% Poly ethylene glycol 2000 MME, 0.3M Sodium acetate trihydrate, 0.1 M Sodium acetate pH 5.5 as a reservoir solution by the sitting-drop vapor diffusion method at 293 K. Diffraction data were collected at Diamond Light Source, beam line I02 and processed with XDS. The crystal structure of LC3A in complex with compound 2-0 was determined by molecular replacement using the LC3A structure (PDB: 3ECI) as a search model. Manual model building and refinement were done with Coot, CCP4 software suite and Phenix. The final statistics of refined models are shown in Suppl. Table 1.

### General procedure for isolation and purification of the LC3 and GABARAP proteins for ITC and NMR experiments

The expression of Ub-fused LC3 and GABARAP proteins was achieved in LB or M9 media as previously described [21]. Ni-NTA affinity chromatography was applied to isolate fused constructs, the self-made TEV protease was added to the fused protein fractions in 1:100 or higher ratio (TEV:protein, w/w) for overnight cleavage at 16°C in presence of 10 mM **0**-mercaptoethanol (El-Met) and 1 mM EDTA (usually, >95% fusions was cleaved). The following purification steps comprised a second Ni-NTA chromatography (after dialysis against the desired buffer for more than 3 hours at room temperature, >1:100 protein:buffer volume ratio) and/or size-exclusion chromatography in desired buffer using Superdex S75 or S200 prepacked HR16-60 columns).

For the CD, NMR and ITC experiments, the proteins were equilibrated with the buffer containing 50 mM Na₂PHO₄ (pH 7.0) and 100 mM NaCl with ~5 mM protease inhibitors cocktail (PIC).

Compounds were dissolved in pure DMSO to concentration of 50 mM, from which the aliquots were used to prepare the water-based stocks for ITC and NMR.

### Isothermal titration calorimetry (ITC) experiments

All ITC experiments were performed at 25 °C using a VP-ITC microcalorimeter (MicroCal Inc, MA, USA) and analyzed with the ITC-Origin 7.0 software (MicroCal Inc.) based on a "one-site binding" reaction. Both compound 2-0 and ***13*** at concentrations of 2000 µM were titrated to 25-30 µM of LC3A and LC3B proteins, respectively (10 µL/injection). Dilution heat of both compounds was measured upon titration of the stocks into buffer.

### NMR spectroscopy

All experiments were performed on a Bruker Avance spectrometer operating at a proton frequency of 500, 600 and 700 MHz at 298 K. Titrations were performed with 150 µM ¹⁵N-labelled LC3B samples to which the compounds of compound 2-0 family were added stepwise to 8 times excess towards LC3B. ¹H-¹⁵N heteronuclear single quantum coherence (HSQC) spectra were recorded at each step. The compound I-1 compounds were added directly to 5-10 times excess towards LC3B and incubated at 37°C for various time.

### GST-tagged protein purification

A gene encoding for human LC3B protein inserted in a pET-GST expression vector was used as a template. Rosetta (DE3) competent cells were transformed by started procedure, plated on an LB agar plate with ampicillin (100 µg/ml) and left overnight at 37 °C. Overnight culture was prepared from a single colony and shaken at 180 rpm at 37 °C. LB medium with ampicillin was inoculated with overnight culture (10 ml per 500 ml of LB) and shaken until the optical density at 600 nm (OD₆₀₀) reached a value of approximately 0.6. Protein expression was then induced with 400 µM IPTG and further incubated for 4 hours. Bacterial culture was centrifuged for 20 minutes at 6000 rpm at 4 °C. The pellet was then resuspended in lysis buffer (20 mM TRIS-HCl pH 7.5, 10 mM EDTA, 5 mM EGTA, 150 mM NaCl, 0.5 % NP40, 1 % Triton X-100, 1 mM DTT, 2 mg/ml lysozyme, 0.05 U/µl benzonase, 1x protease inhibitor cocktail). Lysate was then stored at -20 °C over night. Sample was thawed in water bath and sonicated on ice (40 % amplitude, 30 seconds pulse/30 seconds pause cycle). Lysate was centrifuged at 10000xg at 4 °C for half an hour. Meanwhile, 500 µl of glutathione sepharose beads was equilibrated with lysis buffer and then added to the supernatant. The mixture was incubated and rotated at room temperature for 2 hours. After binding of the protein to the beads, beads were spun down at 500xg at 4 °C for 5 minutes. Pellet was then washed with cold wash buffer at least four times (20 mM Tris-HCl pH 7.5, 10 mM EDTA, 5 mM EGTA, 150 mM NaCl, 1 mM DTT), dried and stored in 5 % glycerol at 4 °C.

### GST-pulldown

293T cells were treated with 100µM compound I-1 or DMSO as control for 1 hour and lysed with MCLB lysis buffer (50 mM Tris pH 7.4, 150 mM NaCl, 0.5 % NP40 ) followed by preclearing of the cell lysate with beads for 1 hour and incubation with purified GST-tagged LC3B over night. Afterwards, beads were washed five times with wash buffer, proteins were heat denatured in Lämmli buffer and SDS-PAGE and western blotting was performed.

### Cell culture experiments

U2OS (osteosarcoma), HEK293T (human embryonic kidney) and PANC1 (pancreatic epithelioid carcinoma) cells were grown in DMEM medium, HCT116 (colorectal carcinoma) cells in McCoy's medium and A549 (lung carcinoma) cells in F12K medium. All media were additionally supplemented with 1 % (v/v) glutamine, 10 % (v/v) FBS and 1 % (v/v) penicillin/streptomycin. Cells were grown in 10 cm Petri dishes at 37 °C in 5 % CO₂.

### Westernblot

Proteins were separated on 4-20 % SDS-PAGE and electrophoretically transferred to nitrocellulose membranes in 192 mM glycine, 25 mM Tris, 1.3 mM SDS and 20 % (v/v) methanol. The membrane was blocked by incubating in Tris-buffered saline (TBS) buffer (pH 7.6) containing 5 % (wt/vol) non-fat dry milk and sequentially incubated with primary antibodies (anti-LC3B (Cell signaling, #2275) and anti-ATG13 (M183-3, MBL). The membranes were incubated with horseradish peroxidise conjugated secondary anti-mouse or anti-rabbit. Proteins were visualized with ECL reagent according to the manufacturer's instructions. Luminescent signal of the bands on the membranes was captured using a Cawomat 2000 IR dark box (Cawo).

### Cell lysates

U2OS cells were treated with 100 µM compound 2-0 and compound I-1 as well as with and without 250 nM torin1, 100 nM BafA1 or DMSO for 2 hours. After harvesting, cells were lysed with radioimmunoprecipitation assay (RIPA) buffer (50 mM Tris-HCl, pH 7.5, 150 mM NaCl, 0.5 % deoxycholate, 1 % Triton X-100, 0.1 % SDS and 1x protease inhibitors) and left on ice for 30 minutes. Cell lysates were clarified by centrifugation at 10000xg for 20 min at 4 °C. Protein concentrations of the supernatants were determined by BCA assay and the absorbance was measured at 562 nm. SDS-PAGE and WB was performed.

### Drug screening and immunocytochemistry

For screening purposes, cells were seeded in 384 well plates and left over night for attachment. Next day, cells were treated in quadruplicates for 1 hour with compound 2-0 and compound I-1 (100 µM f.c.). For controls, cells were treated with 250 nM torin and/or 100 nM BafA1 for 2 hours.

Afterwards, medium was removed and 4 % (w/v) paraformaldehyde (PFA) in PBS buffer was added to the cells for 20 minutes, incubated at room temperature. Cells were permeabilized with 0.1 % (v/v) Triton X-100 for 10 min at room temperature and washed with PBS. Cells were blocked with 3 % (w/v) bovine serum albumin (BSA) in PBS for 45 minutes at 37 °C and then incubated with anti-LC3B (PM036, MBL) or anti-p62 (PM045 or M162-3, MBL) primary antibody diluted in 0.3 % BSA (1:2000 or 1:500) for 1.5-2 hours at 37 °C. Cells were washed three times with PBS. Secondary Alexa Fluor 488 - labeled antibodies (A-11008, Invitrogen) were diluted in 0.3 % BSA (1:2500) and incubated on cells for 15 minutes at 37 °C. Cells were washed once with PBS, incubated with DRAQ5 (#4084, Cell Signaling) diluted in PBS (1:5000) for 10 minutes and washed three times with PBS.

384 well plates were then analyzed with an automated confocal microscope Opera (PerkinElmer) enabling high throughput high content screening image analysis. Confocal images were taken at the Institute of Biochemistry II, Goethe University Medical School, Theodor-Stern-Kai 7, 60590 Frankfurt am Main, Germany. A water immersion 60x objective was used for optical slicing and stacking (5x0.5 µm stacks). Alexa Fluor 488 and DRAQ5 were excited with 488 and 640 nm lasers, respectively. Signal was detected with a fast gated CCD camera. Acapella software (PerkinElmer) is an algorithm based high content imaging and analysis software used in multi-parametric multiplex assays. Acapella was used to follow cell viability (i.e. number of cells) and the number of LC3B and p62 spots per cell.

### MTT cell proliferation assay

The MTT assay is a colorimetric assay for assessing cell viability. NAD(P)H-dependent cellular oxidoreductase enzymes in metabolic active cells reduce the tetrazolium dye MTT 3-(4,5-dimethylthiazol-2-yl)-2,5-diphenyltetrazolium bromide to its insoluble purple formazan. All cell types were seeded in 96 well plates and left over night for attachment. Next day, cells were treated with 100 µM compound I-1 for 24, 48, 72, and 96 hours. For controls, 1 µM staurosporine and 100 nM BafA1 were used. The assay was performed according to manufacturer's instructions and the absorbance of the formazan product was measured at 595 nm.

### Scratch assay

All cell types were seeded in 24 well plates and left over night for attachment or until they were completely confluent. The cell monolayer was scratched with the tip end and then treated with 100 µM compound I-1 for 1 hour. For controls, 100 nM BafA1or 100 µM chloroquine were added for 1 hour. The distance between the monolayers was observed by the Leica DM IL LED light microscope and 10x air-immersion objective every hour for 6 hours. The images were taken in the LAS V3.8 software.

### LC3B IF/WB assay

The LC3B protein is a ubiquitin-like protein that can be conjugated to phosphatidylethanolamine (PE) in the membrane of the incipient autophagosome. The nonlipidated and lipidated forms are usually referred to as LC3B-I and LC3B-II, respectively. The PE-conjugated form of LC3B, although larger in mass (∼17 versus ∼15 kDa), shows elevated electrophoretic mobility in SDS-PAGE gels, probably as a consequence of increased hydrophobicity. Autophagic flux is often inferred on the basis of LC3B-II turnover, measured by western blot (WB) or immunofluorescence (IF) in the presence and absence of lysosomal degradation. The relevant parameter in these assays is the difference in the amount of LC3B-II (in WB) or LC3B-positive punctae in the presence and absence of saturating levels of inhibitors, which can be used to examine the transit of LC3B-II through the autophagic pathway; if flux is occurring, the amount of LC3B-II (WB) and LC3B-positive punctae (IF) will be higher in the presence of the inhibitor. Lysosomal degradation can be prevented e.g. through the use of compounds that neutralize the lysosomal pH such as Bafilomycin A1 (BafA1) or chloroquine (CQ). Increased levels of LC3B-II (WB) or LC3B-positive punctae (IF) in the presence of interfering with autophagosome-lysosome fusion alone (e.g., with BafA1), may be indicative of autophagic flux, but to assess whether a particular treatment alters this flux, the treatment plus BafA1 must be compared with results obtained with treatment alone as well as with BafA1 alone. An additive or supra-additive effect in LC3B-II levels (WB) and LC3B-positive punctae numbers (IF) may indicate that the treatment enhances autophagic flux. Moreover, higher LC3B-II levels (WB) and LC3B-positive punctae numbers (IF) with treatment plus BafA1 compared to BafA1 alone may indicate that the treatment increases the synthesis of autophagy-related membranes. If the treatment by itself increases LC3B-II levels (WB) and LC3B-positive punctae numbers (IF), but the treatment plus BafA1 does not increase LC3B-II (WB) and LC3B-positive punctae numbers (IF) levels compared to BafA1 alone, this may indicate that the treatment induced a block in autophagic flux. This assay can be complemented by simultaneous monitoring of autophagosomal substrates or autophagosomal substrate receptors (e.g. SQSTM1/p62) by WB and/or IF. For example, an increase in LC3B-II levels in combination with the accumulation of SQSTM1/p62 is indicative of a blockage of the autophagy pathway at terminal stages.

### In vitro and cellular characterization of compounds (I) and (II)

### B1 Tetrahydrotriazine Derivatives (I)

The synthesized compounds (I) were tested regarding their affinity for LC3B using alphascreen and the cellular autophagy screening. The results are shown in Table 1a.

### Table 1-a Tetrahydrotriazine derivatives of formula (I-I)

**Table 1-a: Tetrahydrotriazine derivatives (I). The fold increase in LC3B spots was determined relative to the negative DMSO control. The fold increases for positive controls were: bafilomycin A1 (5.5), chloroquine (4.4), torin 1 (3.1), torin 1 + bafilomycin A1 (5.8), and torin 1 + chloroquine (6.3). "n.e." means no effect, and "n.t." means not tested.**

| 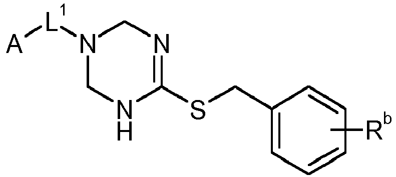 | | | | LC3B inhibition cell free | LC3B inhibition cell based |
|---|---|---|---|---|---|
| Compound | L¹ | A | R^{b} | IC₅₀[µM] | Fold increase of LC3B spots [DMSO control 1.0] |
| I-1 | CH₂ | 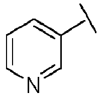 | p-Br | 6.0 | 4.9 |
| I-2 | CH₂ | 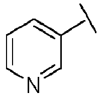 | p-Cl | 16.0 | 3.2 |
| I-3 | CH₂ | 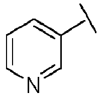 | p-F | 23.6 | 1.8 |
| I-4 | CH₂ | 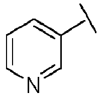 | H | 19.5 | 1.7 |
| I-5 | CH₂ | 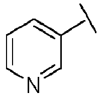 | p-CF₃ | 30.9 | 3.2 |
| I-6 | CH₂ | 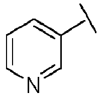 | p-OCH₃ | 12.3 | 1.8 |
| I-7 | CH₂ | 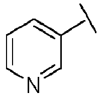 | 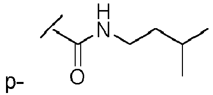 | 26.6 | 3.1 |
| I-8 | CH₂ | 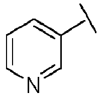 | m- Br | 21.0 | 3.0 |
| I-9 | CH₂ | 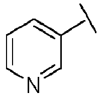 | m- OCH₃ | 18.5 | n.e. |
| I-10 | CH₂ | 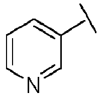 | 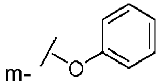 | 1.0 | 6.1 |
| I-11 | CH₂ | 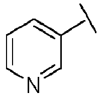 | 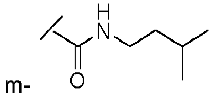 | 12.2 | n.e. |
| I-12 | CH₂ | 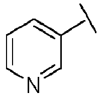 | 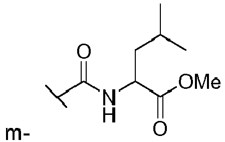 | 8.9 | 5.0 |
| I-13 | CHCH₃ | 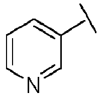 | p- Br | 54.8 | n.e. |
| I-14 | CH₂ | 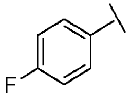 | p- Br | n.e | 5.8 |
| I-15 | CH₂ | 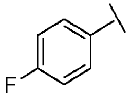 | p-Cl | n.e | 5.9 |
| I-16 | CH₂ | 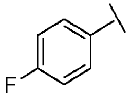 | 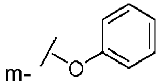 | n.e | n.e. |
| I-17 | CH₂ | 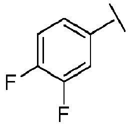 | p-Br | n.e | 4.2 |
| I-18 | CH₂ | 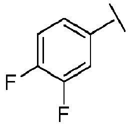 | 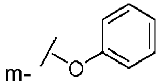 | n.e | n.t. |
| I-19 | CH₂ | 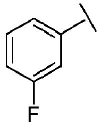 | p-Br | n.e. | n.t. |
| I-20 | CH₂ | 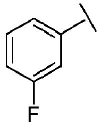 | 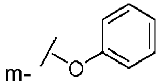 | n.e. | n.t. |

As an example of the tetrahydrotriazine derivatives (I), compound 1-1 represents an unusual mechanism of the interaction with LC3/GABARAP proteins. A 10 fold excess of compound 1-1 does not induce any significant CSPs in LC3B, however, after some time the product of compound 1-1 seem to covalently attach to the protein surface and to induce stable conformational changes, which cannot be reversed after buffer exchange (Figure 10 and supplementary Figure 8). Additionally, modification of compound 1-1 resulted in a number of compounds that enhance LC3B binding and also induce covalent modification.

### Compound 1-1 blocks autophagic flux in U2OS cells

To shown the effect of compound 1-1 on autophagy in cells, both confocal microscopy and western blotting were used. Human osteosarcoma U2OS cells were treated for 1 hr with 100 µM of compound 1-1 and as a control, with and without Bafilomycin A1 (BafA1; 100 nM), an inhibitor of the lysosomal H⁺ ATPase. As expected, BafA1 alone increased the amount of LC3B-positive spots and lipidated LC3B (LC3B-II) due to blockage of autophagic flux (Figure 11A-D). Treatment with compound 1-1 increased both the amount of LC3B-II protein (Figure 11 D) and LC3B spots in U2OS cells (Figure 11A,B). Notably, this increase could be due to induction of autophagy or blockage of autophagic flux. However, numbers of LC3B spots and levels of LC3B-II did not increased further when cells were treated with compound 1-1 and BafA1 together (Figure 11A, 11B, 11D), indicating that compound 1-1 blocks autophagic flux.

### Compound 1-1 inhibits binding of LIR-domain containing proteins to LC3B

To show that compound 1-1 inhibits binding of LC3B to LIR containing proteins in cells, pull down assays were performed with recombinatly purified GST-LC3B fusion protein. Treatment of HEK293T cells with compound I-1 diminished binding of p62 and ATG13, two LIR-domain containing proteins, to GST-tagged LC3B to approximately 50 % when compared to DMSO control in GST-pull down assays (Figure 11E, 11 F)

### Compound 1-1 effects on cell viability in HCT116, PANC1 and A549 cell lines

To show that inhibition of autophagy by compound 1-1 leads to decreased cell viability, cancer cell lines (PANC1, pancreatic epithelioid carcinoma; HCT116, colorectal carcinoma; A549, lung carcinoma) that are dependent on autophagy for tumor growth were used. In all three cell lines the number of LC3B and p62 spots was increased after treatment (1 hr, 100 µM) with compound 1-1 (Figure 12). Scratch and MTT assays were performed. For the latter, cells were treated with different concentrations of compound 1-1 for 96 hr (Figure 13). BafA1 and staurosporine, an inducer of apoptosis, were used as positive controls. After 24 hours, only U2OS cells had decreased viability with 100 µM treatment with compound 1-1. After 48 hours, U2OS cells had similar absorbance values as the positive controls, while out of three other cell lines, only A549 was unaffected. After 72 and 96 hours however both PANC1 and A549 had reduced cell viability for approximately 50 %, while U2OS and HCT116 had almost no cell survival. In the scratch assay, chloroquine and BafA1 were used as positive control. Chloroquine is a lysosomotropic agent that preferentially accumulates in the lysosomes

In contrast the following tetrahydrotriazine derivatives not covered by formula (I) did not show any effect.

Derivatives which are not preferred (Ih) modifications on the 3-pyridinyl moiety are listed in the following table

| 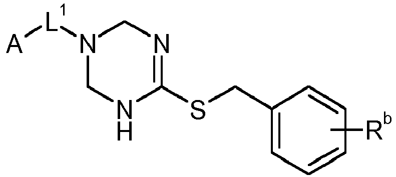 | | | |
|---|---|---|---|
| | L¹ | A | R^{b} |
| I-21 | CH₂ | 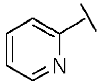 | p- Br |
| I-22 | CH₂ | 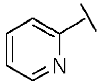 | p- F |
| I-23 | CH₂ | 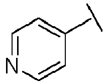 | p- Br |
| I-24 | CH₂CH₂ | 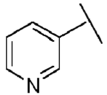 | p- Br |
| I-25 | CH₂ | 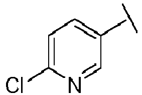 | p- Br |
| I-26 | CH₂ | 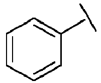 | p- Br |
| I-27 | CH₂ | 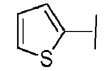 | p-Br |
| I-28 | CH₂ | 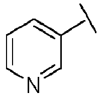 | p-COOH |
| I-29 | CH₂ | 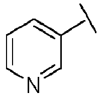 | m-COOH |

### B2 Coumarin Derivatives (II)

Coumarin Derivatives (II) were tested regarding their affinity for LC3B using alphascreen and a cellular autophagy screening assay. Results are shown in Table 2a.

**Table 2a Coumarin Derivatives (II)**

| 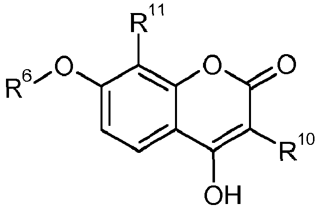 | | | | LC3B Inhibition cell free [µM] | LC3B Inhibition cell based |
|---|---|---|---|---|---|
| Compound | R⁶ | R¹¹ | R¹⁰ | IC₅₀[µM] | Fold increase of LC3B spots |
| 2-0 | 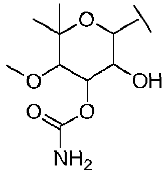 | CH₃ | 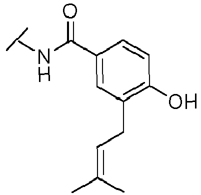 | 21.7 | 1.9 |
| 2-1 | H | CH₃ | 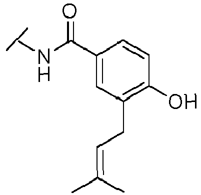 | 87.0 | 1.2 |
| 2-2 | 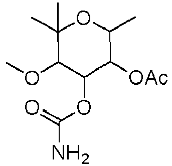 | CH₃ | 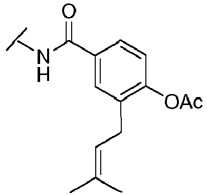 | 19.3 | 1.5 |
| 2-3 | 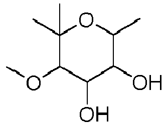 | CH₃ | 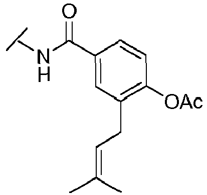 | 31.7 | 1.3 |
| 2-4 | 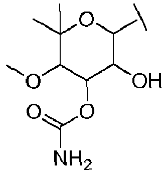 | CH₃ | 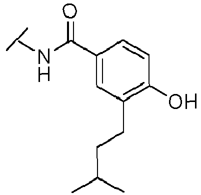 | 4.8 | 1.2 |
| 2-5 | 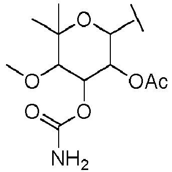 | CH₃ | 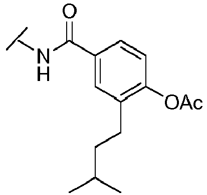 | 19.0 | 1.5 |
| 2-6 | H | CH₃ | 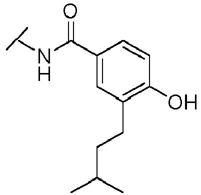 | 68.2 | 1.0 |
| 2-7 | 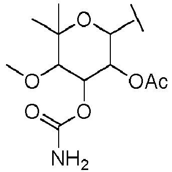 | CH₃ | 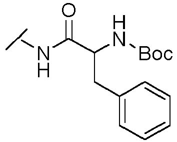 | 145.0 | 1.0 |
| 2-8 | H | CH₃ | 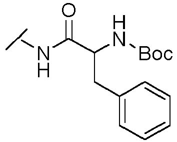 | 122.0 | 1.0 |
| 2-9 | H | CH₃ | 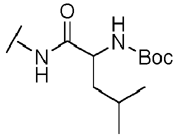 | 73.3 | 1.2 |
| 2-10 | H | CH₃ | 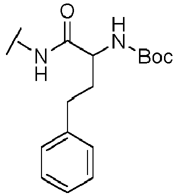 | 155.4 | 1.0 |
| 2-11 | H | CH₃ | 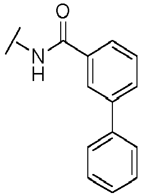 | 63.0 | 1.1 |
| 2-12 | H | CH₃ | 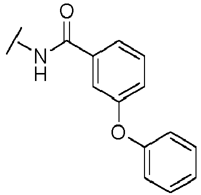 | 54.0 | 1.1 |
| 2-13 | H | H | 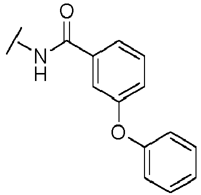 | 54.1 | 1.1 |

Table 2: Coumarin derivatives (II). The fold increase in LC3B spots was determined relative to the negative DMSO control. The fold increases for positive controls were: bafilomycin A1 (5.5), chloroquine (4.4), torin 1 (3.1), torin 1 + bafilomycin A1 (5.8), and torin 1 + chloroquine (6.3)

### NMR and ITC experiments

The titrations of compound 2-0 by ITC and NMR revealed the strong tendency of the compound to bind LC3 proteins with K_{D} values higher than the K_{D} values shown for the most effective LIR peptides (5-50 µM vs 0.5-5 µM) as reported by (Rosenknop A. et al., J. Mol.Biol., 2011, 410: 477-487 and Rogov V.V. et al., Biochem J., 2013, 454: 459-466). However, these K_{D} values are still lower in comparison to some natural LIRs (Novak I. et al., EMBO Rep., 2010, 11: 45-51).

NMR titration experiments to visualize LC3B residues affected by the interaction with compound 2-0 and to map those on the LC3B structure (Figure 3B S2A,S2B) were performed.

Significant chemical shift perturbations (CSP) of LC3B backbone HN protons were observed, predominantly in the fast exchange mode. Only a few resonances show intermediate exchange behavior (like V58 in Figure 3B). This observation, as well as K_{D} values calculated from the CSP, are in agreement with the ITC data (K_{D} and **A**S values), indicating the self-consistency of the data. CSP mapping on the 3D LC3B structure indicates patterns similar to the ones described previously for many peptides (see also Figure 3B right plot for LC3B/p62 peptide CSP), with the largest CSPs observed in hydrophobic pocket 2 (HP2), I -strand 2 and adjacent loops. The residues within HP1 are not very strongly perturbed in comparison with the CSP data for LIR-containing peptides; this fact is in agreement with structural data, where the sugar moiety of compound 2-0 just touches the HP1 but does not penetrate as deep inside as LIR's aromatic residues do. Altogether, one can see a significant binding improvement for compound 2-0 in comparison with the other compounds tested for LC3/GABARAP proteins binding with K_{D} values in the millimolar range (Thielmann Y. et al., Chembiochem 2008, 9: 1767-1775). Compound **2-13** has an at least 5 times increased affinity to LC3B. This is supported by NMR titration experiments, in which the LC3B backbone HN protons are predominantly in intermediate exchange mode (Figure 9B and supplementary Figure 5). The saturation of the LC3B resonances appears at lower concentrations of the small molecule and the CSP values are higher upon interaction with **2-13**, indicating stronger binding. The directions of the CSPs remain however mostly the same.

## Claims

1. A compound, which is
a) a tetrahydrotriazine derivative of the formula (I), 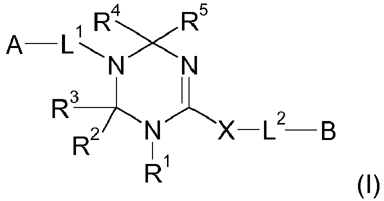 a tautomer, a pharmaceutically acceptable salt, a solvate or hydrate thereof, or
b) a coumarin derivative of the formula (II), 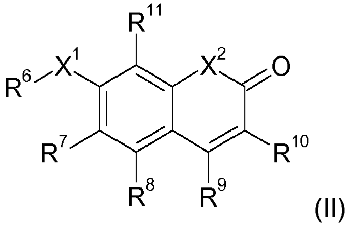 a tautomer, a pharmaceutically acceptable salt, solvate or hydrate thereof, for use in a method treating or preventing a pathologic condition associated with a pathologic cell or tissue having upregulated autophagic activity, wherein in formula (I) the symbols have the following meanings:
A is 3-pyridyl, 3-fluorophenyl, 3,4-difluorophenyl or 4-fluorophenyl;
L¹ is CH₂, CH(CH₃), CHSiR^{a}₃, CH(halogen), C(halogen)₂-, CO-, CS-, C₂-C₄-alkylene, which is unsubstituted or substituted with one or more halogen, with =O or with =S, or is a bond;
X is S; SO, SO₂ or O;
L² is -CH₂-, -CH(CH₃)-, -CH(halogen)-, -C(halogen)₂-, C₂-C₄-alkylene, which is unsubstituted or substituted with one or more halogen, or is a bond;
R¹ is H, C₁-C₈-alkyl, benzyl or phenyl, wherein alkyl is unsubstituted or substituted with one or more halogen and wherein benzyl and phenyl is unsubstituted or substituted with one or more substituents selected from halogen and C₁-C₄-alkyl;
R², R³, R⁴, R⁵ are each independently H or C₁-C₄-alkyl or R² and R³ and/or R³ and R⁴ together are O or S;
each R² is independently halogen, C₁-C₆-alkyl, C₁-C₆-haloalkyl, C₁-C₆-alkoxyalkyl, C₂-C₆-alkenyl, C₂-C₆-haloalkenyl, C₂-C₆-alkynyl, C₂-C₆-halo-alkynyl, C₃-C₈-cycloalkyl, C₃-C₈-halocycloalkyl, C₁-C₆-haloalkoxyalkyl, phenyl, a 3-, 4-, 5-, 6- or 7-membered saturated, partially unsaturated or aromatic heterocyclic ring containing 1, 2 or 3 heteroatoms or heteroatom groups selected from N, O, S, NO, So, SO₂;
B is C₆-C₁₀-aryl, 5- or 6-membered heteroaryl, C₅-C₈-cycloalkyl, 5 to 7 membered heterocyclyl, C₁-C₈-alkyl, which groups are unsubstituted or substituted with one or more substituents R^{b}, or B is H;
each R^{b} is independently halogen, CN, N₃, OCN, NCO, CNO, SCN, NCS, C₁-C₆ alkyl, C₃-C₇ cycloalkyl, C₃-C₇ heterocyclyl, C₂-C₆ alkenyl, C₂-C₆ alkynyl, C₃-C₇ aryl, C₃-C₇ heteroaryl, C₄-C₁₅ aralkyl, C₄-C₁₅ heteroarylalkyl, C₄-C₁₅ cycloalkylalkyl, C₄-C₁₅ heterocyclylalkyl, C(O)R^{bb}, C(O)OR^{bb}, C(O)N(R^{bb}₂), C(O)N(R^{bb})OR^{bb}, C(O)N(R^{bb})N(R^{bb}₂), C(NR^{bb})N(R^{bb}₂), C(NR^{bb})N(R^{bb})OR^{bb}, C(NR^{bb})N(R^{bb})N(R^{bb}₂), C(R^{bb})N(R^{bb}), C(NR^{bb}₂)NOR^{bb}, C(NR^{bb}₂)NOC(O)R^{bb}, C(R^{bb})NN(R^{bb}₂), CR^{bb}NOR^{bb}, OR^{bb}, OC(O)R^{bb}, OC(O)N(R^{bb}₂), SR^{bb}, S(O)R^{bb}, S(O)₂R^{bb}, S(O)₂OR^{bb}, S(O)₂N(R^{bb}₂), S(O)N(R^{bb}₂), S(O)₂N(R^{bb})C(O)N(R^{bb}₂), N(R^{bb})S(O)₂N(R^{bb}₂), N(R^{bb})S(O)₂R^{bb}, N(bb)S(O)R^{bb}, N(R^{bb})S(O)₂OR^{bb}, N(R^{bb}₂), N(R^{bb})C(O)R^{bb}, N(R^{bb})C(O)N(R^{bb}₂), or N(R^{bb})C(O)OR^{bb}, wherein alkyl, cycloalkyl, heterocyclyl, alkenyl, alkynyl, aryl, heteroaryl, aralkyl, and heteroarylalkyl are unsubstituted or substituted with one or more substituents R^{B}.
each R^{bb} is independently H, C₁-C₆ alkyl, C₃-C₇ cycloalkyl, C₃-C₇ heterocyclyl, C₂-C₆ alkenyl, C₂-C₆ alkynyl, C₃-C₇ aryl, C₃-C₇ heteroaryl, C₄-C₁₅ aralkyl or C₄-C₁₅ heteroarylalkyl which are unsubstituted or substituted with one or more R^{B};
each R^{B} is independently halogen, CN, OH, C₁-C₆ alkyl, OR^{BB}, C(O)R^{BB}, C(O)OR^{BB}, C(O)N(RB^{B2})₂, N(R^{BB}₂), OC(O)R^{BB}, N(R^{BB})C(O)R^{BB}, SR^{BB}, S(O)R^{BB}, S(O)₂R^{BB}, S(O)₂OR^{BB}, S(O)₂N(R^{BB}₂), S(O)N(RB^{B2}), S(O)₂N(R^{BB})C(O)N(R^{BB}₂), N(R^{BB})S(O)₂N(R^{BB}₂), N(R^{BB})S(O)₂R^{BB}, N(R^{BB})_{S}(O)R^{BB}, N(R^{BB})C(O)N(R^{BB}₂), N(R^{BB})C(O)OR^{BB}, and OC(O)N(R^{BB}₂), wherein C₁-C₆ alkyl is unsubstituted or substituted with one or more R';
each R^{BB} is independently H or C₁-C₆ alkyl, wherein C₁-C₆ alkyl is unsubstituted or substituted with one or more halogen;
each R' is independently halogen, CN, OH, OR", C(O)R", C(O)OR", C(O)N(R"₂), N(R"₂), OC(O)R", N(R")C(O)R", SR", S(O)R", S(O)₂R", S(O)₂OR", S(O)₂N(R"₂), S(O)N(R"₂), S(O)₂N(R")C(O)N(R"₂), N(R")S(O)₂N(R"₂), N(R")S(O)₂R", N(R")S(O)R", N(R")C(O)N(R"₂), N(R")C(O)O R" or OC(O)N(R"₂) and
each R" is independently H or C₁-C₆ alkyl, wherein C₁-C₆ alkyl is unsubstituted or substituted with one or more halogen;
and wherein in formula (II) the symbols have the following meanings:
X¹ is O, S, SO, SO₂ or NR^{x}, R^{x}NSO₂∼, where ∼ denotes the bond to the coumarin scaffold,
X² is O or NR^{x};
R^{x} is H, CH₃ or C₂H₅;
R⁶ is L³ - D;
L³ is a bond, C₁-C₆-alkylene, wherein one CH₂-group not linked to X¹ can be replaced by NR'; O, S, SO, SO₂, CO, CO-O or O-CO-;
D is H, an optionally derivatized sugar moiety, C₆-C₁₂-aryl, mono or bicyclic 3 to 9 membered hetaryl, C₃-C₈-cycloalkyl or mono or bicyclic 3 to 9 membered heterocyclyl, wherein the aryl, hetaryl, cycloalkyl or heterocyclyl group is unsubstituted or substituted with one or more substituents R^{c};
each R^{c} is independently halogen, CN, N₃, OCN, NCO, CNO, SCN, NCS, C₁-C₆ alkyl, C₃-C₇ cycloalkyl, C₃-C₇ heterocyclyl, C₂-C₆ alkenyl, C₂-C₆ alkynyl, C₃-C₇ aryl, C₃-C₇ heteroaryl, C₄-C₁₅ aralkyl, C₄-C₁₅ heteroarylalkyl, C(O)R^{cc}, C(O)OR^{cc}, C(O)N(R^{cc}₂), C(O)N(R^{cc})OR^{cc}, C(O)N(R^{cc})N(R^{cc}₂), C(NR^{cc})N(R^{cc}₂), C(NR^{cc})N(R^{cc})OR^{cc}, C(NR^{cc})N(R^{cc})N(R^{cc}₂), C(R^{cc})N(R^{cc}), C(NR^{cc}₂)NOR^{cc}, C(NR^{cc}₂)NOC(O)R^{cc}, C(R^{cc})NN(R^{cc}₂), CR^{cc}NOR^{cc}, OR^{cc}, OC(O)R^{cc}, OC(O)N(R^{cc}₂), SR^{cc}, S(O)R^{cc}, S(O)₂R^{cc}, S(O)₂OR^{cc}, S(O)₂N(R^{cc}₂), S(O)N(R^{cc}₂), S(O)₂N(R^{cc})C(O)N(R^{cc}₂), N(R^{cc})S(O)₂N(R^{cc}₂), N(R^{cc})S(O)₂R^{cc}, N(^{cc})S(O)R^{cc}, N(R^{cc})S(O)₂OR^{cc}, N(R^{cc}₂), N(R^{cc})C(O)R^{cc}, N(R^{cc})C(O)N(R^{cc}₂), or N(R^{cc})C(O)OR^{cc}, wherein alkyl, cycloalkyl, heterocyclyl, alkenyl, alkynyl, aryl, heteroaryl, aralkyl, and heteroarylalkyl are unsubstituted or substituted with one or more substituents R^{C}.
each R^{cc} is independently H, C₁-C₆ alkyl, C₃-C₇ cycloalkyl, C₃-C₇ heterocyclyl, C₂-C₆ alkenyl, C₂-C₆ alkynyl, C₃-C₇ aryl, C₃-C₇ heteroaryl, C₄-C₁₅ aralkyl or C₄-C₁₅ heteroarylalkyl which are unsubstituted or substituted with one or more R^{C};
each R^{C} is independently halogen, CN, OH, C₁-C₆ alkyl, OR^{CC}C(O)R^{CC}, C(O)OR^{CC}, C(O)N(R^{CC}₂), N(R^{CC}₂), OC(O)R^{CC}, N(R^{CC})C(O)R^{CC}, SR^{CC}, S(O)R^{CC}, S(O)₂R^{CC}, S(O)₂OR^{CC}, S(O)₂N(R^{CC}₂), S(O)N(R^{CC}₂), S(O)₂N(R^{CC})C(O)N(R^{CC}₂), N(R^{CCc})S(O)₂N(R^{CC}₂), N(R^{CCc})_{S}(O)₂R^{CC}, N(R^{CC})S(O)R^{CC}, N(R^{CC})C(O)N(R^{CC}₂), N(R^{CCc})C(O)OR^{CC}, and OC(O)N(R^{CC}₂), wherein C₁-C₆ alkyl is unsubstituted or substituted with one or more R';
each R^{CC} is independently H or C₁-C₆ alkyl, wherein C₁-C₆ alkyl is unsubstituted or substituted with one or more halogen;
each R' is independently halogen, CN, OH, OR", C(O)R", C(O)OR", C(O)N(R"₂), N(R"₂), OC(O)R", N(R")C(O)R", SR", S(O)R", S(O)₂R", S(O)₂OR", S(O)₂N(R"₂), S(O)N(R"₂), S(O)₂N(R")C(O)N(R"₂), N(R")S(O)₂N(R"₂), N(R")S(O)₂R", N(R")S(O)R", N(R")C(O)N(R"₂), N(R")C(O)O R" or OC(O)N(R"₂) and
each R" is independently H or C₁-C₆ alkyl, wherein C₁-C₆ alkyl is unsubstituted or substituted with one or more halogen;
R⁹ is OH, halogen, O-C₁-C₆-alkyl or O-benzyl, wherein alkyl or benzyl are unsubstituted or substituted by one or more halogen;
R¹⁰ is L⁴ - E;
L⁴ is -NR^{DD}-C(O)-, CO-NR^{DD}, SO₂-NR^{DD} or NR^{DD}-SO₂;
E is a residue of an α-amino acid, wherein the amino group optionally carries a protective group;
C₆-C₁₀-aryl, 5- or 6-membered heteroaryl, C₅-C₈-cycloalkyl, 5 to 7 membered heterocyclyl, C₁-C₈-alkyl, which groups are unsubstituted or substituted with one or more substituents R^{d};
or L⁴-E together with R⁹ forms an oxazole group condensed to the coumarin scaffold;
each R^{d} is independently halogen, CN, N₃, OCN, NCO, CNO, SCN, NCS, C₁-C₆ alkyl, C₃-C₇ cycloalkyl, C₃-C₇ heterocyclyl, C₂-C₆ alkenyl, C₂-C₆ alkynyl, C₃-C₇ aryl, C₃-C₇ heteroaryl, C₄-C₁₅ aralkyl, C₄-C₁₅ heteroarylalkyl, C(O)R^{dd}, C(O)OR^{dd}, C(O)N(R^{dd}₂), C(O)N(R^{dd})OR^{dd}, C(O)N(R^{dd})N(R^{dd}₂), C(NR^{dd})N(R^{dd}₂), C(NR^{dd})N(R^{dd})OR^{dd}, C(NR^{dd})N(R^{dd})N(R^{dd}₂), C(R^{dd})N(R^{dd}), C(NR^{dd}₂)NOR^{dd}, C(NR^{dd}₂)NOC(O)R^{dd}, C(R^{dd})NN(R^{dd}₂), CR^{dd}NOR^{dd}, OR^{dd}, OC(O)R^{dd}, OC(O)N(R^{dd}₂), SR^{dd}, S(O)R^{dd}, S(O)₂R^{dd}, S(O)₂OR^{bb}, S(O)₂N(R^{dd}₂), S(O)N(R^{dd}2), S(O)₂N(R^{dd})C(O)N(R^{dd}₂), N(R^{dd})S(O)₂N(R^{dd}₂), N(R^{dd})S(O)₂R^{dd}, N(^{dd})S(O)R^{dd}, N(R^{dd})S(O)_{2O}R^{dd}, N(R^{dd}2), N(R^{dd})C(O)R^{dd}, N(R^{dd})C(O)N(R^{dd}₂), or N(R^{dd})C(O)OR^{dd}, wherein alkyl, cycloalkyl, heterocyclyl, alkenyl, alkynyl, aryl, heteroaryl, aralkyl, and heteroarylalkyl are unsubstituted or substituted with one or more substituents R^{D};
each R^{dd} is independently H, C₁-C₆ alkyl, C₃-C₇ cycloalkyl, C₃-C₇ heterocyclyl, C₂-C₆ alkenyl, C₂-C₆ alkynyl, C₃-C₇ aryl, C₃-C₇ heteroaryl, C₄-C₁₅ aralkyl or C₄-C₁₅ heteroarylalkyl which are unsubstituted or substituted with one or more R^{D};
each R^{D} is independently halogen, CN, OH, C₁-C₆ alkyl, OR^{DD}, C(O)R^{DD}, C(O)OR^{DD}, C(O)N(R^{DD}₂), N(R^{DD}₂), OC(O)R^{DD}, N(R^{DD})C(O)R^{DD}, SR^{DD}, S(O)R^{DD}, S(O)₂R^{DD}, S(O)₂OR^{DD}, S(O)₂N(R^{DD}₂), S(O)N(R^{DD}₂), S(O)₂N(R^{DD})C(O)N(R^{DD}₂), N(R^{DD})S(O)₂N(R^{DD}₂), N(R^{DD})S(O)₂R^{DD}, N(R^{DD})S(O)R^{DD}, N(R^{DD})C(O)N(R^{DD}₂), N(R^{DD})C(O)OR^{DD}, and OC(O)N(R^{DD}₂), wherein C₁-C₆ alkyl is unsubstituted or substituted with one or more R'; each R^{DD} is independently H or C₁-C₆ alkyl, wherein C₁-C₆ alkyl is unsubstituted or substituted with one or more halogen;
each R' is independently halogen, CN, OH, OR", C(O)R", C(O)OR", C(O)N(R"₂), N(R"₂), OC(O)R", N(R")C(O)R", SR", S(O)R", S(O)₂R", S(O)₂OR", S(O)₂N(R"₂), S(O)N(R"₂), S(O)₂N(R")C(O)N(R"₂), N(R")S(O)₂N(R"₂), N(R")S(O)₂R", N(R")S(O)R", N(R")C(O)N(R"₂), N(R")C(O)O R" or OC(O)N(R"₂) and
each R" is independently H or C₁-C₆ alkyl, wherein C₁-C₆ alkyl is unsubstituted or substituted with one or more halogen and
R⁷ is H, CH₃, halogen or NO₂;
R⁸ is H, CH₃, halogen or NO₂ and
R¹¹ is CH₃, halogen or H.

2. The compound of claim 1, which is a tetrahydrotriazine derivative of the formula (I).

3. The tetrahydrotriazine derivative of claim 2, wherein the symbols in formula (I) have the following meanings:
A is 3-pyridyl, 3-fluorophenyl, 3,4-difluorophenyl or 4-fluorophenyl;
X is S, SO or SO₂;
L¹ is -CH₂-, -CH(CH₃)-, trimethylsilyl, triethylsilyl or tert.-butyldimethylsilyl;
L² is - CH₂-, CH(CH₃)-, -CH₂-CH₂-, -CH(CH₂)-CH₂-, -CH₂-CH(CH₃)- or a bond;
R¹ is H or methyl;
R², R³, R⁴ and R⁵ are H or
R² and R³ together and/or R⁴ and R⁵ together are =O;
B is C₁-C₈-aryl, 5- or 6-membered heteroaryl, C₅-C₆-cycloalkyl, 5 to 6 membered heterocyclyl, C₁-C₆-alkyl, which groups are unsubstituted or substituted with one or more substituents R^{b}, or B is H;
each R^{b} is independently halogen, preferably F, Cl, Br, CN, N₃, OCN, NCO, CNO, SCN, NCS, C₁-C₆ alkyl, C₃-C₆ cycloalkyl, C₃-C₇ heterocyclyl, C₂-C₆ alkenyl, C₂-C₆ alkynyl, phenyl, C₅-C₆ heteroaryl, benzyl, C₆-C₁₄ heteroarylalkyl, C₄-C₁₀ cycloalyklalkyl, C(O)R^{bb}, C(O)OR^{bb}, C(O)N(R^{bb}₂), C(O)N(R^{bb})OR^{bb}, C(O)N(R^{bb})N(R^{bb}₂), C(NR^{bb})N(R^{bb}₂), C(NR^{bb})N(R^{bb})OR^{bb}, C(NR^{bb})N(R^{bb})N(R^{bb}₂), C(R^{bb})N(R^{bb}), C(NR^{bb}₂)NOR^{bb}, C(NR^{bb}₂)NOC(O)R^{bb}, C(R^{bb})NN(R^{bb}₂), CR^{bb}NOR^{bb}, OR^{bb} , OC(O)R^{bb}, OC(O)N(R^{bb}₂), SR^{bb}, S(O)R^{bb}, S(O)₂R^{bb}, S(O)₂OR^{bb}, S(O)₂N(R^{bb}₂), S(O)N(R^{bb}₂), S(O)₂N(R^{bb})C(O)N(R^{bb}₂), N(R^{bb})S(O)₂N(R^{bb}₂), N(R^{bb})S(O)₂R^{bb}, N(^{bb})S(O)R^{bb}, N(R^{bb})S(O)₂OR^{bb}, N(R^{bb}₂), N(R^{bb})C(O)R^{bb}, N(R^{bb})C(O)N(R^{bb2}), or N(R^{bb})C(O)OR^{bb}, wherein alkyl, cycloalkyl, heterocyclyl, alkenyl, alkynyl, phenyl, heteroaryl, benzyl, and heteroarylalkyl are unsubstituted or substituted with one or more substituents R^{B};
each R^{bb} is preferably independently H, C₁-C₆ alkyl, C₃-C₇ cycloalkyl, C₃-C₇ heterocyclyl, C₂-C₆ alkenyl, C₂-C₆ alkynyl, C₃-C₇ aryl, C₃-C₇ heteroaryl, C₄-C₁₅ aralkyl or C₄-C₁₅ heteroarylalkyl which are unsubstituted or substituted with one or more R^{B};
each R^{B} is independently halogen, CN, OH, C₁-C₆ alkyl, OR^{BB}, C(O)R^{BB}, C(O)OR^{BB}, C(O)N(R^{BB}₂), N(R^{BB}₂), OC(O)R^{BB}, N(R^{BB})C(O)R^{BB}, SR^{BB}, S(O)R^{BB}, S(O)₂R^{BB}, S(O)₂OR^{BB}, S(O)₂N(R^{BB}₂), S(O)N(R^{BB2}), S(O)₂N(R^{BB})C(O)N(R^{BB}₂), N(R^{BB})S(O)₂N(R^{BB}₂), N(R^{BB})S(O)₂R^{BB}, N(R^{BB})S(O)R^{BB}, N(R^{BB})C(O)N(R^{BB2}), N(R^{BB})C(O)OR^{BB}, and OC(O)N(R^{BB}₂), wherein C₁-C₆ alkyl is unsubstituted or substituted with one or more substituents R';
each R^{BB} is independently H or C₁-C₆ alkyl, wherein C₁-C₆ alkyl is unsubstituted or substituted with one or more halogen; each R' is independently halogen, CN, OH, OR", C(O)R", C(O)OR", C(O)N(R"₂), N(R"₂), OC(O)R", N(R")C(O)R", SR", S(O)R", S(O)₂R", S(O)₂OR", S(O)₂N(R"₂), S(O)N(R"₂), S(O)₂N(R")C(O)N(R"₂), N(R")S(O)₂N(R"₂), N(R")S(O)₂R", N(R")S(O)R", N(R")C(O)N(R"₂),
N(R")C(O)OR" or OC(O)N(R"₂; and
each R" is independently H or C₁-C₆ alkyl, wherein C₁-C₆ alkyl is unsubstituted or substituted with one or more halogen.

4. The tetrahydrotriazine derivative of claim 2 or 3, wherein the symbols in formula (I) have the following meanings:
A is 3-pyridyl, 3-fluorophenyl, 3,4-difluorophenyl or 4-fluorophenyl;
X is S, SO or SO₂;
L¹ is CH₂ or CH(CH₃);
L² is -CH₂⁻;
B is phenyl or 5 or 6 membered hetero aryl, which groups are unsubstituted or substituted with one or more, preferably one or two, more preferably one substituent R^{b};
each of R¹, R², R³, R⁴ and R⁵ is H or
R² and R³ together and/or R⁴ and R⁵ together are =O;
R^{b} is halogen, preferably F, Cl or Br, CN, OH, C₁-C₄-alkyl, preferably methyl, C₁-C₆ cycloalkyl, C₄-C₈ Cycloalkylalkyl, C₁-C₄-haloalkyl, preferably CF₃, C₁-C₄-alkoxy, preferably methoxy, C₁-C₄-haloalkoxy, preferably OCF₃, COOH, COO-R^{bb}, CONH₂, CONHR^{bb}, CONR^{bb}₂, phenyl or phenoxy, where the last two groups are unsubstituted or substituted with one or more halogen, preferably F, Cl or Br, CN, OH, C₁-C₄-alkyl, preferably methyl, C₁-C₄-haloalkyl, preferably CF₃, C₁-C₄-alkoxy, preferably methoxy, C₁-C₄-haloalkoxy, preferably OCF₃, COOH, COO-R^{bb}, CONH₂, CONHR^{bb} or
CONR^{bb}₂;
R^{bb} is C₁-C₆-alkyl, benzyl or phenyl, which groups are unsubstituted or
substituted with one or more halogen or a substituent R^{B}; and
each R^{B} is independently COOH or COO-C₁-C₆-alkyl.

5. The tetrahydrotriazine derivative of claim 2, 3 or 4 wherein the symbols in formula (I) have the following meanings:
A is 3-pyridyl, 3-fluorophenyl, 3,4-difluorophenyl or 4-fluorophenyl;
X is S, SO or SO₂;
L¹ is CH₂;
L² is CH₂;
each of R¹, R², R³, R⁴ and R⁵ is H.
B is phenyl which is unsubstituted or substituted with one or more, preferably one or two, more preferably one substituent R^{b}, where one substituent R^{b} is preferably in the para position with respect to L²;
each R^{b} is independently F, Cl, Br, CN, OH, C₁-C₄-alkyl, C₁-C₄-halo-alkyl, C₁-C₄-alkoxy, C₁-C₄-haloalkoxy, COOH, COOR^{bb}, CONH₂, CONHR^{bb}, CONR^{bb}₂, phenyl, phenoxy, where the last two groups are unsubstituted or substituted with one or more halogen, preferably F, Cl or Br, CN, OH, C₁-C₄-alkyl, preferably methyl, C₁-C₄-haloalkyl, preferably CF₃, C₁-C₄-alkoxy, preferably methoxy, C₁-C₄-haloalkoxy, preferably OCF₃, COOH, COO-R^{bb}, CONH₂, CONHR^{bb} or CONR^{bb}₂ and
each R^{bb} is independently C₁-C₆-alkyl, benzyl or phenyl.

6. The compound of claim 1, which is a coumarin derivative of formula (II).

7. The coumarin derivative of claim 6, wherein L⁴ is NR^{DD}-SO₂ or SO₂NR^{DD}.

8. The coumarin derivative of claim 6, wherein L⁴ is NR^{DD}-C(O) or C(O)-NR^{DD}.

9. The coumarin derivative of claim 6, 7 or 8, wherein
L³ is a bond, C₁-C₆-alkylene, wherein one CH₂-group not linked to X¹ can be replaced by NR'; O, S, SO, SO₂, CO, CO-O or O-CO- and
D is H, C₆-C₁₂-aryl, mono or bicyclic 3 to 9 membered hetaryl, C₃-C₈-cycloalkyl or mono or bicyclic 3 to 9 membered heterocyclyl, wherein the aryl, hetaryl, cycloalkyl or heterocyclyl group is unsubstituted or substituted with one or more substituents R^{c}.

10. A tetrahydrotriazine derivative of formula (I) according to any one of claims 1 to 5 or a derivative of formula (II) according to any one of claims 1 or 6 to 9, a tautomer coumarin, pharmaceutically acceptable salt, solvate or hydrate of either, for use in a method for treating cancer in a patient with the proviso that R⁶ in formula (II) is not a sugar moiety.

11. A tetrahydrotriazine derivative of formula (I) according to any one of claims 1 to 5, a tautomer, pharmaceutically acceptable salt, solvate or hydrate thereof.

12. A coumarin derivative of formula (II) according to any one of claims 1 or 6 to 9, a tautomer, pharmaceutically acceptable salt, solvate or hydrate thereof, with the proviso that R⁶ in formula (II) is not a sugar moiety.

13. A pharmaceutical composition comprising
a) a tetrahydrotriazine derivative of formula (I) according to any one of claims 1 to 5 or a coumarin derivative of formula (II) according to any one of claims 1 or 6 to 9, a tautomer, pharmaceutically acceptable salt, solvate or hydrate of either
and
b) a pharmaceutically acceptable carrier
with the proviso that R⁶ in formula (II) is not a sugar moiety.

14. A method for treating cancer in a patient comprising the step of administering to the patient a therapeutically effective amount of a tetrahydrotriazine derivative of formula (I) according to any one of claims 1 to 5 or a coumarin derivative of formula (II) according to any one of claims 1 or 6 to 9, a tautomer, pharmaceutically acceptable salt, solvate or hydrate of either, with the proviso that R⁶ in formula (II) is not a sugar moiety.

15. The use of a tetrahydrotriazine derivative of formula (I) according to any one of claims 1 to 5, a coumarin derivative of formula (II) according to any one of claims 1 or 6 to 9, a tautomer, pharmaceutically acceptable salt, solvate or hydrate of either for the preparation of a medicament for treating cancer with the proviso that R⁶ in formula (II) is not a sugar moiety.
